(19) Europäisches Patentamt
European Patent Office
Office européen des brevets

(11) Publication number: **0 588 194 A2**

(12) **EUROPEAN PATENT APPLICATION**

(21) Application number: **93114248.3**

(22) Date of filing: **06.09.93**

(51) Int. Cl.[5]: **C07D 239/60**, C07D 401/12, C07D 409/12, C07D 417/12, C07D 403/12, C07D 405/12, C07D 413/12, A01N 43/54

(30) Priority: **18.09.92 JP 273446/92**
**19.02.93 JP 53172/93**

(43) Date of publication of application:
**23.03.94 Bulletin 94/12**

(84) Designated Contracting States:
**BE CH DE FR GB IT LI NL**

(71) Applicant: **NIHON BAYER AGROCHEM K.K.**
**10-8, Takanawa 4-chome**
**Minato-ku**
**Tokyo 108(JP)**

(72) Inventor: **Goto, Toshio**
**214-18, Koganei,**
**Kokubunji-machi**
**Shimotsuga-gun, Tochigi(JP)**
Inventor: **Kitagawa, Yoshinori**
**1085, Ara-machi**
**Mohka-shi, Tochigi(JP)**
Inventor: **Hayakawa, Hidenori**
**3-17-20, Ekiminami-machi**
**Oyama-shi, Tochigi(JP)**
Inventor: **Ito, Seishi**
**1-39-1, Ekihigashidori**
**Oyama-shi, Tochigi(JP)**
Inventor: **Shibuya, Katsuhiko**
**1425-2, Hitotonoya,**
**Oh-aza**
**Oyama-shi, Tochigi(JP)**
Inventor: **Minegishi, Natsuko**
**1-9-31, Wakagi-cho**
**Oyama-shi, Tochigi(JP)**
Inventor: **Ukawa, Kazuhiro**
**1-23-13, Ekihigashidori**
**Oyama-shi, Tochigi(JP)**
Inventor: **Yamaoka, Tatsuya**
**934-7, Hitotonoya,**
**Oh-aza**
**Oyama-shi, Tochigi(JP)**
Inventor: **Ueno, Chieko**
**934-7, Hitotonoya,**
**Oh-aza**
**Oyama-shi, Tochigi(JP)**
Inventor: **Kyo, Yoshiko**
**934-7, Hitotonoya,**
**Oh-aza**
**Oyama-shi, Tochigi(JP)**

(74) Representative: **Schumacher, Günter, Dr. et al**
**Bayer AG**
**Konzernverwaltung RP**
**Patentabteilung**
**D-51368 Leverkusen Bayerwerk (DE)**

(54) **Hydrazones and their use as herbicides.**

EP 0 588 194 A2

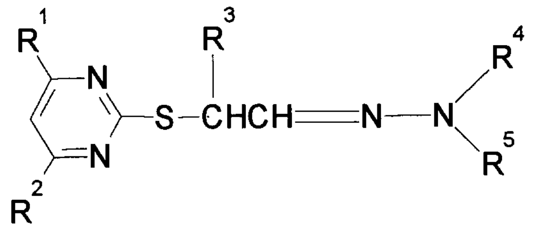

(I)

(57) wherein $R^1$, $R^2$, $R^3$, $R^4$ and $R^5$ have those meanings as described in the specification, processes for their preparation, and the use of the new compounds as herbicides.

The present invention relates to novel hydrazones, to processes for their preparation, and to their use as herbicides.

It has already been disclosed that a certain group of pyrimidinylthiocarboxylic acid derivatives is useful as herbicides (see Japanese Laid-Open Patent Application Nos. 85262/1990, 135963/1991 and 240777/1991).

There have now been found novel hydrazones of the formula (I)

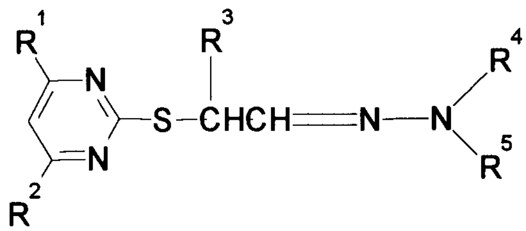 (I)

wherein

$R^1$ represents $C_{1-4}$ alkyl, $C_{1-4}$ alkoxy, halogeno-$C_{1-4}$ alkyl, halogeno-$C_{1-4}$ alkoxy or halogen,

$R^2$ represents $C_{1-4}$ alkoxy, halogeno-$C_{1-4}$ alkyl, halogeno-$C_{1-4}$ alkoxy or halogen,

$R^3$ represents $C_{3-8}$ cycloalkyl which may be substituted, $C_{1-15}$ alkyl which may be substituted or phenyl which may be substituted,

$R^4$ represents hydrogen, $C_{1-6}$ alkyl which may be substituted, $C_{2-6}$ alkenyl, $C_{2-6}$ alkynyl, phenyl which may be substituted, a 5- or 6-membered heterocyclic ring which may be substituted, a condensed heterocyclic ring which may be substituted, or one of the following groups:

$-SO_2-R^6$, and

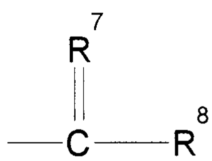

$R^5$ represents hydrogen, phenyl which may be substituted, or $C_{1-8}$ alkyl,

or wherein $R^4$ and $R^5$ together may represent a group

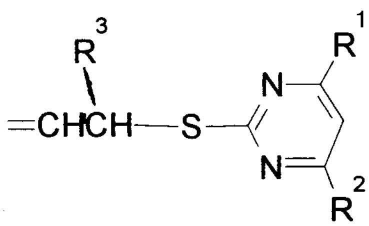

wherein $R^1$, $R^2$ and $R^3$ have the same meanings as mentioned above,

$R^6$ represents $C_{1-6}$ alkyl, phenyl which may be substituted, or a 5- or 6-membered heterocyclic ring which may be substituted,

$R^7$ represents oxygen or sulfur,

$R^8$ represents $C_{1-15}$ alkyl which may be substituted, $C_{1-6}$ alkoxy, phenyl which may be substituted, a 5- or 6-membered heterocyclic ring which may be substituted, a condensed heterocyclic ring which may be substituted, $C_{1-4}$ alkoxy-carbonyl, amino-carbonyl or one of the following groups:

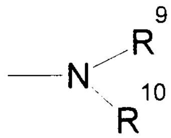 , 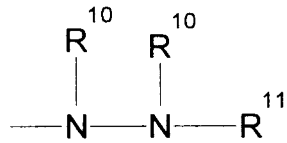

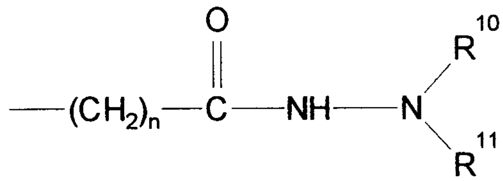

or 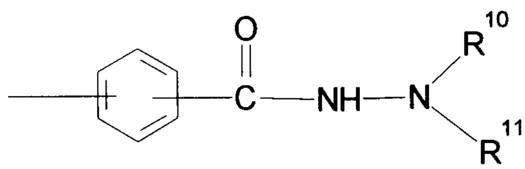

- $R^9$ represents hydrogen, $C_{3-7}$ cycloalkyl which may be substituted by $C_{1-4}$ alkyl, $C_{1-6}$ alkyl which may be substituted, phenyl which may be substituted, $C_{2-6}$ alkenyl, $C_{2-6}$ alkynyl, benzenesulfonyl which may be substituted or benzoyl which may be substituted,
- $R^{10}$ represents hydrogen, $C_{1-8}$ alkyl or phenyl, if two $R^{10}$ are in the same compound then both $R^{10}$ may have the same or different meanings
- $R^{11}$ represents hydrogen, $C_{1-15}$ alkyl which may be substituted, or phenyl which may be substituted, or $R^{10}$ and $R^{11}$ together represent a group of the following formula:

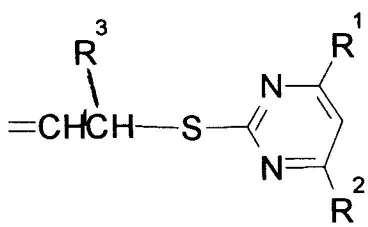

wherein $R^1$, $R^2$ and $R^3$ have the same meanings as mentioned above,
and
n represents an integer of 0 to 10.

Hydrazones of the formula (I) are obtained when

(a) compounds of the formule (II)

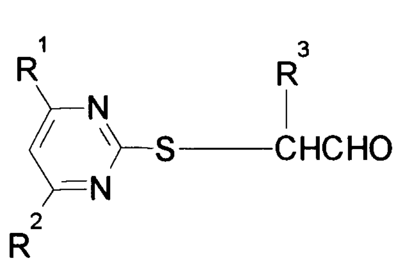 (II)

wherein $R^1$, $R^2$ and $R^3$ represent the same meanings as mentioned above,
are reacted with a hydrazine derivative of the formula (III)

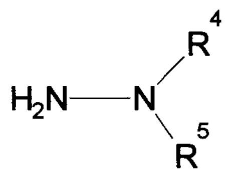 (III)

wherein $R^4$ and $R^5$ represent the same meanings as mentioned above, in the presence of inert solvents and, if appropriate, in the presence of an acid binder or an acid,
or
(b) in the case where $R^4$ and $R^5$ together form a group of the formula

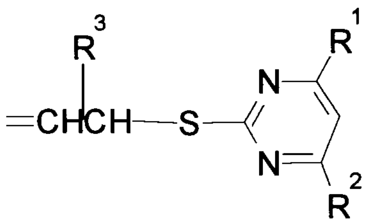

one mol hydrazine is reacted with two mols of a compound of the formula (II), in the presence of inert solvents and, if appropriate, in the presence of an acid,
or
(c) in the case where $R^4$ represents $-SO_2-R^6$: a compound of the formula (Ia)

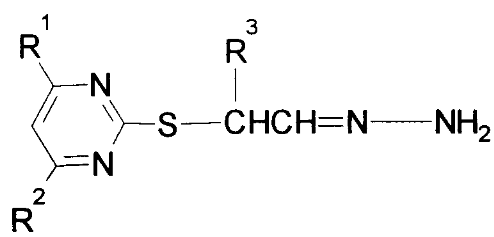 (Ia)

wherein $R^1$, $R^2$ and $R^3$ have the same meanings as mentioned above,
are reacted with a compound of the formula (IV)

$R^{12}—SO_2—R^6$

wherein $R^6$ has the same meaning as mentioned above, and $R^{12}$ represents chlorine or bromine, in the presence of inert solvents and, if appropriate, in the presence of a acid binder,
or
(d) in the case where $R^4$ represents

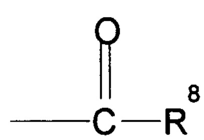

the above mentioned compound of the formula (Ia) is reacted with a compound of the formula (V)

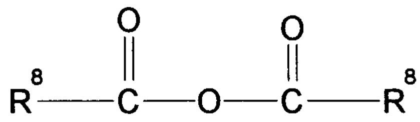 (V)

wherein $R^8$ has the same meaning as mentioned above,
in the presence of inert solvents and, if appropriate, in the presence of an acid binder,
or
(e) in the case where $R^4$ represents

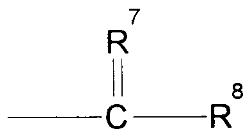 :

the above mentioned compound of the formula (Ia) is reacted with a compound of the formula (VI)

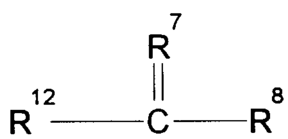 (VI)

wherein $R^7$, $R^8$ and $R^{12}$ have the same meanings as mentioned above,
in the presence of inert solvents and, if appropriate, in the presence of an acid binder.

The novel hydrazones according to the invention exhibit powerful herbicidal properties.

Surprisingly, the hydrazones according to the invention exhibit a substantially higher selective herbicidal action than those known from the prior art, for instance, the aforementioned Japanese Laid-open Patent Application Nos. 85262/1990, 135963/1991 and 240777/1991.

In the compounds of the formula (I) and their intermediates of the formulae (II) to (VI), the halogen and the halogen atoms of the halogenoalkyl and halogenoalkoxy represent fluorine, chlorine, bromine and iodine, preferably chlorine or fluorine,
the $C_{1-15}$ alkyl and alkyl moiety of the alkoxy halogenoalkyl and halogenoalkoxy, represents straight chain or branched alkyl such as methyl, ethyl, propyl, isopropyl, n-(iso-, sec- or tert-)butyl, n-(iso-, sec- and neo)-pentyl, hexyl, heptyl, nonyl, decyl, undecyl, dodecyl, tetradecyl, pentadecyl, 1-ethyl-1-methylpropane, and so on.

The $C_{2-6}$ alkenyl includes vinyl, allyl, 1-propenyl, 1-(2-, or 3-)butenyl, and the like, the $C_{2-6}$ alkynyl includes ethynyl, propargyl, 1-propenyl and the like, preferably propargyl and 1-propynyl.

The 5- or 6-membered heterocyclic ring having one to four hetero atoms selected from the group consisting of nitrogen, sulfur and oxygen, such as thiadiazolyl, diazolyl, furyl, thiazolyl, imidazolyl, pyridyl, pyrimidinyl, thienyl and the like.

The condensed heterocyclic ring represents a 9- or 10-membered bicyclic group which has been formed by the abovementioned 5- or 6-membered ring being condensed with phenyl, such as quinolyl, benzoxazolyl, benzothiazolyl, benzisoxazolyl, benzimidazolyl, phthalazinyl, indolyl and the like.

Among the hydrazones according to the invention of the formula (I), the preferred compounds are those in which:

$R^1$ represents $C_{1-4}$alkyl, $C_{1-4}$alkoxy, halogeno-$C_{1-4}$ alkyl, halogeno-$C_{1-4}$alkoxy or halogen,
$R^2$ represents $C_{1-4}$ alkoxy, halogeno-$C_{1-4}$ alkyl, halogeno-$C_{1-4}$ alkoxy or halogen,
$R^3$ represents $C_{3-6}$ cycloalkyl which is unsubstituted or substituted by $C_{1-4}$ alkyl, $C_{1-15}$ alkyl which is unsubstituted or substituted by one of the group consisting of halogen, $C_{3-8}$ cycloalkyl, phenyl which may be substituted by one of the groups consisting of nitro, cyano, halogen, $C_{1-4}$ alkyl, halogeno$C_{1-2}$ alkyl and $C_{1-4}$ alkoxy, and naphthyl,
$R^3$ further represents phenyl which is unsubstituted or substituted by one of the group consisting of $C_{1-4}$ alkyl, $C_{1-4}$ alkoxy halogen, halogeno-$C_{1-4}$ alkyl, nitro and cyano,
$R^4$ represents hydrogen, $C_{1-6}$ alkyl which is unsubstituted or substituted by halogen or phenyl which

may be substituted by one of the group consisting of nitro, cyano, halogen, $C_{1-4}$ alkyl, halogeno-$C_{1-4}$ alkyl and $C_{1-4}$-alkoxy,

$R^4$ further represents $C_{2-6}$ alkenyl, $C_{2-6}$ alkynyl, phenyl which is unsubstituted or substituted by one of the group consisting of hydroxycarbonyl, halogen, $C_{1-4}$ alkyl, $C_{1-4}$ alkoxy, halogeno-$C_{1-4}$ alkyl, halogeno-$C_{1-4}$ alkoxy, nitro, cyano and trifluoromethylsulfonyl,

$R^4$ further represents an optionally substituted 5- or 6-membered heterocyclic ring, the hetero atom(s) of the heterocyclic ring are selected from the group consisting of nitrogen, oxygen and sulfur, and the substituent is selected from the group consisting of halogen, $C_{1-4}$ alkyl and halogeno-$C_{1-4}$ alkyl,

$R^4$ further represents a 5- or 6-membered heterocyclic ring condensed with phenyl which may be substituted (the hetero atom(s) of the condensed heterocyclic ring are selected from the group consisting of nitrogen, oxygen and sulfur, and the substituent is selected from the group consisting of halogen, $C_{1-4}$ alkyl and halogeno-$C_{1-4}$ alkyl), or one of the following groups;

$-SO_2-R^6$ and

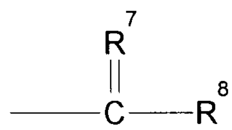

$R^5$ represents hydrogen, phenyl which may be unsubstituted or substituted by one of the group consisting of halogen and $C_{1-4}$ alkyl, or $C_{1-8}$ alkyl,

or wherein $R^4$ and $R^5$ together may represent a group represented by

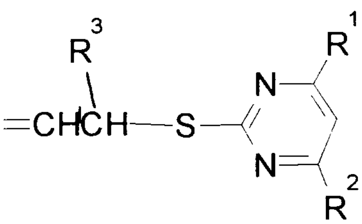

wherein $R^1$, $R^2$ and $R^3$ have the same meanings as mentioned above,

$R^6$ represents $C_{1-4}$ alkyl, phenyl which is unsubstituted or substituted by one of the group consisting of halogen, $C_{1-4}$ alkyl, $C_{1-4}$ alkoxy, $C_{1-4}$ alkoxy-carbonyl, halogeno-$C_{1-4}$ alkyl, halogeno-$C_{1-4}$ alkoxy, nitro, cyano and trifluoromethylsulphonyl,

$R^6$ further represents an optionally substituted 5- or 6-membered heterocyclic ring (the hetero atom(s) of the heterocyclic ring are selected from the group consisting of nitrogen, oxygen and sulfur, and the substituent is selected from the group consisting of halogen, $C_{1-4}$ alkyl and halogeno-$C_{1-4}$ alkyl),

$R^7$ represents oxygen or sulfur,

$R^8$ represents $C_{1-15}$ alkyl which may be unsubstituted or substituted by one of the group consisting of halogen, nitro, cyano, $C_{1-4}$ alkyl, $C_{1-4}$ alkoxy, $C_{1-4}$ alkoxycarbonyl, halogeno-$C_{1-4}$ alkyl, halogeno-$C_{1-4}$ alkoxy, and phenoxy which may be substituted by one of the group consisting of nitro, cyano, halogen, $C_{1-4}$ alkyl, halogeno-$C_{1-4}$ alkyl and $C_{1-4}$ alkoxy, phenoxy-$C_{1-4}$ alkylthio which may be unsubstituted or substituted by one of the group consisting of nitro, cyano, halogen, $C_{1-4}$ alkyl, halogeno-$C_{1-4}$ alkyl and $C_{1-4}$ alkoxy,

$R^8$ further represents $C_{1-6}$ alkoxy, phenyl which may be substituted by one of the group consisting of hydroxy, halogen, $C_{1-4}$ alkyl, $C_{1-4}$ alkoxy, halogeno-$C_{1-4}$ alkyl, halogeno-$C_{1-4}$ alkoxy, nitro and cyano,

$R^8$ further represents an optionally substituted 5- or 6-membered heterocyclic ring (the hetero atom(s) of the heterocyclic ring are selected from the group consisting of nitrogen, oxygen and sulfur, and the substituent is selected from the group consisting of halogen, $C_{1-4}$ alkyl and halogeno-$C_{1-4}$ alkyl), or

$R^8$ further represents $C_{1-4}$ alkoxy-carbonyl, aminocarbonyl or one of the following groups;

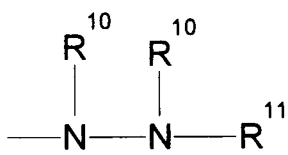 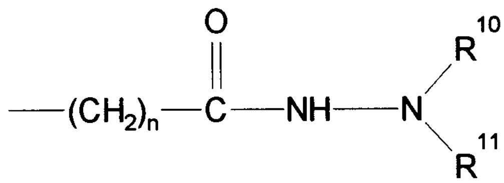 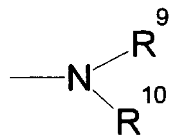 ,

or 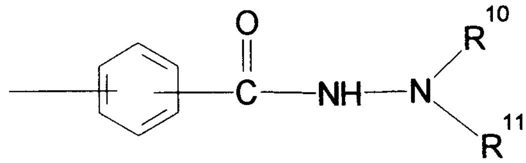

$R^9$ represents hydrogen, $C_{3-7}$ cycloalkyl which is unsubstituted or substituted by $C_{1-4}$ alkyl, $C_{1-6}$ alkyl which is unsubstituted or substituted by one of the group consisting of halogen, $C_{3-8}$ cycloalkyl and phenyl which may be substituted by one of the group consisting of nitro, cyano, halogen, $C_{1-4}$ alkyl, halogeno-$C_{1-4}$ alkyl, $C_{1-4}$ alkoxy,
and naphthyl,

$R^9$ further represents phenyl which may be unsubstituted or substituted by one of the group consisting of halogen, $C_{1-4}$ alkyl, $C_{1-4}$ alkoxy, halogeno-$C_{1-4}$ alkyl, nitro and cyano, or

$R^9$ further represents $C_{2-6}$ alkenyl, $C_{2-6}$ alkynyl bensenesulfonyl which may be unsubstituted or substituted by one of the group consisting of halogen, $C_{1-4}$ alkyl, $C_{1-4}$ alkoxy, halogeno-$C_{1-4}$ alkyl, nitro and cyano, or

$R^9$ further represents benzoyl which may be unsubstituted or substituted by one of the group consisting of halogen, $C_{1-4}$ alkyl, $C_{1-4}$ alkoxy, halogeno-$C_{1-4}$ alkyl, nitro and cyano,

$R^{10}$ represents hydrogen, $C_{1-8}$ alkyl, or phenyl, and if two $R^{10}$ are in the same compound then both $R^{10}$ may have the same or different meanings,

$R^{11}$ represents hydrogen, $C_{1-15}$ alkyl which may be unsubstituted or substituted by halogen or phenyl which may be substituted by one of the group consisting of nitro, cyano, halogen, $C_{1-4}$ alkyl, halogeno-$C_{1-4}$ alkyl and $C_{1-4}$ alkoxy,

$R^{11}$ further represents phenyl which may be unsubstituted or substituted by one of the group consisting of halogen, $C_{1-4}$ alkyl, $C_{1-4}$ alkoxy, halogeno-$C_{1-4}$ alkyl, nitro and cyano,

or wherein $R^{10}$ and $R^{11}$ together may represent a group

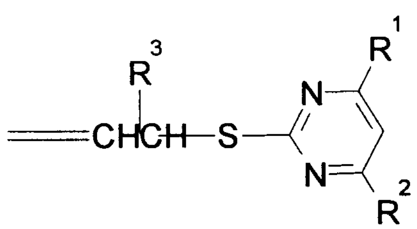

wherein $R^1$, $R^2$ and $R^3$ have the same meanings as mentioned above,
and

n represents an integer of 0 to 10.

Particularly preferred compounds are those, in which:

$R^1$ represents $C_{1-2}$ alkyl, $C_{1-2}$ alkoxy chloro-$C_{1-2}$ alkyl, fluoro-$C_{1-2}$ alkyl, chloro-$C_{1-2}$ alkoxy, fluoro-$C_{1-2}$ alkoxy, difluoro-$C_{1-2}$ alkoxy, trifluoro-$C_{1-2}$-alkoxy or chloro,

$R^2$ represents $C_{1-2}$ alkoxy, chloro-$C_{1-2}$ alkyl, fluoro-$C_{1-2}$ alkyl, chloro-$C_{1-2}$ alkoxy, fluoro-$C_{1-2}$ alkoxy, difluoro-$C_{1-2}$ alkoxy, tnfluoro-$C_{1-2}$ alkoxy or chloro,

$R^3$ represents $C_{3-6}$ cycloalkyl which is unsubstituted or substituted by $C_{1-2}$ alkyl, $C_{1-12}$ alkyl which is unsubstituted or substituted by one of the group consisting of fluoro, chloro, $C_{3-6}$ cycloalkyl and phenyl which may be substituted by one of the group consisting of nitro, cyano, fluorine, chlorine, bromine, $C_{1-2}$ alkyl, fluoro-$C_{1-2}$ alkyl or $C_{1-2}$ alkoxy,

$R^3$ further represents phenyl which may be unsubstituted or substituted by one of the group consisting of $C_{1-2}$ alkyl, $C_{1-2}$ alkoxy, fluorine, chlorine, bromine, fluoro-$C_{1-2}$ alkyl, nitro or cyano,

$R^4$ represents hydrogen, $C_{1-4}$ alkyl which may be unsubstituted or substituted by one of the group consisting of fluorine, chlorine, bromine, phenyl which may be substituted by one of the group consisting of nitro, cyano, fluorine, chlorine, bromine, $C_{1-2}$ alkyl, fluoro-$C_{1-2}$ alkyl and $C_{1-2}$ alkoxy,

$R^4$ further represents $C_{3-6}$ alkenyl, $C_{3-6}$ alkynyl, phenyl which may be unsubstituted or substituted by one of the group consisting of hydroxycarbonyl, fluorine, chlorine, bromine, $C_{1-4}$ alkyl, $C_{1-4}$ alkoxy, fluoro-$C_{1-4}$ alkyl, fluoro-$C_{1-4}$ alkoxy, nitro, cyano and trifluoromethylsulfonyl,

$R^4$ further represents an optionally substituted 5- or 6-membered heterocyclic ring the hetero atom(s) of the heterocyclic ring being selected from the group consisting of nitrogen, oxygen and sulfur, and the substituent is selected from the group consisting of fluorine, chlorine, methyl, ethyl and fluoro-$C_{1-2}$ alkyl,

$R^4$ further represents an optionally substituted 5- or 6-membered heterocyclic ring condensed with phenyl, the hetero atom(s) of the condensed heterocyclic ring are selected from the group consisting of nitrogen, oxygen and sulfur, and the substituent is selected from the group consisting of fluorine, chlorine, methyl, ethyl and fluoro-$C_{1-2}$ alkyl, or one of the following groups: $-SO_2-R^6$ and

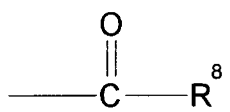
,

$R^5$ represents hydrogen, $C_{1-6}$ alkyl or phenyl which is unsubstituted or substituted by one of the group consisting of fluoro, chloro, methyl, ethyl and butyl,

or $R^4$ and $R^5$ together may represent a group

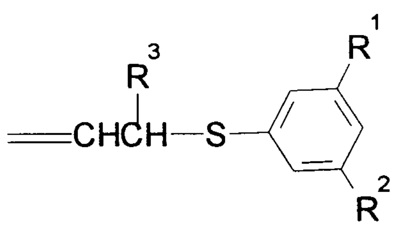

wherein $R^1$ , $R^2$ and $R^3$ have the same meanings as mentioned above,

$R^6$ represents $C_{1-4}$ alkyl, phenyl which may be unsubstituted or substituted by one of the group consisting of fluorine, chlorine, bromine, $C_{1-2}$ alkyl, $C_{1-2}$ alkoxy, $C_{1-2}$ alkoxy-carbonyl, fluoro-$C_{1-2}$ alkyl, chloro-$C_{1-2}$ alkyl, fluoro-$C_{1-2}$ alkoxy, chloro-$C_{1-2}$ alkoxy, nitro, cyano and trifluoromethylsulphonyl,

$R^6$ further represents an optionally substituted 5- or 6-membered heterocyclic ring, the hetero atom(s) of the heterocyclic ring being selected from the group consisting of nitrogen, oxygen and sulfur, and the substituent is selected from the group consisting of fluorine, chlorine, methyl, ethyl and fluoro-$C_{1-2}$ alkyl,

$R^8$ represents $C_{1-8}$ alkyl which is unsubstituted or substituted by one of the group consisting of fluorine, chlorine, bromine, $C_{1-2}$ alkyl, $C_{1-2}$ alkoxy, fluoro-$C_{1-2}$ alkyl, chloro-$C_{1-2}$ alkyl, fluoro-$C_{1-2}$ alkoxy, chloro-$C_{1-2}$ alkoxy, nitro, cyano and phenoxy which may be substituted by one of the group consisting of nitro, cyano, fluoro, chloro, bromo, $C_{1-2}$ alkyl, fluoro-$C_{1-2}$ alkyl, $C_{1-2}$ alkoxy and phenoxy-$C_{1-2}$ alkylthio, wherein the phenyl ring may be unsubstituted or substituted by one of the group consisting of nitro, cyano, fluorine, chlorine, bromine, $C_{1-2}$ alkyl, fluoro-$C_{1-2}$ alkyl and $C_{1-2}$ alkoxy,

$R^8$ further represents $C_{1-4}$ alkoxy, phenyl which may be substituted by one of the group consisting of hydroxy, fluorine, chlorine, bromine, $C_{1-4}$ alkyl, $C_{1-4}$ alkoxy, fluoro-$C_{1-2}$ alkyl, fluoro-$C_{1-2}$ alkoxy, nitro and cyano,

$R^8$ further represents an optionally substituted 5- or 6-membered heterocyclic ring, the hetero atoms of the heterocyclic ring are selected from the group consisting of nitrogen, oxygen and sulfur, and the substituent is selected from the group consisting of fluorine, chlorine, methyl, ethyl and

fluoro-$C_{1-2}$ alkyl, or the optionally substituted 5- or 6-membered heterocyclic ring is condensed with phenyl, the hetero atoms of the heterocyclic ring are selected from the group consisting of nitrogen, oxygen and sulfur, and the substituent of the heterocyclic or phenyl ring is selected from the group consisting of fluorine, chlorine, methyl, ethyl and fluoro-$C_{1-2}$ alkyl,

$R^8$ further represents $C_{1-2}$ alkoxy-carbonyl, aminocarbonyl or one of the following groups;

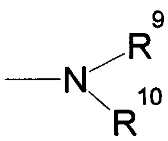
,
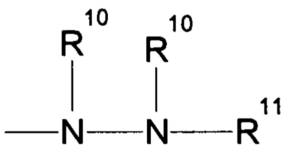
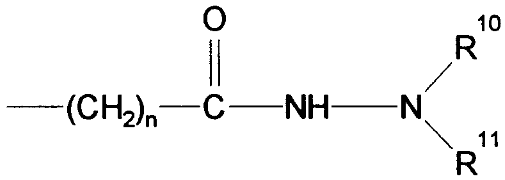

or

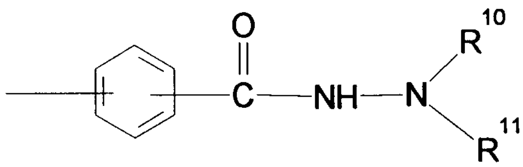

$R^9$ represents hydrogen, $C_{3-6}$ cycloalkyl which may be unsubstituted or substituted by $C_{1-2}$ alkyl, $C_{1-4}$ alkyl which may be unsubstituted or substituted by one of the group consisting of fluorine, chlorine, bromine and phenyl which may be substituted by one of the group consisting of nitro, cyano, fluorine, chlorine, bromine, $C_{1-2}$ alkyl, fluoro-$C_{1-2}$ alkyl and $C_{1-2}$ alkoxy,

$R^9$ further represents phenyl which may be unsubstituted or substituted by one of the group consisting of fluorine, chlorine, bromine, $C_{1-2}$ alkyl, $C_{1-2}$ alkoxy, fluoro-$C_{1-2}$ alkyl, fluoro-$C_{1-2}$ alkoxy, nitro and cyano,

$R^9$ further represents allyl, propargyl or benzenesulfonyl which may be unsubstituted or substituted by one of the group consisting of fluorine, chlorine, bromine, $C_{1-2}$ alkyl, $C_{1-2}$ alkoxy, fluoro-$C_{1-2}$ alkyl, nitro and cyano, or

$R^9$ further represents benzoyl which may be unsubstituted or substituted by one of the group consisting of fluorine, chlorine, bromine, $C_{1-2}$ alkyl, $C_{1-2}$ alkoxy, fluoro-$C_{1-2}$ alkyl, nitro and cyano,

$R^{10}$ represents hydrogen, $C_{1-4}$ alkyl, or phenyl, and if two $R^{10}$ are in the same compound then both $R^{10}$ may have the same or different meanings,

$R^{11}$ represents hydrogen, $C_{1-8}$ alkyl which may be unsubstituted or substituted by one of the group consisting of fluorine, chlorine, bromine and phenyl which may be unsubstituted or substituted by one of the group consisting of nitro, cyano, fluorine, chlorine, bromine, $C_{1-2}$ alkyl, fluoro-$C_{1-2}$ alkyl and $C_{1-2}$ alkoxy,

$R^{11}$ further represents phenyl which may be unsubstituted or substituted by one of the group consisting of fluorine, chlorine, bromine, $C_{1-2}$ alkyl, $C_{1-2}$ alkoxy, fluoro-$C_{1-2}$ alkyl, nitro and cyano,

or wherein $R^{10}$ and $R^{11}$ together may represent a group

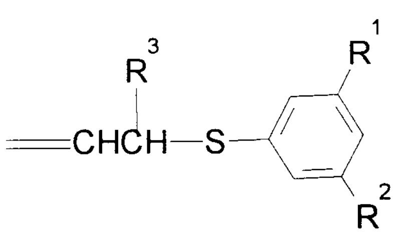

wherein $R^1$, $R^2$ and $R^3$ have the same meanings as mentioned above,
and

n represents an integer of 0 to 8.

Very particularly preferred hydrazones according to the invention of the formula (I) are those in which;

$R^1$ represents methoxy, difluoromethoxy or trifluoromethoxy,

$R^2$ represents methoxy, difluoromethoxy or trifluoromethoxy,

$R^3$ represents in each case optionally methyl substituted cyclopropyl, cyclopentyl or cyclohexyl,

$R^3$ further represents $C_{1-5}$ alkyl which may be unsubstituted or substituted by at least one substituent selected from the group consisting of chlorine, fluorine, cyclopentyl, cyclohexyl and phenyl which may be unsubstituted or substituted by at least one substituent selected from the group consisting of nitro, cyano, fluorine, chlorine, methyl, trifluoromethyl and methoxy, or

$R^3$ further represents phenyl which may be unsubstituted or substituted by at least one substituent selected from the group consisting of ethyl, methoxy, fluorine, chlorine, trifluoromethyl, nitro and cyano,

$R^4$ represents hydrogen, $C_{1-4}$ alkyl which may be substituted at least by one substituent selected from the group consisting of fluorine, chlorine and phenyl which may be unsubstituted or substituted by at least one substituent selected from the group consisting of nitro, cyano, fluorine, chlorine, methyl, trifluoromethyl and methoxy,

$R^4$ further represents allyl, propargyl or phenyl which may be substituted at least by one substituent selected from the group consisting of fluorine, chlorine, methyl, ethyl, methoxy, trifluoromethyl, trifluoromethoxy, nitro and cyano, pyridyl, furyl, thienyl, imidazolyl, pyrimidinyl, diazolyl and thiazolyl (those heterocyclic rings may be substituted at least by one substituent selected from the group consisting of fluorine, chlorine, methyl and trifluoromethyl,

$R^4$ further represents benzoxazolyl, benzothiazolyl, indolyl, quinolyl, phthalazinyl and benzimidazolyl (those condensed heterocyclic rings may be substituted at least by one substituent selected from the group consisting of fluorine, chlorine, methyl and trifluoromethyl, or one of the following groups:

$-SO_2-R^6$ and

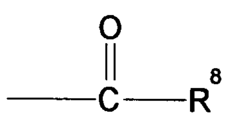

,

$R^5$ represents hydrogen, $C_{1-4}$ alkyl or phenyl, or

$R^4$ and $R^5$ together may represent a group

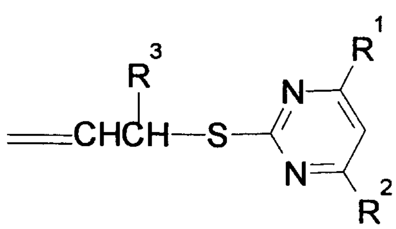

wherein $R^1$, $R^2$ and $R^3$ have the same meanings as mentioned above,

$R^6$ represents $C_{1-4}$ alkyl, phenyl which may be unsubstituted or substituted by one of the group consisting of fluorine, chlorine, methyl, methoxy and trifluoromethyl, or

$R^6$ further represents thiazolyl which may be unsubstituted or substituted by fluorine or chlorine,

$R^8$ represents $C_{1-4}$ alkyl, $C_{1-4}$ alkoxy, phenyl which may be substituted at least by one to five substituents selected from the group consisting of fluorine, chlorine, methyl, ethyl and methoxy, pyridyl, furyl, thienyl, indolyl, quinolyl, methoxy-carbonyl, or one of the following groups:

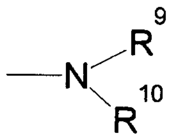
,
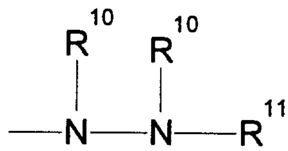
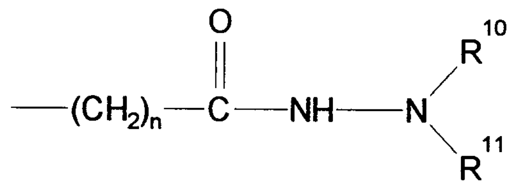

or
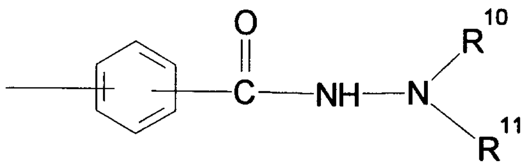

- $R^9$ represents hydrogen, in each case optionally by methyl substituted cyclopentyl and cyclohexyl,
- $R^9$ further represents $C_{1-4}$ alkyl which may be unsubstituted or substituted at least by one substituent selected from the group consisting of chlorine, fluorine and phenyl which may be unsubstituted or substituted at least by one substituent selected from the group consisting of fluorine, chlorine and methyl,
- $R^9$ further represents phenyl which may be unsubstituted or substituted at least by one substituent selected from the group consisting of fluorine, chlorine, methyl, ethyl, methoxy and trifluoromethyl,
- $R^9$ further represents allyl, propargyl or benzenesulfonyl which may be unsubstituted or substituted at least by one substituent selected from the group consisting of chlorine, fluorine, methyl, ethyl and methoxy,
- $R^9$ further represents benzoyl which may be unsubstituted or substituted at least by one substituent selected from the group consisting of chlorine, fluorine, methyl, ethyl and methoxy,
- $R^{10}$ represents hydrogen, methyl, ethyl, phenyl, and if two $R^{10}$ are in the same compound then both $R^{10}$ have the same or different meanings,
- $R^{11}$ represents hydrogen, $C_{1-4}$ alkyl which may be unsubstituted or substituted at least by one substituent selected from the group consisting of chlorine, fluorine and phenyl which may be unsubstituted or substituted at least by one substituent selected from the group consisting of fluorine, chlorine, methyl, trifluoromethyl and methoxy,
- $R^{11}$ further represents phenyl which may be unsubstituted or substituted at least by one substituent selected from the group consisting of fluorine, chlorine, methyl, ethyl, methoxy and trifluoromethyl,

or wherein $R^{10}$ and $R^{11}$ together may represent a group

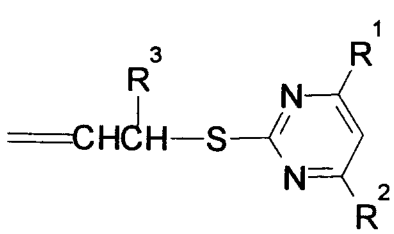

wherein $R^1$, $R^2$ and $R^3$ have the same meanings as mentioned above,
and

- n represents 0,4 or 8.

As examples of the compounds of the formula (I) according to the invention, there may be mentioned in the following table 1, in addition to the compounds that will be enumerated in the Examples hereinafter.

Table 1

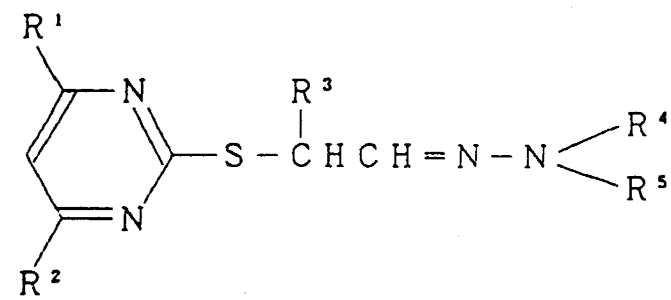

| $R^1$ | $R^2$ | $R^3$ | $R^4$ | $R^5$ |
|---|---|---|---|---|
| $-OCH_3$ | $-OCH_3$ | $-CH_3$ | H | H |
| $-OCH_3$ | $-OCH_3$ | $-C_2H_5$ | H | H |
| $-OCH_3$ | $-OCH_3$ | $-CH_2CH_2CH_3$ | H | H |
| $-OCH_3$ | $-OCH_3$ | $-CH(CH_3)_2$ | H | H |
| $-OCH_3$ | $-OCH_3$ | $-CH_2CH_2CH_2CH_3$ | H | H |
| $-OCH_3$ | $-OCH_3$ | $-CH(CH_3)CH_2CH_3$ | H | H |
| $-OCH_3$ | $-OCH_3$ | $-CH_2CH(CH_3)_2$ | H | H |
| $-OCH_3$ | $-OCH_3$ | $-C(CH_3)_3$ | H | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2Cl$ | H | H |
| $-OCH_3$ | $-OCH_3$ | $-CH_2CF_3$ | H | H |
| $-OCH_3$ | $-OCH_3$ | 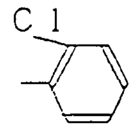 | H | H |
| $-OCH_3$ | $-OCH_3$ | 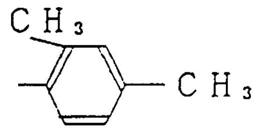 | H | H |
| $-OCH_3$ | $-OCH_3$ | 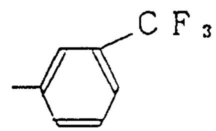 | H | H |
| $-OCH_3$ | $-OCH_3$ | 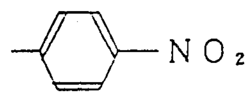 | H | H |
| $-OCH_3$ | $-OCH_3$ | 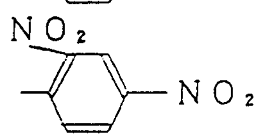 | H | H |
| $-OCH_3$ | $-OCH_3$ | 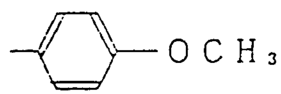 | H | H |

Table 1 (Continued)

| $R^1$ | $R^2$ | $R^3$ | $R^4$ | $R^5$ |
|---|---|---|---|---|
| $-OCH_3$ | $-OCH_3$ | 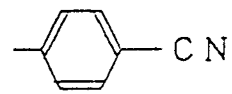 | H | H |
| $-OCH_3$ | $-OCH_3$ | 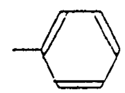 | H | H |
| $-OCH_3$ | $-OCH_3$ | 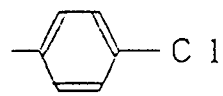 | H | H |
| $-OCH_3$ | $-OCH_3$ | 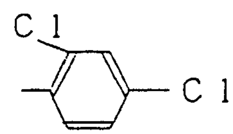 | H | H |
| $-OCH_3$ | $-OCH_3$ | 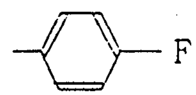 | H | H |
| $-OCH_3$ | $-OCH_3$ | 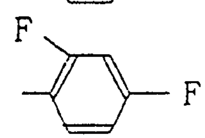 | H | H |
| $-OCH_3$ | $-OCH_3$ | 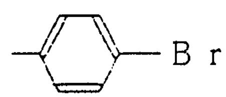 | H | H |

Table 1 (Continued)

| $R^1$ | $R^2$ | $R^3$ | $R^4$ | $R^5$ |
|---|---|---|---|---|
| $-OCH_3$ | $-OCH_3$ | 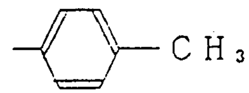 | H | H |
| $-OCH_3$ | $-OCH_3$ | 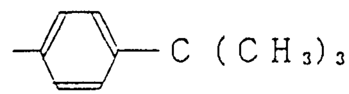 | H | H |
| $-OCH_3$ | $-OCH_3$ | 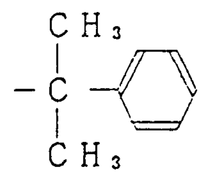 | H | H |
| $-OCH_3$ | $-OCH_3$ | 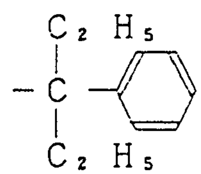 | H | H |
| | | | H | H |
| $-OCH_3$ | $-OCH_3$ | 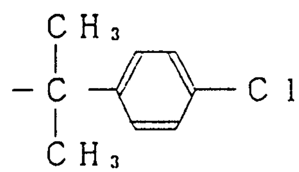 | H | H |

Table 1 (Continued)

| $R^1$ | $R^2$ | $R^3$ | $R^4$ | $R^5$ |
|---|---|---|---|---|
| $-OCH_3$ | $-OCH_3$ | 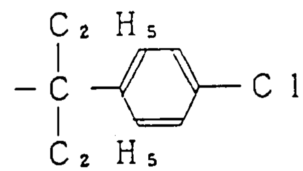 | H | H |
| $-OCH_3$ | $-OCH_3$ | 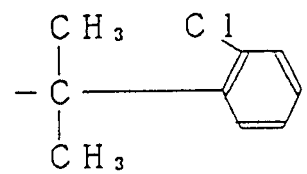 | H | H |
| $-OCH_3$ | $-OCH_3$ | 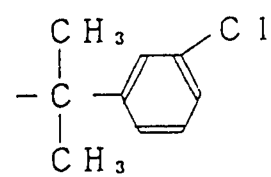 | H | H |
| $-OCH_3$ | $-OCH_3$ | 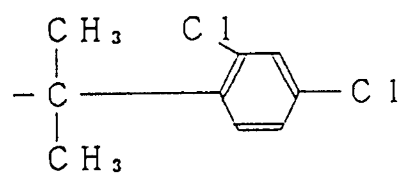 | H | H |

Table 1 (Continued)

| $R^1$ | $R^2$ | $R^3$ | $R^4$ | $R^5$ |
|---|---|---|---|---|
| $-OCH_3$ | $-OCH_3$ | 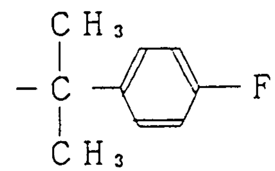 | H | H |
| $-OCH_3$ | $-OCH_3$ | 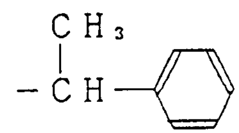 | H | H |
| $-OCH_3$ | $-OCH_3$ | 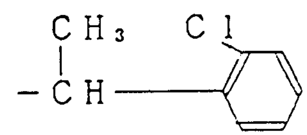 | H | H |
| $-OCH_3$ | $-OCH_3$ | 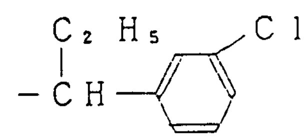 | H | H |

Table 1 (Continued)

| $R^1$ | $R^2$ | $R^3$ | $R^4$ | $R^5$ |
|---|---|---|---|---|
| $-OCH_3$ | $-OCH_3$ | 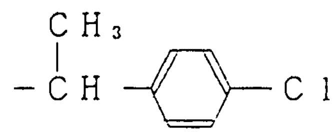 | H | H |
| $-OCH_3$ | $-OCH_3$ | 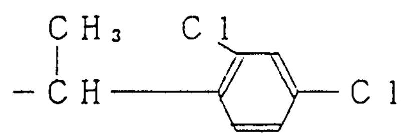 | H | H |
| $-OCH_3$ | $-OCH_3$ | 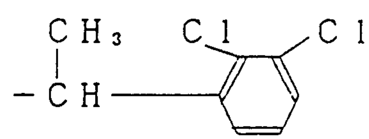 | H | H |
| $-OCH_3$ | $-OCH_3$ | 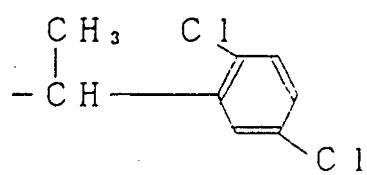 | H | H |

Table 1 (Continued)

| $R^1$ | $R^2$ | $R^3$ | $R^4$ | $R^5$ |
|---|---|---|---|---|
| $-OCH_3$ | $-OCH_3$ | 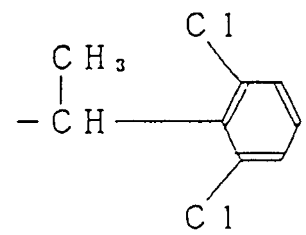 | H | H |
| $-OCH_3$ | $-OCH_3$ | 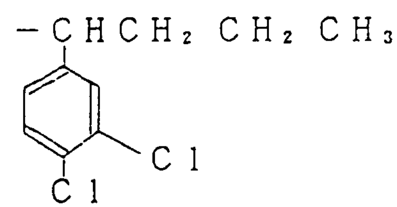 | H | H |
| $-OCH_3$ | $-OCH_3$ | 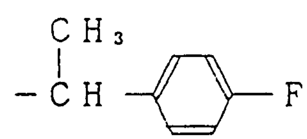 | H | H |
| $-OCH_3$ | $-OCH_3$ | 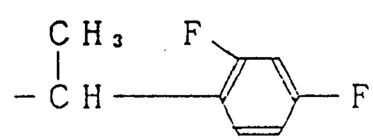 | H | H |

Table 1 (Continued)

| $R^1$ | $R^2$ | $R^3$ | $R^4$ | $R^5$ |
|---|---|---|---|---|
| $-OCH_3$ | $-OCH_3$ | 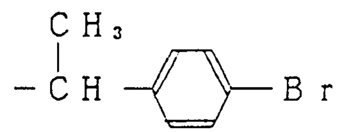 | H | H |
| $-OCH_3$ | $-OCH_3$ | 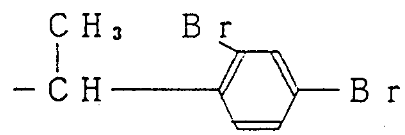 | H | H |
| $-OCH_3$ | $-OCH_3$ | 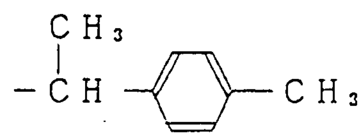 | H | H |
| $-OCH_3$ | $-OCH_3$ | 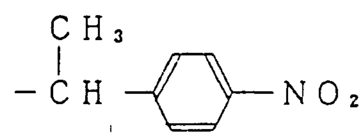 | H | H |
| $-OCH_3$ | $-OCH_3$ | 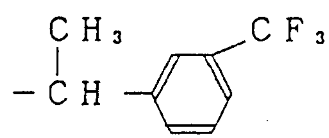 | H | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2-$ 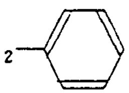 | H | H |
| $-OCH_3$ | $-OCH_3$ | $-CH_2-$ 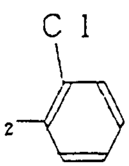 | H | H |
| $-OCH_3$ | $-OCH_3$ | $-CH_2-$ 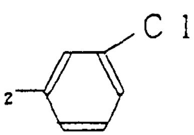 | H | H |
| $-OCH_3$ | $-OCH_3$ | $-CH_2-$ 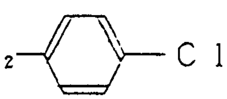 | H | H |
| $-OCH_3$ | $-OCH_3$ | $-CH_2-$ 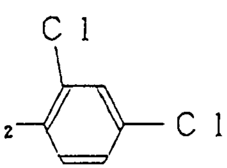 | H | H |

Table 1 (Continued)

| $R^1$ | $R^2$ | $R^3$ | $R^4$ | $R^5$ |
|---|---|---|---|---|
| $-OCH_3$ | $-OCH_3$ | 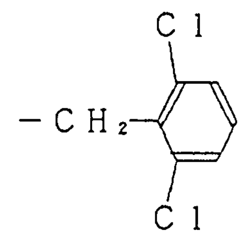 | H | H |
| $-OCH_3$ | $-OCH_3$ | 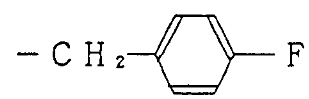 | H | H |
| $-OCH_3$ | $-OCH_3$ | 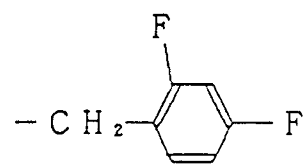 | H | H |
| $-OCH_3$ | $-OCH_3$ | 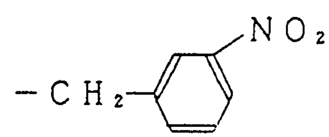 | H | H |

Table 1 (Continued)

| $R^1$ | $R^2$ | $R^3$ | $R^4$ | $R^5$ |
|---|---|---|---|---|
| $-OCH_3$ | $-OCH_3$ | 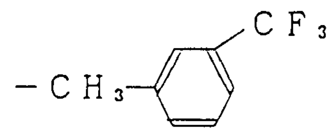 | H | H |
| $-OCH_3$ | $-OCH_3$ | 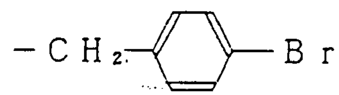 | H | H |
| $-OCH_3$ | $-OCH_3$ | 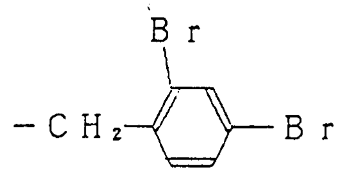 | H | H |
| $-OCH_3$ | $-OCH_3$ | 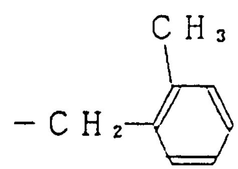 | H | H |
| $-OCH_3$ | $-OCH_3$ | 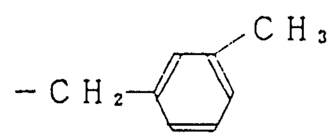 | H | H |

Table 1 (Continued)

| $R^1$ | $R^2$ | $R^3$ | $R^4$ | $R^5$ |
|---|---|---|---|---|
| $-OCH_3$ | $-OCH_3$ | 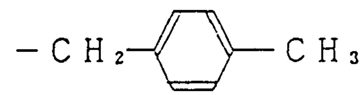 | H | H |
| $-OCH_3$ | $-OCH_3$ | 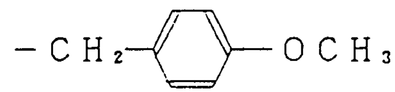 | H | H |
| $-OCH_3$ | $-OCH_3$ | 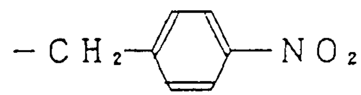 | H | H |
| $-OCH_3$ | $-OCH_3$ | 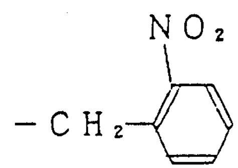 | H | H |
| $-OCH_3$ | $-OCH_3$ | 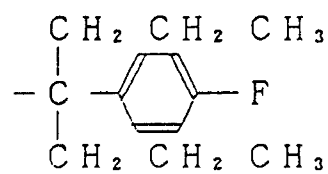 | H | H |

Table 1 (Continued)

| $R^1$ | $R^2$ | $R^3$ | $R^4$ | $R^5$ |
|---|---|---|---|---|
| $-OCH_3$ | $-OCH_3$ | 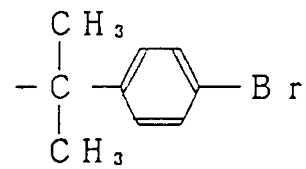 | H | H |
| $-OCH_3$ | $-OCH_3$ | 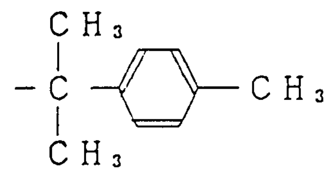 | H | H |
| $-OCH_3$ | $-OCH_3$ | 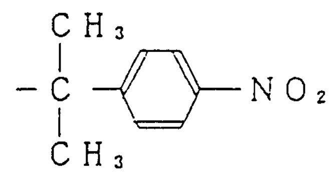 | H | H |
| $-OCH_3$ | $-OCH_3$ | 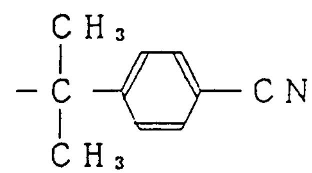 | H | H |

Table 1 (Continued)

| $R^1$ | $R^2$ | $R^3$ | $R^4$ | $R^5$ |
|---|---|---|---|---|
| $-OCH_3$ | $-OCH_3$ | 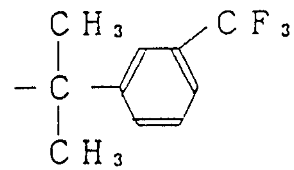 | H | H |
| $-OCH_3$ | $-OCH_3$ | 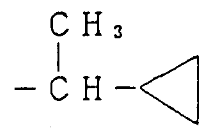 | H | H |
| $-OCH_3$ | $-OCH_3$ | 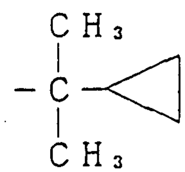 | H | H |
| $-OCH_3$ | $-OCH_3$ | 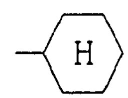 | H | H |
| $-OCH_3$ | $-OCH_3$ | 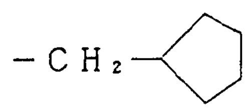 | H | H |

Table 1 (Continued)

| $R^1$ | $R^2$ | $R^3$ | $R^4$ | $R^5$ |
|---|---|---|---|---|
| $-OCH_3$ | $-OCH_3$ | 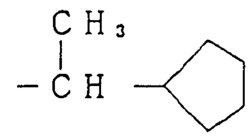 | H | H |
| $-OCH_3$ | $-OCH_3$ | 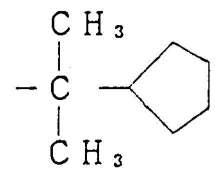 | H | H |
| $-OCH_3$ | $-OCH_3$ | 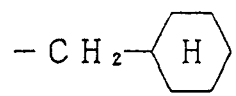 | H | H |
| $-OCH_3$ | $-OCH_3$ | 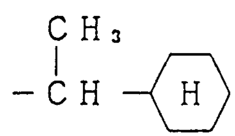 | H | H |
| $-OCH_3$ | $-OCH_3$ | 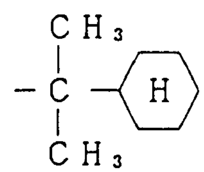 | H | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2-$ 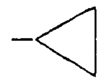 | H | H |
| $-OCH_3$ | $-OCH_3$ | $-CH(CH_3)(CH_2)_{12}CH_3$ | H | H |
| $-OCH_3$ | $-OCH_3$ | 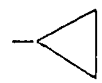 | H | H |
| $-OCH_3$ | $-OCH_3$ | 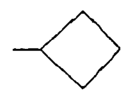 | H | H |
| $-OCH_3$ | $-OCH_3$ | 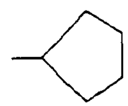 | H | H |
| $-OCH_3$ | $-OCH_3$ | 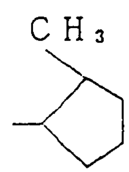 | H | H |

Table 1 (Continued)

| $R^1$ | $R^2$ | $R^3$ | $R^4$ | $R^5$ |
|---|---|---|---|---|
| $-OCH_3$ | $-OCH_3$ | 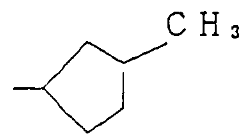 | H | H |
| $-OCH_3$ | $-OCH_3$ | 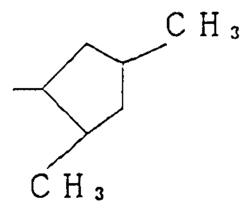 | H | H |
| $-OCH_3$ | $-OCH_3$ | $-\overset{CH_3}{\overset{\vert}{C}}H(CH_2)_5CH_3$ | H | H |
| $-OCH_3$ | $-OCH_3$ | $-(CH_2)_8CH_3$ | H | H |
| $-OCH_3$ | $-OCH_3$ | $-\overset{CH_3}{\overset{\vert}{C}}H(CH_2)_6CH_3$ | H | H |
| $-OCH_3$ | $-OCH_3$ | $-\overset{CH_3}{\overset{\vert}{C}}H(CH_2)_7CH_3$ | H | H |

Table 1 (Continued)

| $R^1$ | $R^2$ | $R^3$ | $R^4$ | $R^5$ |
|---|---|---|---|---|
| $-OCH_3$ | $-OCH_3$ | $CH_3$<br>\|<br>$-CH(CH_2)_8CH_3$ | H | H |
| $-OCH_3$ | $-OCH_3$ | $CH_3$<br>\|<br>$-CH(CH_2)_9CH_3$ | H | H |
| $-OCH_3$ | $-OCH_3$ | $CH_3$<br>\|<br>$-CH(CH_2)_{10}CH_3$ | H | H |
| $-OCH_3$ | $-OCH_3$ | $CH_3$<br>\|<br>$-CH(CH_2)_{11}CH_3$ | H | H |
| $-OCH_3$ | $-OCH_3$ | $CH_3$<br>\|<br>$-C-CH_2CH_2CH_3$<br>\|<br>$CH_3$ | H | H |

Table 1 (Continued)

| $R^1$ | $R^2$ | $R^3$ | $R^4$ | $R^5$ |
|---|---|---|---|---|
| $-OCH_3$ | $-OCH_3$ | $-C(C_2H_5)_2-CH_3$ | H | H |
| $-OCH_3$ | $-OCH_3$ | $-(CH_2)_6CH_3$ | H | H |
| $-OCH_3$ | $-OCH_3$ | $-CH(CH_3)(CH_2)_3CH_3$ | H | H |
| $-OCH_3$ | $-OCH_3$ | $-CH(CH_3)(CH_2)_4CH_3$ | H | H |
| $-OCH_3$ | $-OCH_3$ | $-CH(CH_3)CH_2C(CH_3)_2CH_3$ | H | H |
| $-OCH_3$ | $-OCH_3$ | $-(CH_2)_7CH_3$ | H | H |
| $-OCH_3$ | $-OCH_3$ | $-(CH_2)_4CH_3$ | H | H |

Table 1 (Continued)

| $R^1$ | $R^2$ | $R^3$ | $R^4$ | $R^5$ |
|---|---|---|---|---|
| $-OCH_3$ | $-OCH_3$ | 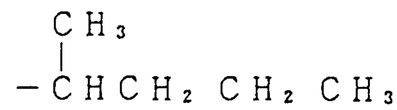 | H | H |
| $-OCH_3$ | $-OCH_3$ | 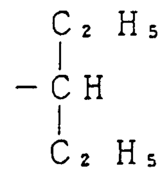 | H | H |
| $-OCH_3$ | $-OCH_3$ | 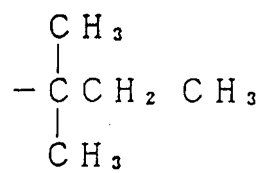 | H | H |
| $-OCH_3$ | $-OCH_3$ | 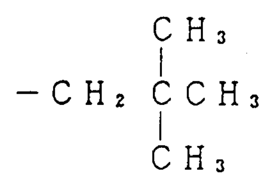 | H | H |
| $-OCH_3$ | $-OCH_3$ | 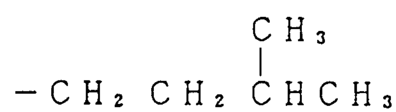 | H | H |

Table 1 (Continued)

| $R^1$ | $R^2$ | $R^3$ | $R^4$ | $R^5$ |
|---|---|---|---|---|
| $-OCH_3$ | $-OCH_3$ | $-(CH_2)_5CH_3$ | H | H |
| $-OCH_3$ | $-OCH_3$ | $-C_2H_5$ | $-CH_3$ | H |
| $-OCH_3$ | $-OCH_3$ | $-C_2H_5$ | $-C_2H_5$ | H |
| $-OCH_3$ | $-OCH_3$ | $-C_2H_5$ | $-CH_2CH_2CH_3$ | H |
| $-OCH_3$ | $-OCH_3$ | $-C_2H_5$ | $-CH(CH_3)_2$ | H |
| $-OCH_3$ | $-OCH_3$ | $-C_2H_5$ | $-CH_2CH_2CH_2CH_3$ | H |
| $-OCH_3$ | $-OCH_3$ | $-C_2H_5$ | $-CH(CH_3)CH_2CH_3$ | H |
| $-OCH_3$ | $-OCH_3$ | $-C_2H_5$ | $-CH_2CH(CH_3)_2$ | H |
| $-OCH_3$ | $-OCH_3$ | $-C_2H_5$ | $-C(CH_3)_3$ | H |
| $-OCH_3$ | $-OCH_3$ | $-C_2H_5$ | $-CH_3$ | $-CH_3$ |
| $-OCH_3$ | $-OCH_3$ | $-C_2H_5$ | $-C_2H_5$ | $-CH_3$ |
| $-OCH_3$ | $-OCH_3$ | $-C_2H_5$ | $-CH_2CH_2CH_3$ | $-CH_3$ |
| $-OCH_3$ | $-OCH_3$ | $-C_2H_5$ | $-CH(CH_3)_2$ | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | $-CH_2CH_2CH_2CH_3$ | $-CH_3$ |
| $-OCH_3$ | $-OCH_3$ | $-C_2H_5$ | $-CH(CH_3)CH_2CH_3$ | $-CH_3$ |
| $-OCH_3$ | $-OCH_3$ | $-C_2H_5$ | $-CH_2CH(CH_3)_2$ | $-CH_3$ |
| $-OCH_3$ | $-OCH_3$ | $-C_2H_5$ | $-C(CH_3)_3$ | $-CH_3$ |
| $-OCH_3$ | $-OCH_3$ | $-C_2H_5$ | $-C_2H_5$ | $-C_2H_5$ |
| $-OCH_3$ | $-OCH_3$ | $-C_2H_5$ | $-CH_2CH_2CH_3$ | $-C_2H_5$ |
| $-OCH_3$ | $-OCH_3$ | $-C_2H_5$ | $-CH(CH_3)_2$ | $-C_2H_5$ |
| $-OCH_3$ | $-OCH_3$ | $-C_2H_5$ | $-CH_2CH_2CH_2CH_3$ | $-C_2H_5$ |
| $-OCH_3$ | $-OCH_3$ | $-C_2H_5$ | $-CH(CH_3)CH_2CH_3$ | $-C_2H_5$ |
| $-OCH_3$ | $-OCH_3$ | $-C_2H_5$ | $-CH_2CH(CH_3)_2$ | $-C_2H_5$ |
| $-OCH_3$ | $-OCH_3$ | $-C_2H_5$ | $-C(CH_3)_3$ | $-C_2H_5$ |
| $-OCH_3$ | $-OCH_3$ | $-C_2H_5$ | $-CH_2CH=CH_2$ | H |
| $-OCH_3$ | $-OCH_3$ | $-C_2H_5$ | $-CH_2C\equiv CH$ | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 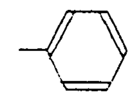 | H |
| $-OCH_3$ | $-OCH_3$ | $-C_2H_5$ | 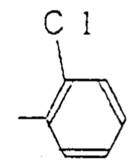 | H |
| $-OCH_3$ | $-OCH_3$ | $-C_2H_5$ | 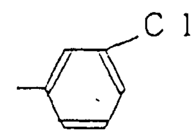 | H |
| $-OCH_3$ | $-OCH_3$ | $-C_2H_5$ | 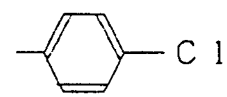 | H |
| $-OCH_3$ | $-OCH_3$ | $-C_2H_5$ | 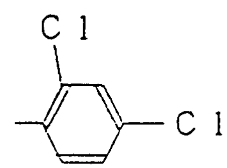 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 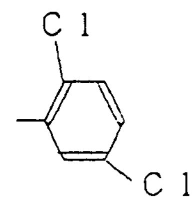 | H |
| $-OCH_3$ | $-OCH_3$ | $-C_2H_5$ | 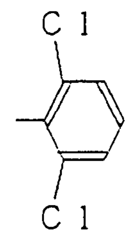 | H |
| $-OCH_3$ | $-OCH_3$ | $-C_2H_5$ | 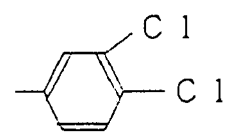 | H |
| $-OCH_3$ | $-OCH_3$ | $-C_2H_5$ | 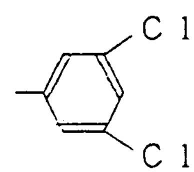 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 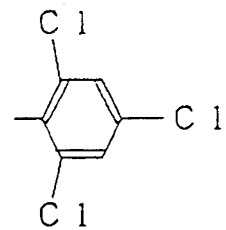 | H |
| $-OCH_3$ | $-OCH_3$ | $-C_2H_5$ | 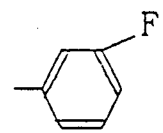 | H |
| $-OCH_3$ | $-OCH_3$ | $-C_2H_5$ | 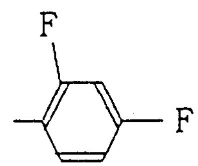 | H |
| $-OCH_3$ | $-OCH_3$ | $-C_2H_5$ | 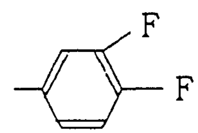 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 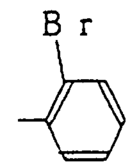 | H |
| $-OCH_3$ | $-OCH_3$ | $-C_2H_5$ | 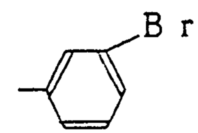 | H |
| $-OCH_3$ | $-OCH_3$ | $-C_2H_5$ | 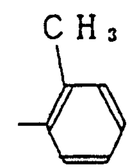 | H |
| $-OCH_3$ | $-OCH_3$ | $-C_2H_5$ | 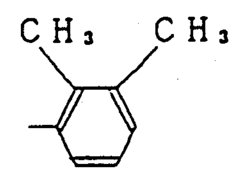 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 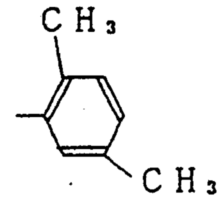 | H |
| $-OCH_3$ | $-OCH_3$ | $-C_2H_5$ | 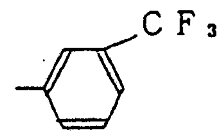 | H |
| $-OCH_3$ | $-OCH_3$ | $-C_2H_5$ | 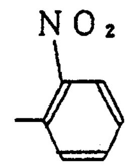 | H |
| $-OCH_3$ | $-OCH_3$ | $-C_2H_5$ | 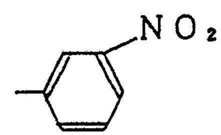 | H |
| $-OCH_3$ | $-OCH_3$ | $-C_2H_5$ | 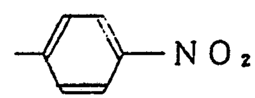 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 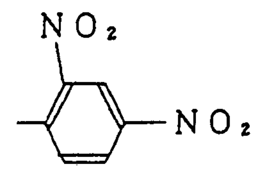 | H |
| $-OCH_3$ | $-OCH_3$ | $-C_2H_5$ | 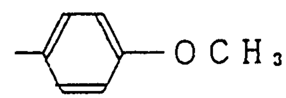 | H |
| $-OCH_3$ | $-OCH_3$ | $-C_2H_5$ | 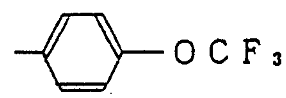 | H |
| $-OCH_3$ | $-OCH_3$ | $-C_2H_5$ | 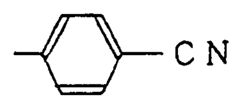 | H |
| $-OCH_3$ | $-OCH_3$ | $-C_2H_5$ | 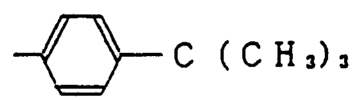 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 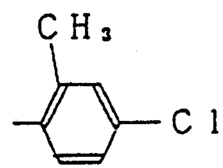 | H |
| $-OCH_3$ | $-OCH_3$ | $-C_2H_5$ | 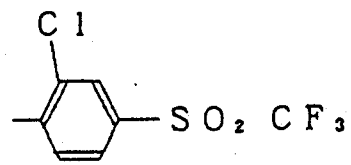 | H |
| $-OCH_3$ | $-OCH_3$ | $-C_2H_5$ | 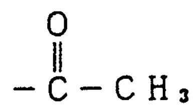 | H |
| $-OCH_3$ | $-OCH_3$ | $-C_2H_5$ | 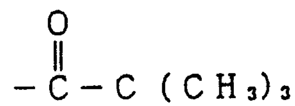 | H |
| $-OCH_3$ | $-OCH_3$ | $-C_2H_5$ | 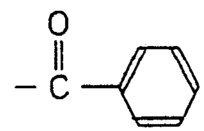 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 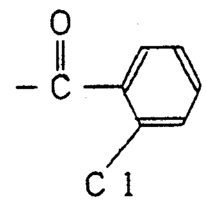 | H |
| $-OCH_3$ | $-OCH_3$ | $-C_2H_5$ | 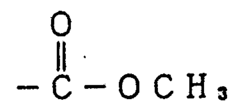 | H |
| $-OCH_3$ | $-OCH_3$ | $-C_2H_5$ | 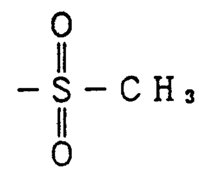 | H |
| $-OCH_3$ | $-OCH_3$ | $-C_2H_5$ | 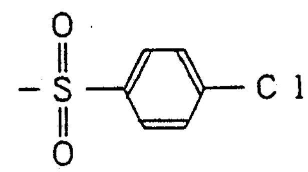 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_3$ | $-CH_3$ | H |
| $-OCH_3$ | $-OCH_3$ | $-CH_2CH_2CH_3$ | $-C_2H_5$ | H |
| $-OCH_3$ | $-OCH_3$ | $-CH_2CH_2CH_3$ | $-CH_2CH_2CH_3$ | H |
| $-OCH_3$ | $-OCH_3$ | $-CH_2CH_2CH_3$ | $-CH(CH_3)_2$ | H |
| $-OCH_3$ | $-OCH_3$ | $-CH_2CH_2CH_3$ | $-CH_2CH_2CH_2CH_3$ | H |
| $-OCH_3$ | $-OCH_3$ | $-CH_2CH_2CH_3$ | $-CH(CH_3)CH_2CH_3$ | H |
| $-OCH_3$ | $-OCH_3$ | $-CH_2CH_2CH_3$ | $-CH_2CH(CH_3)_2$ | H |
| $-OCH_3$ | $-OCH_3$ | $-CH_2CH_2CH_3$ | $-C(CH_3)_3$ | H |
| $-OCH_3$ | $-OCH_3$ | $-CH_2CH_2CH_3$ | $-CH_3$ | $-CH_3$ |
| $-OCH_3$ | $-OCH_3$ | $-CH_2CH_2CH_3$ | $-C_2H_5$ | $-CH_3$ |
| $-OCH_3$ | $-OCH_3$ | $-CH_2CH_2CH_3$ | $-CH_2CH_2CH_3$ | $-CH_3$ |
| $-OCH_3$ | $-OCH_3$ | $-CH_2CH_2CH_3$ | $-C(CH_3)_3$ | $-CH_3$ |
| $-OCH_3$ | $-OCH_3$ | $-CH_2CH_2CH_3$ | $-C_2H_5$ | $-C_2H_5$ |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_3$ | $-CH_2CH_2CH_3$ | $-C_2H_5$ |
| $-OCH_3$ | $-OCH_3$ | $-CH_2CH_2CH_3$ | $-C(CH_3)_3$ | $-C_2H_5$ |
| $-OCH_3$ | $-OCH_3$ | $-CH_2CH_2CH_3$ | $-CH_2CH{=}CH_2$ | H |
| $-OCH_3$ | $-OCH_3$ | $-CH_2CH_2CH_3$ | $-CH_2CH{=}CH_2$ | H |
| $-OCH_3$ | $-OCH_3$ | $-CH_2CH_2CH_3$ | 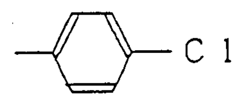 | H |
| $-OCH_3$ | $-OCH_3$ | $-CH_2CH_2CH_3$ | 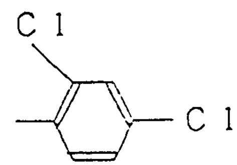 | H |
| $-OCH_3$ | $-OCH_3$ | $-CH_2CH_2CH_3$ | 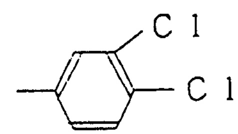 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_3$ | 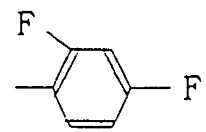 | H |
| $-OCH_3$ | $-OCH_3$ | $-CH_2CH_2CH_3$ | 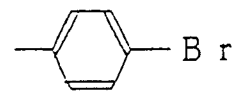 | H |
| $-OCH_3$ | $-OCH_3$ | $-CH_2CH_2CH_3$ | 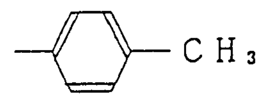 | H |
| $-OCH_3$ | $-OCH_3$ | $-CH_2CH_2CH_3$ | 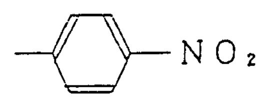 | H |
| $-OCH_3$ | $-OCH_3$ | $-CH_2CH_2CH_3$ | 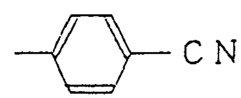 | H |
| $-OCH_3$ | $-OCH_3$ | $-CH_2CH_2CH_3$ | 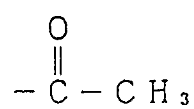 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_3$ | 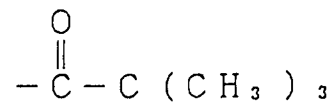 | H |
| $-OCH_3$ | $-OCH_3$ | $-CH_2CH_2CH_3$ | 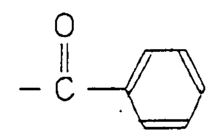 | H |
| $-OCH_3$ | $-OCH_3$ | $-CH_2CH_2CH_3$ | 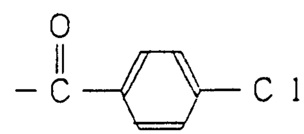 | H |
| $-OCH_3$ | $-Cl$ | $-CH(CH_3)_2$ | H | $-CH_3$ |
| $-OCH_3$ | $-Cl$ | $-C(CH_3)_3$ | H | H |
| $-OCF_3$ | $-OCF_3$ | $-CH(CH_3)_2$ | H | $-C(CH_3)_3$ |
| $-OCF_3$ | $-OCF_3$ | $-C(CH_3)_3$ | H | $-CH_3$ |
| $-OCHF_2$ | $-OCHF_2$ | $-CH(CH_3)_2$ | H | 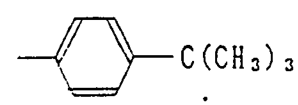 |

Table 1 (Continued)

| $R^1$ | $R^2$ | $R^3$ | $R^4$ | $R^5$ |
|---|---|---|---|---|
| $-OCHF_2$ | $-OCHF_2$ | $-C(CH_3)_3$ | $-CH_3$ | $-CH_3$ |
| $-CH_3$ | $Cl$ | $-CH(CH_3)_2$ | H | H |
| $-CH_3$ | $-OCH_3$ | $-C(CH_3)_3$ | H | 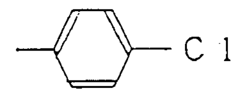 |
| $-OCH_3$ | $-OCH_3$ | $-CH(CH_3)_2$ | $-CH_3$ | H |
| $-OCH_3$ | $-OCH_3$ | $-CH(CH_3)_2$ | $-C_2H_5$ | H |
| $-OCH_3$ | $-OCH_3$ | $-CH(CH_3)_2$ | $-CH_2CH_2CH_3$ | H |
| $-OCH_3$ | $-OCH_3$ | $-CH(CH_3)_2$ | $-CH(CH_3)_2$ | H |
| $-OCH_3$ | $-OCH_3$ | $-CH(CH_3)_2$ | $-CH_2CH_2CH_2CH_3$ | H |
| $-OCH_3$ | $-OCH_3$ | $-CH(CH_3)_2$ | $-CH(CH_3)CH_2CH_3$ | H |
| $-OCH_3$ | $-OCH_3$ | $-CH(CH_3)_2$ | $-CH_2CH(CH_3)_2$ | H |
| $-OCH_3$ | $-OCH_3$ | $-CH(CH_3)_2$ | $-C(CH_3)_3$ | H |
| $-OCH_3$ | $-OCH_3$ | $-CH(CH_3)_2$ | $-CH_3$ | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | $-C_2H_5$ | $-CH_3$ |
| $-OCH_3$ | $-OCH_3$ | $-CH(CH_3)_2$ | $-CH_2CH_2CH_3$ | $-CH_3$ |
| $-OCH_3$ | $-OCH_3$ | $-CH(CH_3)_2$ | $-CH(CH_3)_2$ | $-CH_3$ |
| $-OCH_3$ | $-OCH_3$ | $-CH(CH_3)_2$ | $-CH_2CH_2CH_2CH_3$ | $-CH_3$ |
| $-OCH_3$ | $-OCH_3$ | $-CH(CH_3)_2$ | $-CH(CH_3)CH_2CH_3$ | $-CH_3$ |
| $-OCH_3$ | $-OCH_3$ | $-CH(CH_3)_2$ | $-CH_2CH(CH_3)_2$ | $-CH_3$ |
| $-OCH_3$ | $-OCH_3$ | $-CH(CH_3)_2$ | $-C(CH_3)_3$ | $-CH_3$ |
| $-OCH_3$ | $-OCH_3$ | $-CH(CH_3)_2$ | $-C_2H_5$ | $-C_2H_5$ |
| $-OCH_3$ | $-OCH_3$ | $-CH(CH_3)_2$ | $-CH_2CH_2CH_3$ | $-C_2H_5$ |
| $-OCH_3$ | $-OCH_3$ | $-CH(CH_3)_2$ | $-CH(CH_3)_2$ | $-C_2H_5$ |
| $-OCH_3$ | $-OCH_3$ | $-CH(CH_3)_2$ | $-CH_2CH_2CH_2CH_3$ | $-C_2H_5$ |
| $-OCH_3$ | $-OCH_3$ | $-CH(CH_3)_2$ | $-CH(CH_3)CH_2CH_3$ | $-C_2H_5$ |
| $-OCH_3$ | $-OCH_3$ | $-CH(CH_3)_2$ | $-CH_2CH(CH_3)_2$ | $-C_2H_5$ |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | $-CH_2CH_2CH_2CH_2CH_3$ | H |
| $-OCH_3$ | $-OCH_3$ | $-CH(CH_3)_2$ | $-CH_2CH=CH_2$ | H |
| $-OCH_3$ | $-OCH_3$ | $-CH(CH_3)_2$ | $-CH_2C\equiv CH$ | H |
| $-OCH_3$ | $-OCH_3$ | $-CH(CH_3)_2$ | $-CH_2CH_2CH_2CH_2CH_2CH_3$ | H |
| $-OCH_3$ | $-OCH_3$ | $-CH(CH_3)_2$ | 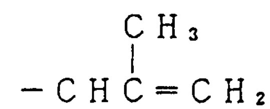 | H |
| $-OCH_3$ | $-OCH_3$ | $-CH(CH_3)_2$ | 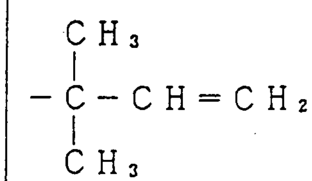 | H |
| $-OCH_3$ | $-OCH_3$ | $-CH(CH_3)_2$ | 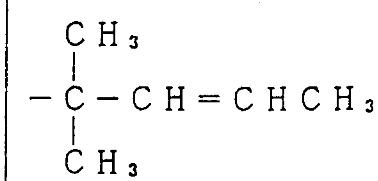 | H |
| $-OCH_3$ | $-OCH_3$ | $-CH(CH_3)_2$ | $-CH_2C\equiv CCH_2CH_2CH_3$ | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 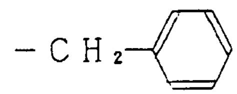 | H |
| $-OCH_3$ | $-OCH_3$ | $-CH(CH_3)_2$ | 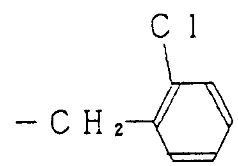 | H |
| $-OCH_3$ | $-OCH_3$ | $-CH(CH_3)_2$ | 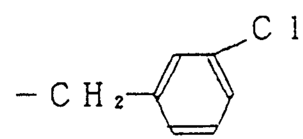 | $-CH_3$ |
| $-OCH_3$ | $-OCH_3$ | $-CH(CH_3)_2$ | 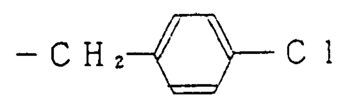 | H |
| $-OCH_3$ | $-OCH_3$ | $-CH(CH_3)_2$ | 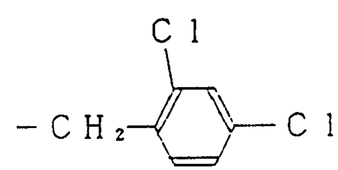 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 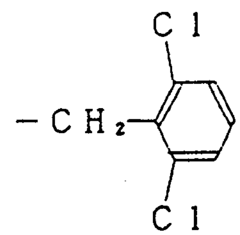 | H |
| $-OCH_3$ | $-OCH_3$ | $-CH(CH_3)_2$ | 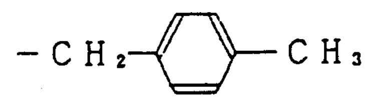 | H |
| $-OCH_3$ | $-OCH_3$ | $-CH(CH_3)_2$ | 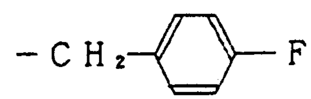 | H |
| $-OCH_3$ | $-OCH_3$ | $-CH(CH_3)_2$ | 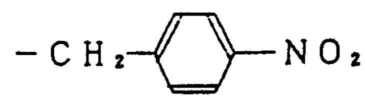 | H |
| $-OCH_3$ | $-OCH_3$ | $-CH(CH_3)_2$ | 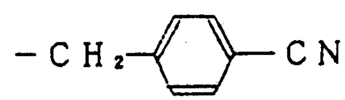 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 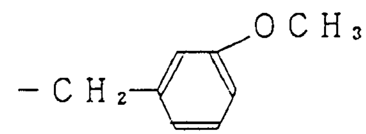 | H |
| $-OCH_3$ | $-OCH_3$ | $-CH(CH_3)_2$ | 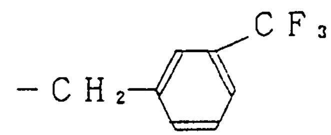 | H |
| $-OCH_3$ | $-OCH_3$ | $-CH(CH_3)_2$ | 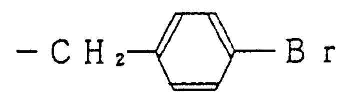 | H |
| $-OCH_3$ | $-OCH_3$ | $-CH(CH_3)_2$ | 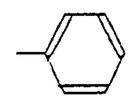 | H |
| $-OCH_3$ | $-OCH_3$ | $-CH(CH_3)_2$ | 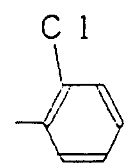 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 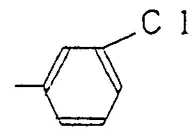 | H |
| $-OCH_3$ | $-OCH_3$ | $-CH(CH_3)_2$ | 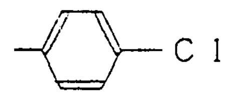 | H |
| $-OCH_3$ | $-OCH_3$ | $-CH(CH_3)_2$ | 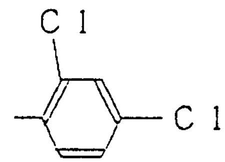 | H |
| $-OCH_3$ | $-OCH_3$ | $-CH(CH_3)_2$ | 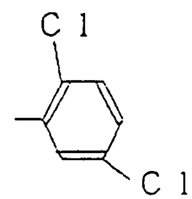 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 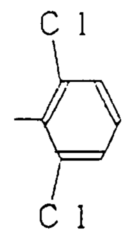 | H |
| $-OCH_3$ | $-OCH_3$ | $-CH(CH_3)_2$ | 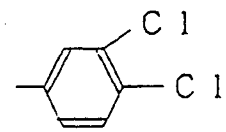 | H |
| $-OCH_3$ | $-OCH_3$ | $-CH(CH_3)_2$ | 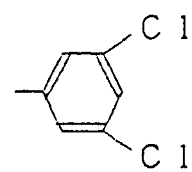 | H |
| $-OCH_3$ | $-OCH_3$ | $-CH(CH_3)_2$ | 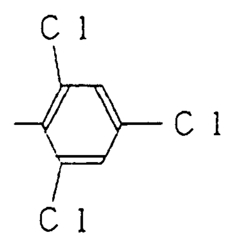 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 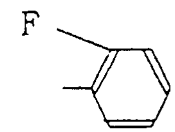 | H |
| $-OCH_3$ | $-OCH_3$ | $-CH(CH_3)_2$ | 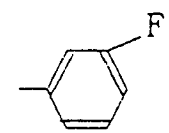 | H |
| $-OCH_3$ | $-OCH_3$ | $-CH(CH_3)_2$ | 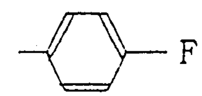 | H |
| $-OCH_3$ | $-OCH_3$ | $-CH(CH_3)_2$ | 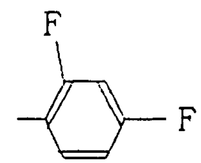 | H |
| $-OCH_3$ | $-OCH_3$ | $-CH(CH_3)_2$ | 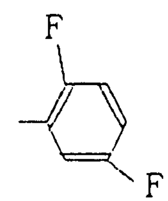 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 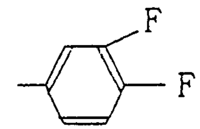 | H |
| $-OCH_3$ | $-OCH_3$ | $-CH(CH_3)_2$ | 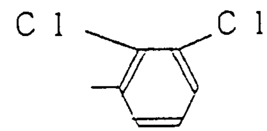 | H |
| $-OCH_3$ | $-OCH_3$ | $-CH(CH_3)_2$ | 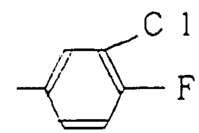 | H |
| $-OCH_3$ | $-OCH_3$ | $-CH(CH_3)_2$ | 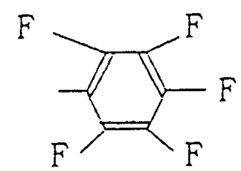 | H |
| $-OCH_3$ | $-OCH_3$ | $-CH(CH_3)_2$ | 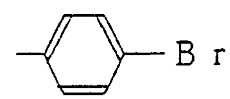 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 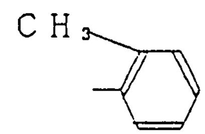 | H |
| $-OCH_3$ | $-OCH_3$ | $-CH(CH_3)_2$ | 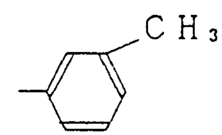 | H |
| $-OCH_3$ | $-OCH_3$ | $-CH(CH_3)_2$ | 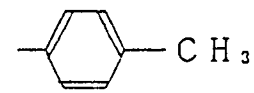 | H |
| $-OCH_3$ | $-OCH_3$ | $-CH(CH_3)_2$ | 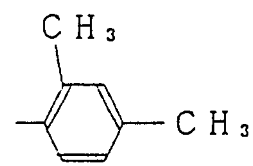 | H |
| $-OCH_3$ | $-OCH_3$ | $-CH(CH_3)_2$ | 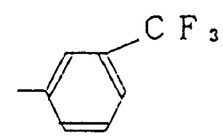 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 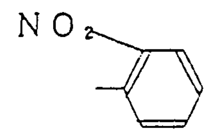 | H |
| $-OCH_3$ | $-OCH_3$ | $-CH(CH_3)_2$ | 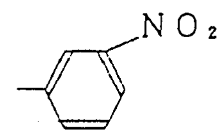 | H |
| $-OCH_3$ | $-OCH_3$ | $-CH(CH_3)_2$ | 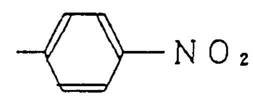 | H |
| $-OCH_3$ | $-OCH_3$ | $-CH(CH_3)_2$ | 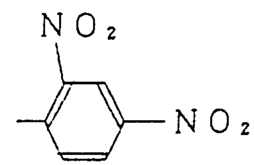 | H |
| $-OCH_3$ | $-OCH_3$ | $-CH(CH_3)_2$ | 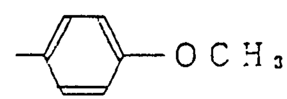 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 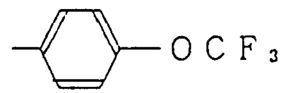 | H |
| $-OCH_3$ | $-OCH_3$ | $-CH(CH_3)_2$ | 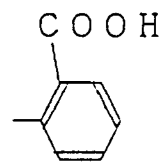 | H |
| $-OCH_3$ | $-OCH_3$ | $-CH(CH_3)_2$ | 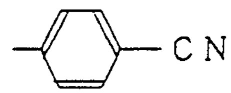 | H |
| $-OCH_3$ | $-OCH_3$ | $-CH(CH_3)_2$ | 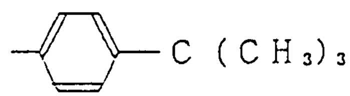 | H |
| $-OCH_3$ | $-OCH_3$ | $-CH(CH_3)_2$ | 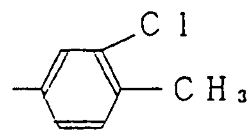 | H |
| $-OCH_3$ | $-OCH_3$ | $-CH(CH_3)_2$ | 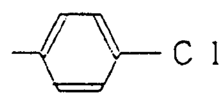 | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 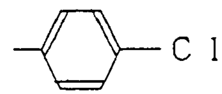 | $-C_2H_5$ |
| $-OCH_3$ | $-OCH_3$ | $-CH(CH_3)_2$ | 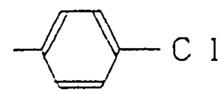 | $-CH_2CH_2CH_3$ |
| $-OCH_3$ | $-OCH_3$ | $-CH(CH_3)_2$ | 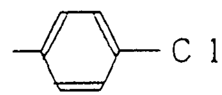 | $-CH(CH_3)_2$ |
| $-OCH_3$ | $-OCH_3$ | $-CH(CH_3)_2$ | 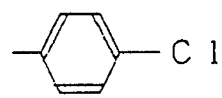 | $-CH_2CH_2CH_2CH_3$ |
| $-OCH_3$ | $-OCH_3$ | $-CH(CH_3)_2$ | 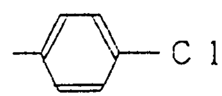 | $-CH_2CH_2CH_2CH_2CH_3$ |
| $-OCH_3$ | $-OCH_3$ | $-CH(CH_3)_2$ | 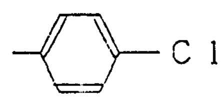 | $-(CH_2)_5CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 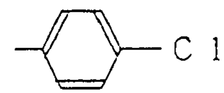 | $-(CH_2)_6CH_3$ |
| $-OCH_3$ | $-OCH_3$ | $-CH(CH_3)_2$ | 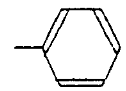 | $-CH(CH_3)_2$ |
| $-OCH_3$ | $-OCH_3$ | $-CH(CH_3)_2$ | 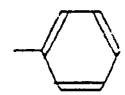 | $-CH(CH_3)C_2H_5$ |
| $-OCH_3$ | $-OCH_3$ | $-CH(CH_3)_2$ | 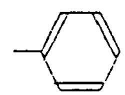 | 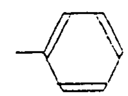 |
| $-OCH_3$ | $-OCH_3$ | $-CH(CH_3)_2$ | 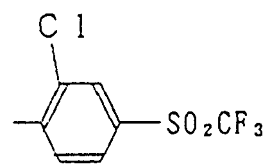 | H |
| $-OCH_3$ | $-OCH_3$ | $-CH(CH_3)_2$ | 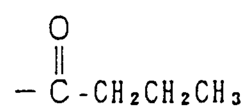 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | $-\overset{O}{\overset{\|}{C}}-C_2H_5$ | H |
| $-OCH_3$ | $-OCH_3$ | $-CH(CH_3)_2$ | $-\overset{O}{\overset{\|}{C}}-CH_3$ | H |
| $-OCH_3$ | $-OCH_3$ | $-CH(CH_3)_2$ | $-\overset{O}{\overset{\|}{C}}-C(CH_3)_3$ | H |
| $-OCH_3$ | $-OCH_3$ | $-CH(CH_3)_2$ | 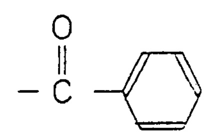 | H |
| $-OCH_3$ | $-OCH_3$ | $-CH(CH_3)_2$ | 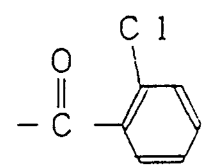 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | $-SO_2-C_2H_5$ | H |
| $-OCH_3$ | $-OCH_3$ | $-CH(CH_3)_2$ | $-SO_2-CH_2CH_2CH_3$ | H |
| $-OCH_3$ | $-OCH_3$ | $-CH(CH_3)_2$ | $-SO_2-CH_2CH_2CH_2CH_3$ | H |
| $-OCH_3$ | $-OCH_3$ | $-CH(CH_3)_2$ | 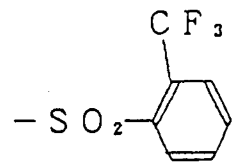 | H |
| $-OCH_3$ | $-OCH_3$ | $-CH(CH_3)_2$ | 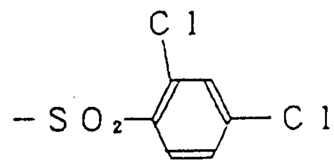 | H |
| $-OCH_3$ | $-OCH_3$ | $-CH(CH_3)_2$ | 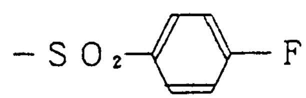 | H |
| $-OCH_3$ | $-OCH_3$ | $-CH(CH_3)_2$ | 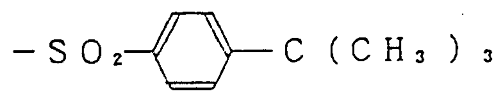 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_2CH_3$ | $-CH_3$ | H |
| $-OCH_3$ | $-OCH_3$ | $-CH_2CH_2CH_2CH_3$ | $-C_2H_5$ | H |
| $-OCH_3$ | $-OCH_3$ | $-CH_2CH_2CH_2CH_3$ | $-CH_2CH_2CH_3$ | H |
| $-OCH_3$ | $-OCH_3$ | $-CH_2CH_2CH_2CH_3$ | $-CH(CH_3)_2$ | H |
| $-OCH_3$ | $-OCH_3$ | $-CH_2CH_2CH_2CH_3$ | $-CH_2CH_2CH_2CH_3$ | H |
| $-OCH_3$ | $-OCH_3$ | $-CH_2CH_2CH_2CH_3$ | $-CH(CH_3)CH_2CH_3$ | H |
| $-OCH_3$ | $-OCH_3$ | $-CH_2CH_2CH_2CH_3$ | $-C(CH_3)_3$ | H |
| $-OCH_3$ | $-OCH_3$ | $-CH_2CH_2CH_2CH_3$ | $-CH_3$ | $-CH_3$ |
| $-OCH_3$ | $-OCH_3$ | $-CH_2CH_2CH_2CH_3$ | $-CH_2CH_3$ | $-CH_3$ |
| $-OCH_3$ | $-OCH_3$ | $-CH_2CH_2CH_2CH_3$ | $-C(CH_3)_3$ | $-CH_2$ |
| $-OCH_3$ | $-OCH_3$ | $-CH_2CH_2CH_2CH_3$ | $-C_2H_5$ | $-C_2H_5$ |
| $-OCH_3$ | $-OCH_3$ | $-CH_2CH_2CH_2CH_3$ | 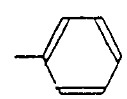 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_2CH_3$ | 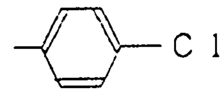 | H |
| $-OCH_3$ | $-OCH_3$ | $-CH_2CH_2CH_2CH_3$ | 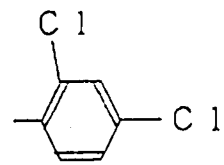 | H |
| $-OCH_3$ | $-OCH_3$ | $-CH_2CH_2CH_2CH_3$ | 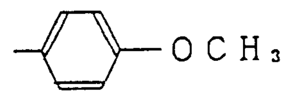 | H |
| $-OCH_3$ | $-OCH_3$ | $-CH_2CH_2CH_2CH_3$ | 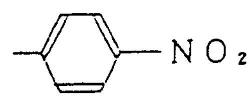 | H |
| $-OCH_3$ | $-OCH_3$ | $-CH_2CH_2CH_2CH_3$ | 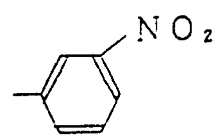 | H |
| $-OCH_3$ | $-OCH_3$ | $-CH_2CH_2CH_2CH_3$ | 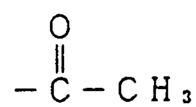 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH(CH_3)_2$ | $-C_2H_5$ | H |
| $-OCH_3$ | $-OCH_3$ | $-CH(CH_3)CH_2CH_3$ | $-CH(CH_3)_2$ | H |
| $-OCH_3$ | $-OCH_3$ | $-CH(CH_3)CH_2CH_3$ | $-CH_2CH_2CH_2CH_3$ | H |
| $-OCH_3$ | $-OCH_3$ | $-CH_2CH_2CH_2CH_3$ | $-CH(CH_3)CH_2CH_3$ | H |
| $-OCH_3$ | $-OCH_3$ | $-CH_2CH_2CH_2CH_3$ | $-C(CH_3)_3$ | H |
| $-OCH_3$ | $-OCH_3$ | $-CH_2CH_3CH_2CH_3$ | $-CH_3$ | $-CH_3$ |
| $-OCH_3$ | $-OCH_3$ | $-CH_2CH_2CH_2CH_3$ | $-C_2H_5$ | $-CH_3$ |
| $-OCH_3$ | $-OCH_3$ | $-CH(CH_3)CH_2CH_3$ | $-CH_2CH_2CH_2CH_3$ | $-CH_3$ |
| $-OCH_3$ | $-OCH_3$ | $-CH_2CH(CH_3)_2$ | $-C(CH_3)_3$ | $-CH_3$ |
| $-OCH_3$ | $-OCH_3$ | $-CH_2CH(CH_3)_2$ | $-C_2H_5$ | $-C_2H_5$ |
| $-OCH_3$ | $-OCH_3$ | $-CH(CH_3)CH_2CH_3$ | $-C(CH_3)_3$ | $-C_2H_5$ |
| $-OCH_3$ | $-OCH_3$ | $-CH_2CH(CH_3)_2$ | 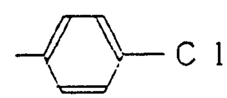 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 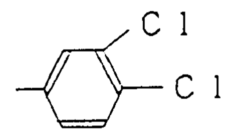 | H |
| $-OCH_3$ | $-OCH_3$ | $-CH(CH_3)CH_2CH_3$ | 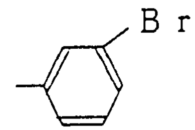 | H |
| $-OCH_3$ | $-OCH_3$ | $-CH_2CH(CH_3)_2$ | 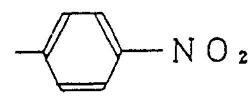 | H |
| $-OCH_3$ | $-OCH_3$ | $-CH_2CH_2CH_2CH_3$ | 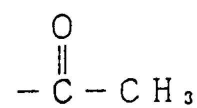 | H |
| $-OCH_3$ | $-OCH_3$ | $-CH(CH_3)CH_2CH_3$ | 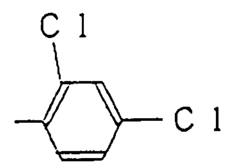 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 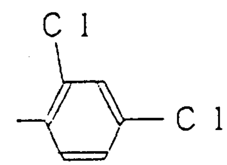 | $-CH_3$ |
| $-OCH_3$ | $-OCH_3$ | $-C(CH_3)_3$ | $-CH_3$ | H |
| $-OCH_3$ | $-OCH_3$ | $-C(CH_3)_3$ | $-C_2H_5$ | H |
| $-OCH_3$ | $-OCH_3$ | $-C(CH_3)_3$ | $-CH_2CH_2CH_3$ | H |
| $-OCH_3$ | $-OCH_3$ | $-C(CH_3)_3$ | $-CH(CH_3)_2$ | H |
| $-OCH_3$ | $-OCH_3$ | $-C(CH_3)_3$ | $-CH_2CH_2CH_2CH_3$ | H |
| $-OCH_3$ | $-OCH_3$ | $-C(CH_3)_3$ | $-CH(CH_3)CH_2CH_3$ | H |
| $-OCH_3$ | $-OCH_3$ | $-C(CH_3)_3$ | $-CH_2CH(CH_3)_2$ | H |
| $-OCH_3$ | $-OCH_3$ | $-C(CH_3)_3$ | $-C(CH_3)_3$ | H |
| $-OCH_3$ | $-OCH_3$ | $-C(CH_3)_3$ | $-CH_3$ | $-CH_3$ |
| $-OCH_3$ | $-OCH_3$ | $-C(CH_3)_3$ | $-C_2H_5$ | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | $-CH_2CH_2CH_2CH_3$ | $-CH_3$ |
| $-OCH_3$ | $-OCH_3$ | $-C(CH_3)_3$ | $-C(CH_3)_3$ | $-CH_3$ |
| $-OCH_3$ | $-OCH_3$ | $-C(CH_3)_3$ | $-C_2H_5$ | $-C_2H_5$ |
| $-OCH_3$ | $-OCH_3$ | $-C(CH_3)_3$ | $-CH_2CH=CH_2$ | H |
| $-OCH_3$ | $-OCH_3$ | $-C(CH_3)_3$ | $-CH_2C\equiv CH$ | H |
| $-OCH_3$ | $-OCH_3$ | $-C(CH_3)_3$ | 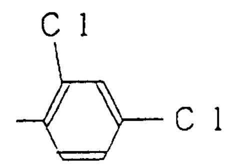 | H |
| $-CCH_3$ | $-OCH_3$ | $-C(CH_3)_3$ | 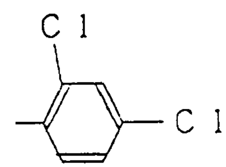 | $-CH_3$ |
| $-OCH_3$ | $-OCH_3$ | $-C(CH_3)_3$ | 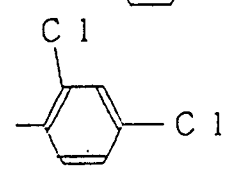 | $-C_2H_5$ |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 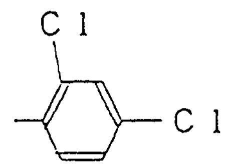 | $-CH_2CH_2CH_3$ |
| $-OCH_3$ | $-OCH_3$ | $-C(CH_3)_3$ | 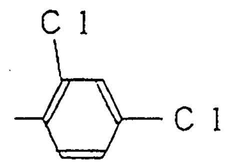 | $-CH_2CH_2CH_2CH_3$ |
| $-OCH_3$ | $-OCH_3$ | $-C(CH_3)_3$ | 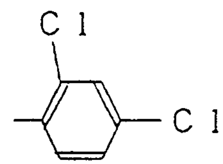 | $-(CH_2)_4CH_3$ |
| $-OCH_3$ | $-OCH_3$ | $-C(CH_3)_3$ | 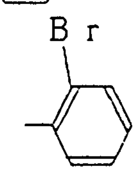 | H |
| $-OCH_3$ | $-OCH_3$ | $-C(CH_3)_3$ | 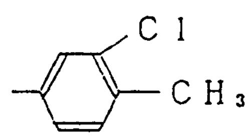 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 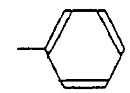 | H |
| $-OCH_3$ | $-OCH_3$ | $-C(CH_3)_3$ | 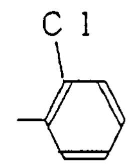 | H |
| $-OCH_3$ | $-OCH_3$ | $-C(CH_3)_3$ | 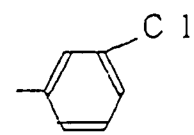 | H |
| $-OCH_3$ | $-OCH_3$ | $-C(CH_3)_3$ | 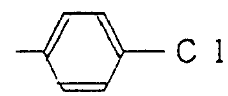 | H |
| $-OCH_3$ | $-OCH_3$ | $-C(CH_3)_3$ | 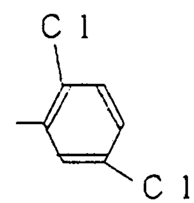 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 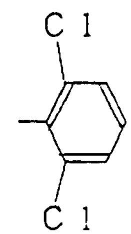 | H |
| $-OCH_3$ | $-OCH_3$ | $-C(CH_3)_3$ | 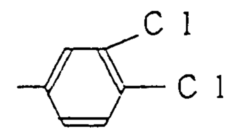 | H |
| $-OCH_3$ | $-OCH_3$ | $-C(CH_3)_3$ | 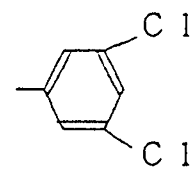 | H |
| $-OCH_3$ | $-OCH_3$ | $-C(CH_3)_3$ | 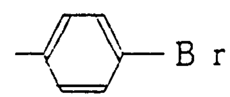 | H |
| $-OCH_3$ | $-OCH_3$ | $-C(CH_3)_3$ | 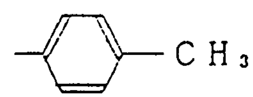 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 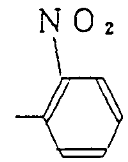 | H |
| $-OCH_3$ | $-OCH_3$ | $-C(CH_3)_3$ | 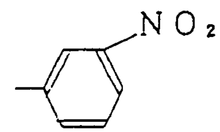 | H |
| $-OCH_3$ | $-OCH_3$ | $-C(CH_3)_3$ | 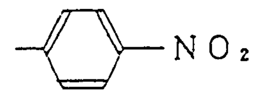 | H |
| $-OCH_3$ | $-OCH_3$ | $-C(CH_3)_3$ | 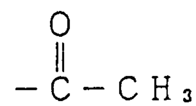 | H |
| $-OCH_3$ | $-OCH_3$ | $-C(CH_3)_3$ | 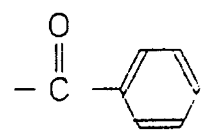 | H |

Table 1 (Continued)

| $R^1$ | $R^2$ | $R^3$ | $R^4$ | $R^5$ |
| --- | --- | --- | --- | --- |
| $-OCH_3$ | $-OCH_3$ | $-C(CH_3)_3$ | 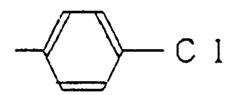 | $-CH_3$ |
| $-OCH_3$ | $-OCH_3$ | $-C(CH_3)_3$ | 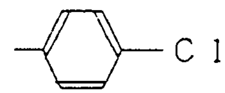 | $-C_2H_5$ |
| $-OCH_3$ | $-OCH_3$ | $-C(CH_3)_3$ | 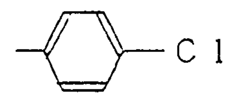 | $-CH_2CH_2CH_3$ |
| $-OCH_3$ | $-OCH_3$ | $-C(CH_3)_3$ | 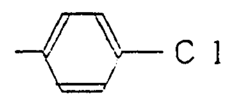 | $-CH_2CH_2CH_2CH_3$ |
| $-OCH_3$ | $-OCH_3$ | $-C(CH_3)_3$ | 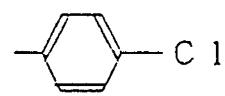 | $-(CH_2)_4CH_3$ |
| $-OCH_3$ | $-OCH_3$ | $-C(CH_3)_3$ | 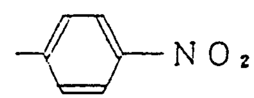 | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 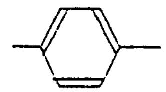 $NO_2$ | $-C_2H_5$ |
| $-OCH_3$ | $-OCH_3$ | $-C(CH_3)_3$ | 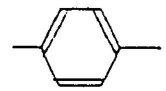 $NO_2$ | $-C_2H_5$ |
| $-OCH_3$ | $-OCH_3$ | $-C(CH_3)_3$ | 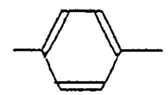 $NO_2$ | $-CH_2CH_2CH_3$ |
| $-OCH_3$ | $-OCH_3$ | $-C(CH_3)_3$ | 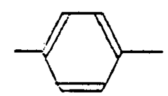 $CH(CH_3)_2$ | H |
| $-OCH_3$ | $-OCH_3$ | $-C(CH_3)_3$ | 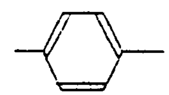 $C_2H_5$ | H |
| $-OCH_3$ | $-OCH_3$ | $-C(CH_3)_3$ | 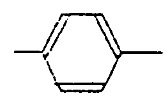 $OC_2H_5$ | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 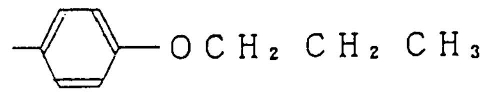 | H |
| $-OCH_3$ | $-OCH_3$ | $-C(CH_3)_3$ | 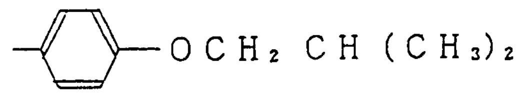 | H |
| $-OCH_3$ | $-OCH_3$ | $-C(CH_3)_3$ | 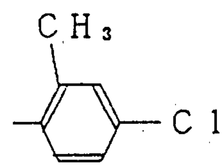 | H |
| $-OCH_3$ | $-OCH_3$ | $-C(CH_3)_3$ | 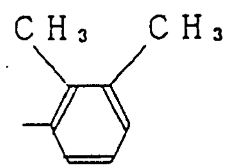 | H |
| $-OCH_3$ | $-OCH_3$ | $-C(CH_3)_3$ | 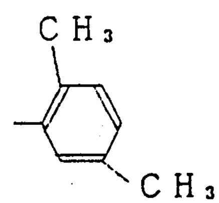 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 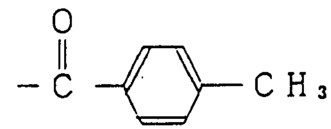 | H |
| $-OCH_3$ | $-OCH_3$ | $-C(CH_3)_3$ | 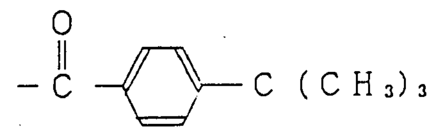 | H |
| $-OCH_3$ | $-OCH_3$ | $-C(CH_3)_3$ | 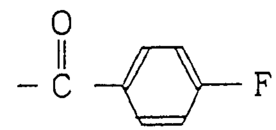 | H |
| $-OCH_3$ | $-OCH_3$ | $-C(CH_3)_3$ | 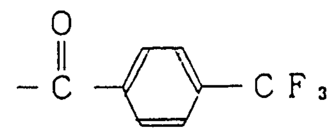 | H |
| $-OCH_3$ | $-OCH_3$ | $-C(CH_3)_3$ | 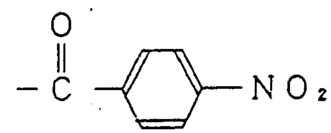 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 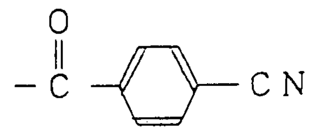 | H |
| $-OCH_3$ | $-OCH_3$ | $-C(CH_3)_3$ | 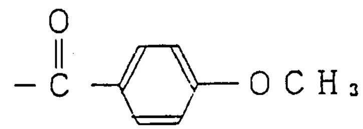 | H |
| $-OCH_3$ | $-OCH_3$ | $-(CH_2)_4CH_3$ | $-CH_3$ | H |
| $-OCH_3$ | $-OCH_3$ | $-(CH_2)_4CH_3$ | $-C_2H_5$ | H |
| $-OCH_3$ | $-OCH_3$ | $-(CH_2)_4CH_3$ | $-CH_2CH_2CH_3$ | H |
| $-OCH_3$ | $-OCH_3$ | $-(CH_2)_4CH_3$ | $-CH(CH_3)_2$ | H |
| $-OCH_3$ | $-OCH_3$ | $-(CH_2)_4CH_3$ | $-CH_2CH_2CH_2CH_3$ | H |
| $-OCH_3$ | $-OCH_3$ | $-(CH_2)_4CH_3$ | $-CH(CH_3)CH_2CH_3$ | H |
| $-OCH_3$ | $-OCH_3$ | $-(CH_2)_4CH_3$ | $-CH_2CH(CH_3)_2$ | H |
| $-OCH_3$ | $-OCH_3$ | $-(CH_2)_4CH_3$ | $-C(CH_3)_3$ | H |

Table 1 (Continued)

| $R^1$ | $R^2$ | $R^3$ | $R^4$ | $R^5$ |
|---|---|---|---|---|
| $-OCH_3$ | $-OCH_3$ | $-(CH_2)_4CH_3$ | $-CH_3$ | $-CH_3$ |
| $-OCH_3$ | $-OCH_3$ | $-(CH_2)_4CH_3$ | $-C_2H_5$ | $-CH_3$ |
| $-OCH_3$ | $-OCH_3$ | $-(CH_2)_4CH_3$ | $-CH_2CH_2CH_3$ | $-CH_3$ |
| $-OCH_3$ | $-OCH_3$ | $-(CH_2)_4CH_3$ | $-CH_2CH_2CH_2CH_3$ | $-CH_3$ |
| $-OCH_3$ | $-OCH_3$ | $-(CH_2)_4CH_3$ | $-CH(CH_3)_2$ | $-CH_3$ |
| $-OCH_3$ | $-OCH_3$ | $-(CH_2)_4CH_3$ | $-CH(CH_3)CH_2CH_3$ | $-CH_3$ |
| $-OCH_3$ | $-OCH_3$ | $-(CH_2)_4CH_3$ | $-CH_2CH(CH_3)_2$ | $-CH_3$ |
| $-OCH_3$ | $-OCH_3$ | $-(CH_2)_4CH_3$ | $-C(CH_3)_3$ | $-CH_3$ |
| $-OCH_3$ | $-OCH_3$ | $-(CH_2)_4CH_3$ | $-C_2H_5$ | $-C_2H_5$ |
| $-OCH_3$ | $-OCH_3$ | $-(CH_2)_4CH_3$ | 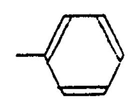 | H |
| $-OCH_3$ | $-OCH_3$ | $-(CH_2)_4CH_3$ | 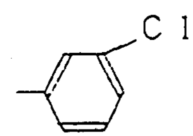 | H |

Table 1 (Continued)

| $R^1$ | $R^2$ | $R^3$ | $R^4$ | $R^5$ |
|---|---|---|---|---|
| $-OCH_3$ | $-OCH_3$ | $-(CH_2)_4CH_3$ | 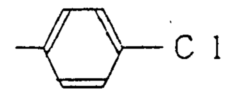 | H |
| $-OCH_3$ | $-OCH_3$ | $-(CH_2)_4CH_3$ | 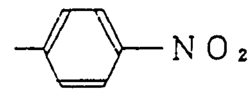 | H |
| $-OCH_3$ | $-OCH_3$ | $-(CH_2)_4CH_3$ | 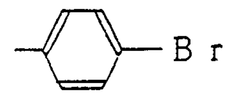 | H |
| $-OCH_3$ | $-OCH_3$ | $-(CH_2)_4CH_3$ | 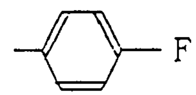 | H |
| $-OCH_3$ | $-OCH_3$ | $-(CH_2)_4CH_3$ | 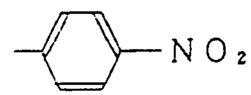 | $-CH_3$ |
| $-OCH_3$ | $-OCH_3$ | $-(CH_2)_4CH_3$ | 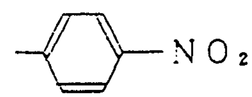 | $-C_2H_5$ |

Table 1 (Continued)

| $R^1$ | $R^2$ | $R^3$ | $R^4$ | $R^5$ |
|---|---|---|---|---|
| $-OCH_3$ | $-OCH_3$ | $-(CH_2)_4CH_3$ | 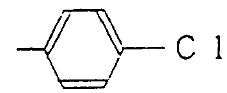 | $-CH_3$ |
| $-OCH_3$ | $-OCH_3$ | $-(CH_2)_4CH_3$ | 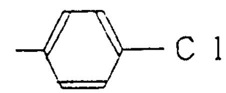 | $-C_2H_5$ |
| $-OCH_3$ | $-OCH_3$ | $-(CH_2)_4CH_3$ | $-\overset{O}{\overset{\|}{C}}-CH_3$ | H |
| $-OCH_3$ | $-OCH_3$ | $-(CH_2)_4CH_3$ | $-\overset{O}{\overset{\|}{C}}-C(CH_3)_3$ | H |
| $-OCH_3$ | $-OCH_3$ | $-CH(CH_3)CH_2CH_2CH_3$ | $-CH_3$ | H |
| $-OCH_3$ | $-OCH_3$ | $-CH(CH_3)CH_2CH_2CH_3$ | $-C(CH_3)_3$ | H |
| $-OCH_3$ | $-OCH_3$ | $-CH_2CH_2CH(CH_3)_2$ | $-CH_3$ | $-CH_3$ |
| $-OCH_3$ | $-OCH_3$ | $-CH_2CH_2CH(CH_3)_2$ | $-C(CH_3)_3$ | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_2CH_3$ | 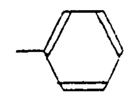 | H |
| $-OCH_3$ | $-OCH_3$ | 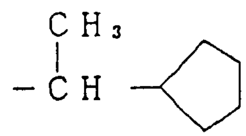 | $-CH_3$ | H |
| $-OCH_3$ | $-OCH_3$ | $-CH(CH_3)CH_2CH_2CH_2CH_3$ | 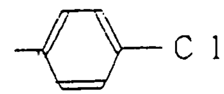 | H |
| $-OCH_3$ | $-OCH_3$ | $-CH(CH_3)CH_2CH_2CH_2CH_3$ | 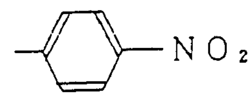 | H |
| $-OCH_3$ | $-OCH_3$ | 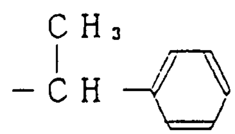 | 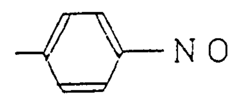 | H |
| $-OCH_3$ | $-OCH_3$ | 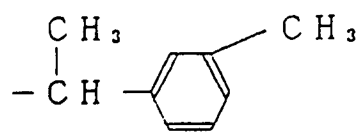 | 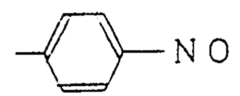 | H |

Table 1 (Continued)

| $R^1$ | $R^2$ | $R^3$ | $R^4$ | $R^5$ |
|---|---|---|---|---|
| $-OCH_3$ | $-OCH_3$ | 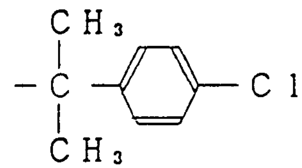 | 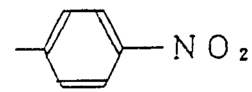 | H |
| $-OCH_3$ | $-OCH_3$ | 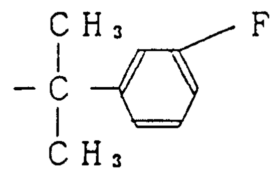 | 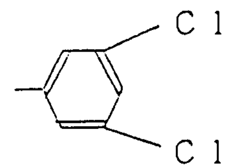 | H |
| $-OCH_3$ | $-OCH_3$ | 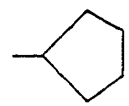 | 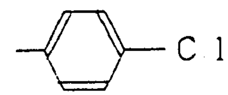 | H |
| $-OCH_3$ | $-OCH_3$ | 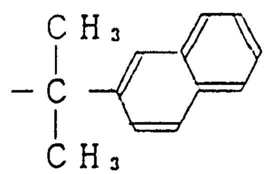 | 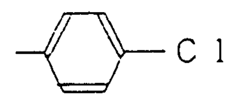 | H |
| $-OCH_3$ | $-OCH_3$ | 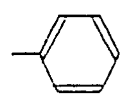 | $-CH_3$ | H |

Table 1 (Continued)

| $R^1$ | $R^2$ | $R^3$ | $R^4$ | $R^5$ |
|---|---|---|---|---|
| $-OCH_3$ | $-OCH_3$ | 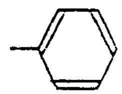 | $-C_2H_5$ | H |
| $-OCH_3$ | $-OCH_3$ | 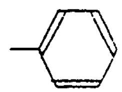 | $-CH_2CH_2CH_3$ | H |
| $-OCH_3$ | $-OCH_3$ | 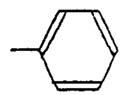 | $-CH(CH_3)_2$ | H |
| $-OCH_3$ | $-OCH_3$ | 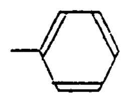 | $-CH_2CH_2CH_2CH_3$ | H |
| $-OCH_3$ | $-OCH_3$ | 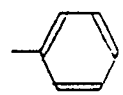 | $-CH(CH_3)CH_2CH_3$ | H |
| $-OCH_3$ | $-OCH_3$ | 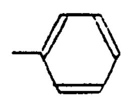 | $-CH_2CH(CH_3)_2$ | H |

Table 1 (Continued)

| $R^1$ | $R^2$ | $R^3$ | $R^4$ | $R^5$ |
|---|---|---|---|---|
| $-OCH_3$ | $-OCH_3$ | 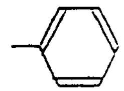 | $-C(CH_3)_3$ | H |
| $-OCH_3$ | $-OCH_3$ | 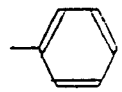 | $-CH_3$ | H |
| $-OCH_3$ | $-OCH_3$ | 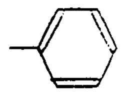 | 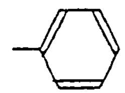 | H |
| $-OCH_3$ | $-OCH_3$ | 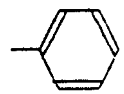 | 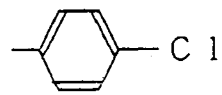 | H |
| $-OCH_3$ | $-OCH_3$ | 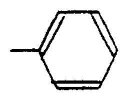 | 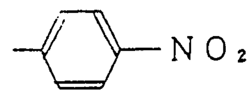 | H |
| $-OCH_3$ | $-OCH_3$ | 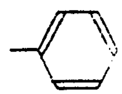 | 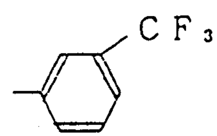 | H |

Table 1 (Continued)

| $R^1$ | $R^2$ | $R^3$ | $R^4$ | $R^5$ |
|---|---|---|---|---|
| $-OCH_3$ | $-OCH_3$ | 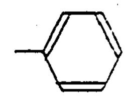 | 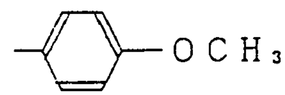 | H |
| $-OCH_3$ | $-OCH_3$ | 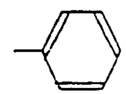 | 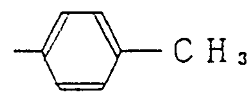 | H |
| $-OCH_3$ | $-OCH_3$ | 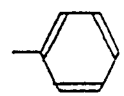 | 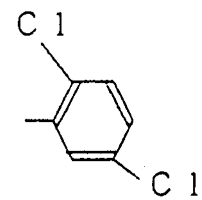 | H |
| $-OCH_3$ | $-OCH_3$ | 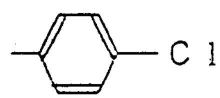 | $-CH_3$ | H |
| $-OCH_3$ | $-OCH_3$ | 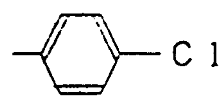 | $-C_2H_5$ | H |
| $-OCH_3$ | $-OCH_3$ | 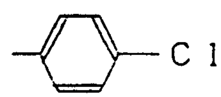 | $-CH_2CH_2CH_3$ | H |

Table 1 (Continued)

| $R^1$ | $R^2$ | $R^3$ | $R^4$ | $R^5$ |
|---|---|---|---|---|
| $-OCH_3$ | $-OCH_3$ | 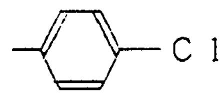 | $-CH(CH_3)_2$ | H |
| $-OCH_3$ | $-OCH_3$ | 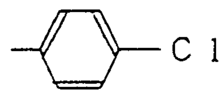 | $-CH_2CH_2CH_2CH_3$ | H |
| $-OCH_3$ | $-OCH_3$ | 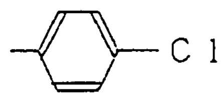 | $-CH(CH_3)CH_2CH_3$ | H |
| $-OCH_3$ | $-OCH_3$ | 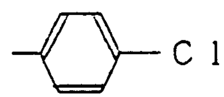 | $-CH_2CH(CH_3)_2$ | H |
| $-OCH_3$ | $-OCH_3$ | 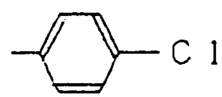 | $-C(CH_3)_3$ | H |
| $-OCH_3$ | $-OCH_3$ | 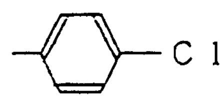 | $-CH_3$ | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | 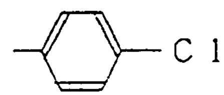 | 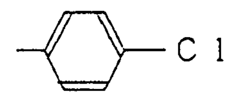 | H |
| $-OCH_3$ | $-OCH_3$ | 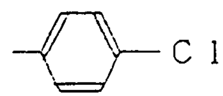 | 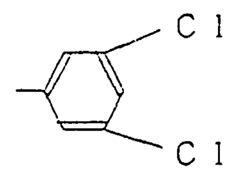 | H |
| $-OCH_3$ | $-OCH_3$ | 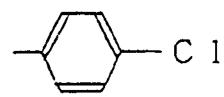 | 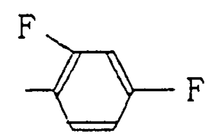 | H |
| $-OCH_3$ | $-OCH_3$ | 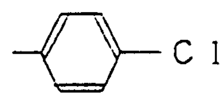 | 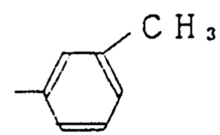 | H |
| $-OCH_3$ | $-OCH_3$ | 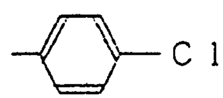 | 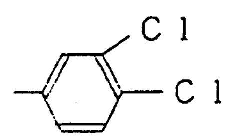 | H |

Table 1 (Continued)

| $R^1$ | $R^2$ | $R^3$ | $R^4$ | $R^5$ |
|---|---|---|---|---|
| $-OCH_3$ | $-OCH_3$ | 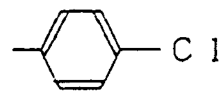 | 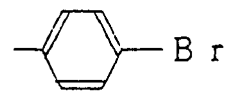 | H |
| $-OCH_3$ | $-OCH_3$ | 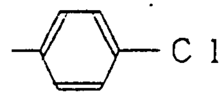 | 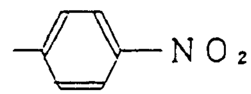 | H |
| $-OCH_3$ | $-OCH_3$ | 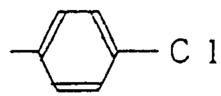 | 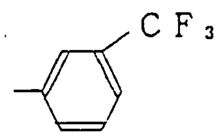 | H |
| $-OCH_3$ | $-OCH_3$ | 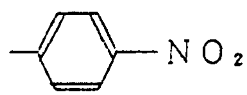 | 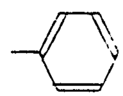 | H |
| $-OCH_3$ | $-OCH_3$ | 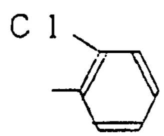 | 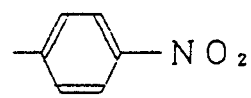 | H |

Table 1 (Continued)

| $R^1$ | $R^2$ | $R^3$ | $R^4$ | $R^5$ |
|---|---|---|---|---|
| $-OCH_3$ | $-OCH_3$ | 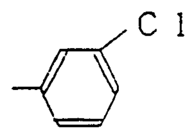 | 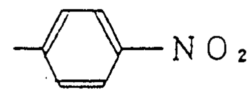 | H |
| $-OCH_3$ | $-OCH_3$ | 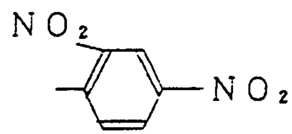 | 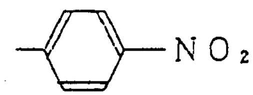 | H |
| $-OCH_3$ | $-OCH_3$ | 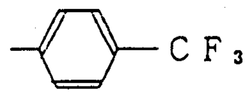 | 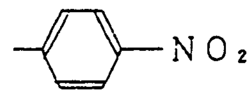 | H |
| $-OCH_3$ | $-OCH_3$ | 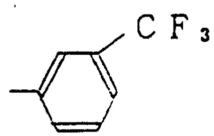 | 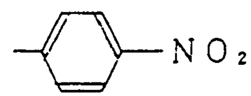 | H |
| $-OCH_3$ | $-OCH_3$ | 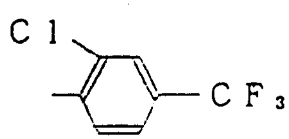 | 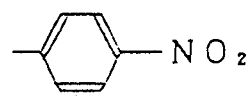 | H |

Table 1 (Continued)

| $R^1$ | $R^2$ | $R^3$ | $R^4$ | $R^5$ |
|---|---|---|---|---|
| $-OCH_3$ | $-OCH_3$ | 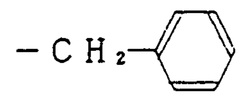 | 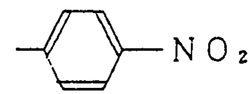 | H |
| $-OCH_3$ | $-OCH_3$ | $-CH(CH_3)_2$ | 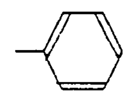 | $-CH_3$ |
| $-OCH_3$ | $-OCH_3$ | 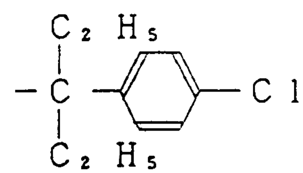 | $-CH_3$ | H |
| $-OCH_3$ | $-OCH_3$ | 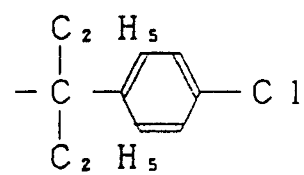 | $-C(CH_3)_3$ | H |
| $-OCH_3$ | $-OCH_3$ | 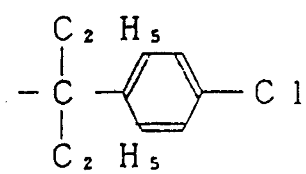 | 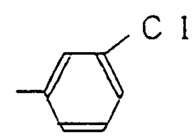 | H |

Table 1 (Continued)

| $R^1$ | $R^2$ | $R^3$ | $R^4$ | $R^5$ |
|---|---|---|---|---|
| $-OCH_3$ | $-OCH_3$ | 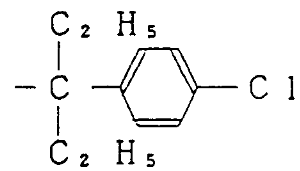 | 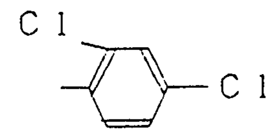 | H |
| $-OCH_3$ | $-OCH_3$ | 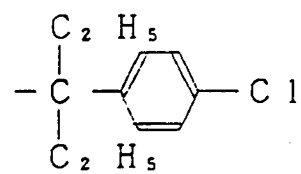 | 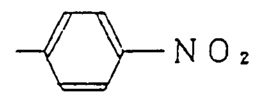 | H |
| $-OCH_3$ | $-OCH_3$ | 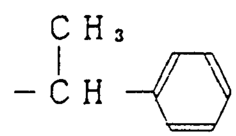 | $-CH_3$ | H |
| $-OCH_3$ | $-OCH_3$ | 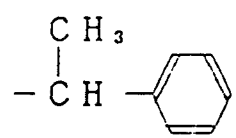 | $-C(CH_3)_3$ | H |
| $-OCH_3$ | $-OCH_3$ | 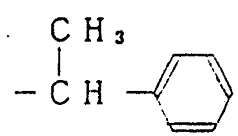 | 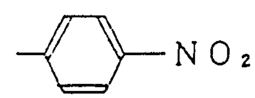 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CF_3$ | $-CH_3$ | H |
| $-OCH_3$ | $-OCH_3$ | $-CH_2CF_3$ | $-C(CH_3)_3$ | H |
| $-OCH_3$ | $-OCH_3$ | $-CH_2CF_3$ | 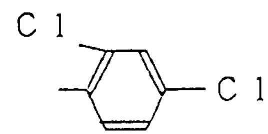 | H |
| $-OCH_3$ | $-OCH_3$ | $-CH_2CF_3$ | 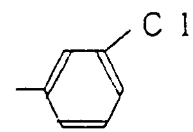 | H |
| $-OCH_3$ | $-OCH_3$ | 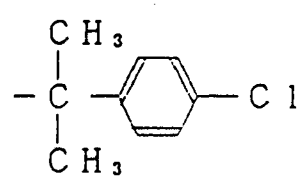 | $-CH_3$ | H |
| $-OCH_3$ | $-OCH_3$ | 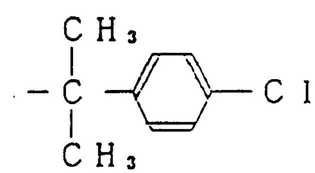 | $-C(CH_3)_3$ | H |

Table 1 (Continued)

| $R^1$ | $R^2$ | $R^3$ | $R^4$ | $R^5$ |
|---|---|---|---|---|
| $-OCH_3$ | $-OCH_3$ | 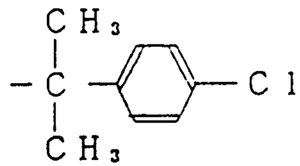 | 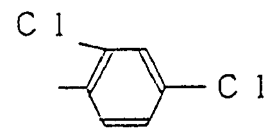 | H |
| $-OCH_3$ | $-OCH_3$ | 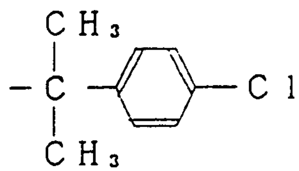 | 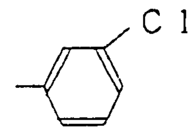 | H |
| $-OCH_3$ | $-OCH_3$ | 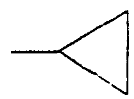 | $-CH_3$ | H |
| $-OCH_3$ | $-OCH_3$ | 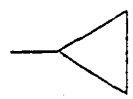 | $-CH_3$ | $-CH_3$ |
| $-OCH_3$ | $-OCH_3$ | 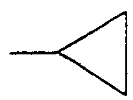 | $-C(CH_3)_3$ | H |

Table 1 (Continued)

| $R^1$ | $R^2$ | $R^3$ | $R^4$ | $R^5$ |
|---|---|---|---|---|
| $-OCH_3$ | $-OCH_3$ | 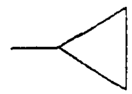 | 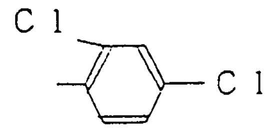 | H |
| $-OCH_3$ | $-OCH_3$ | $-CH(C_2H_5)_2$ | $-CH_3$ | H |
| $-OCH_3$ | $-OCH_3$ | $-CH(C_2H_5)_2$ | $-C(CH_3)_3$ | H |
| $-OCH_3$ | $-OCH_3$ | $-CH(C_2H_5)_2$ | 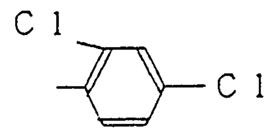 | H |
| $-OCH_3$ | $-OCH_3$ | $-CH(C_2H_5)_2$ | 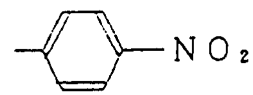 | H |
| $-OCH_3$ | $-OCH_3$ | 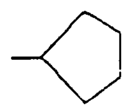 | $-CH_3$ | H |
| $-OCH_3$ | $-OCH_3$ | 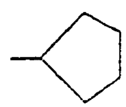 | $-CH_3$ | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | 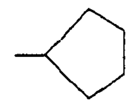 | $-CH_2CH(CH_3)_2$ | H |
| $-OCH_3$ | $-OCH_3$ | 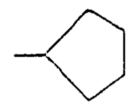 | 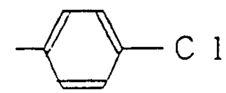 | $-CH_3$ |
| $-OCH_3$ | $-OCH_3$ | 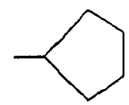 | 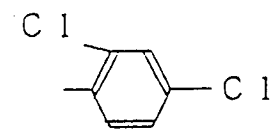 | H |
| $-OCH_3$ | $-OCH_3$ | 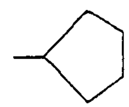 | 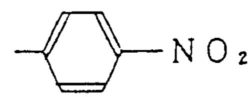 | H |
| $-OCH_3$ | $-OCH_3$ | 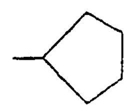 | 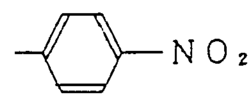 | $-CH_3$ |
| $-OCH_3$ | $-OCH_3$ | 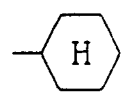 | $-CH_3$ | H |

Table 1 (Continued)

| $R^1$ | $R^2$ | $R^3$ | $R^4$ | $R^5$ |
|---|---|---|---|---|
| $-OCH_3$ | $-OCH_3$ | 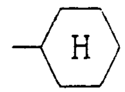 | $-C(CH_3)_3$ | H |
| $-OCH_3$ | $-OCH_3$ | 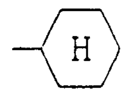 | $-CH_3$ | $-CH_3$ |
| $-OCH_3$ | $-OCH_3$ | 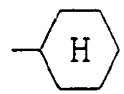 | 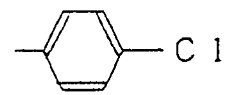 | H |
| $-OCH_3$ | $-OCH_3$ | 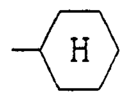 | 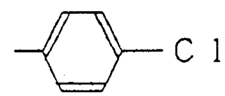 | $-CH_3$ |
| $-OCH_3$ | $-OCH_3$ | 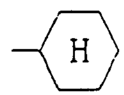 | 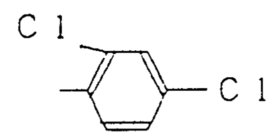 | H |
| $-OCH_3$ | $-OCH_3$ | 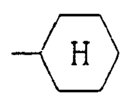 | 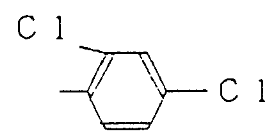 | $-CH_3$ |

Table 1 (Continued)

| $R^1$ | $R^2$ | $R^3$ | $R^4$ | $R^5$ |
|---|---|---|---|---|
| $-OCH_3$ | $-OCH_3$ | 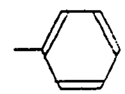 | 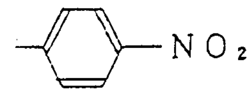 | H |
| $-OCH_3$ | $-OCH_3$ | 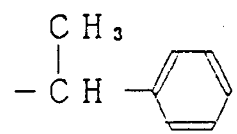 | 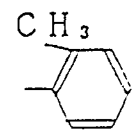 | H |
| $-OCH_3$ | $-OCH_3$ | 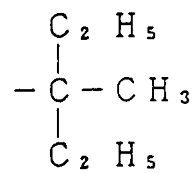 | $-CH_3$ | H |
| $-OCH_3$ | $-OCH_3$ | 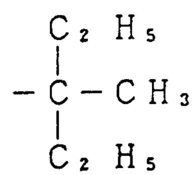 | $-C(CH_3)_3$ | H |
| $-OCH_3$ | $-OCH_3$ | 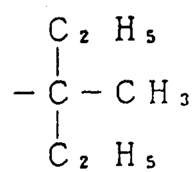 | 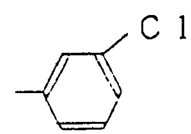 | H |

Table 1 (Continued)

| $R^1$ | $R^2$ | $R^3$ | $R^4$ | $R^5$ |
|---|---|---|---|---|
| $-OCH_3$ | $-OCH_3$ | 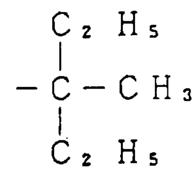 | 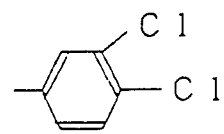 | $-CH_3$ |
| $-OCH_3$ | $-OCH_3$ | 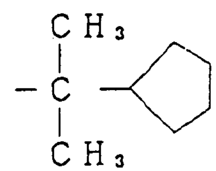 | $-CH_3$ | H |
| $-OCH_3$ | $-OCH_3$ | 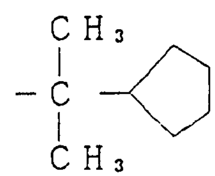 | $-C(CH_3)_3$ | H |
| $-OCH_3$ | $-OCH_3$ | 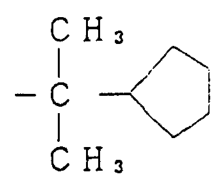 | 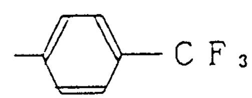 | H |

Table 1 (Continued)

| $R^1$ | $R^2$ | $R^3$ | $R^4$ | $R^5$ |
|---|---|---|---|---|
| $-OCH_3$ | $-OCH_3$ | 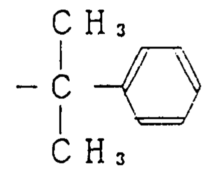 | 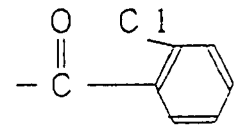 | H |
| $-OCH_3$ | $-OCH_3$ | 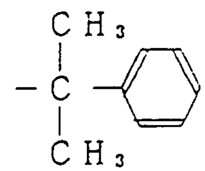 | 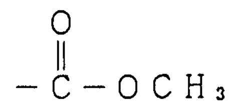 | H |
| $-OCH_3$ | $-OCH_3$ | 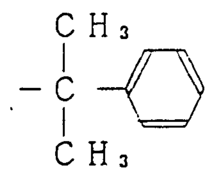 | 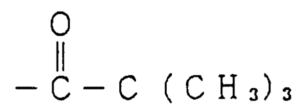 | H |
| $-OCH_3$ | $-OCH_3$ | 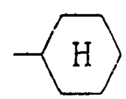 | 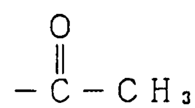 | H |
| $-OCH_3$ | $-OCH_3$ | 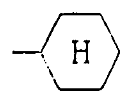 | 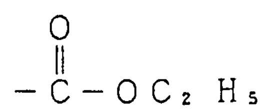 | H |

Table 1 (Continued)

| $R^1$ | $R^2$ | $R^3$ | $R^4$ | $R^5$ |
|---|---|---|---|---|
| $-OCH_3$ | $-OCH_3$ | 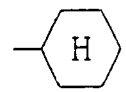 | 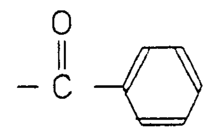 | H |
| $-OCH_3$ | $-OCH_3$ | $-CH(C_2H_5)_2$ | 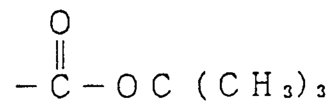 | H |
| $-OCH_3$ | $-OCH_3$ | 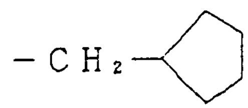 | $-SO_2-CH_3$ | H |
| $-OCH_3$ | $-OCH_3$ | $-C(CH_3)_3$ | 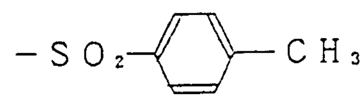 | H |
| $-OCH_3$ | $-OCH_3$ | 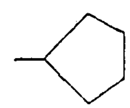 | $-SO_2CH_3$ | H |
| $-OCH_3$ | $-OCH_3$ | $-CH(CH_3)_2$ | 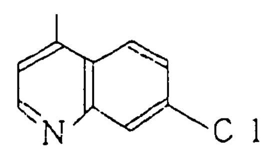 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 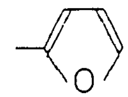 | H |
| $-OCH_3$ | $-OCH_3$ | $-CH(CH_3)_2$ | 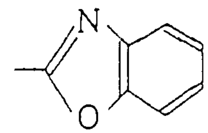 | H |
| $-OCH_3$ | $-OCH_3$ | $-CH(CH_3)_2$ | 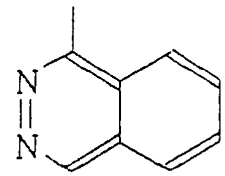 | H |
| $-OCH_3$ | $-OCH_3$ | $-CH(CH_3)_2$ | 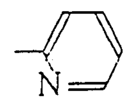 | H |
| $-OCH_3$ | $-OCH_3$ | $-CH(CH_3)_2$ | 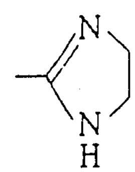 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 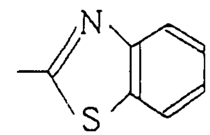 | H |
| $-OCH_3$ | $-OCH_3$ | $-CH(CH_3)_2$ | 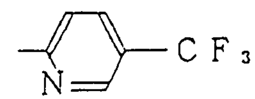 | H |
| $-OCH_3$ | $-OCH_3$ | $-CH(CH_3)_2$ | 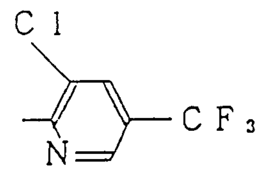 | H |
| $-OCH_3$ | $-OCH_3$ | $-CH(CH_3)_2$ | 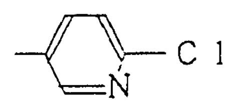 | H |
| $-OCH_3$ | $-OCH_3$ | $-CH(CH_3)_2$ | 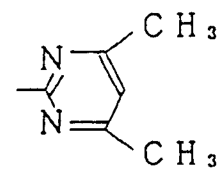 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 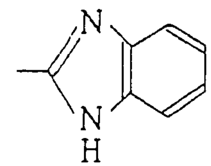 | H |
| $-OCH_3$ | $-OCH_3$ | $-CH(CH_3)_2$ | 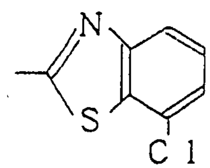 | H |
| $-OCH_3$ | $-OCH_3$ | $-CH(CH_3)_2$ | 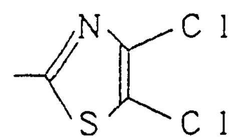 | H |
| $-OCH_3$ | $-OCH_3$ | $-CH(CH_3)_2$ | 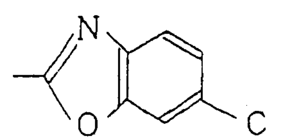 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 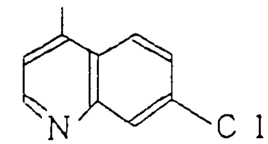 | H |
| $-OCH_3$ | $-OCH_3$ | $-CH(CH_3)CH_2CH_3$ | 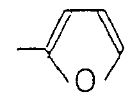 | H |
| $-OCH_3$ | $-OCH_3$ | $-CH(CH_3)CH_2CH_3$ | 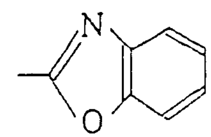 | H |
| $-OCH_3$ | $-OCH_3$ | $-CH(CH_3)CH_2CH_3$ | 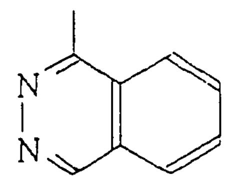 | H |
| $-OCH_3$ | $-OCH_3$ | $-CH(CH_3)CH_2CH_3$ | 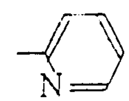 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 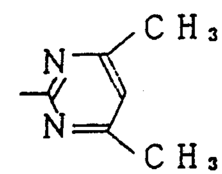 | H |
| $-OCH_3$ | $-OCH_3$ | $-CH(CH_3)CH_2CH_3$ | 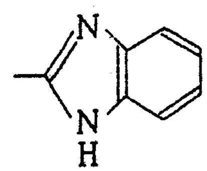 | H |
| $-OCH_3$ | $-OCH_3$ | $-CH(CH_3)CH_2CH_3$ | 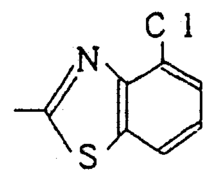 | H |
| $-OCH_3$ | $-OCH_3$ | $-CH(CH_3)CH_2CH_3$ | 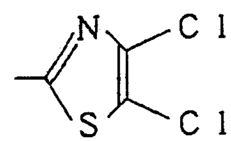 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 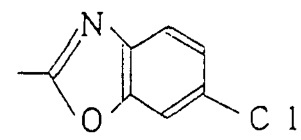 | H |
| $-OCH_3$ | $-OCH_3$ | $-CH(CH_3)CH_2CH_3$ | 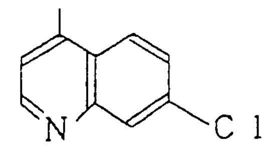 | H |
| $-OCH_3$ | $-OCH_3$ | $-C(CH_3)_3$ | 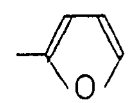 | H |
| $-OCH_3$ | $-OCH_3$ | $-C(CH_3)_3$ | 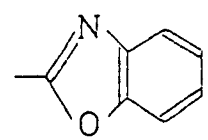 | H |
| $-OCH_3$ | $-OCH_3$ | $-C(CH_3)_3$ | 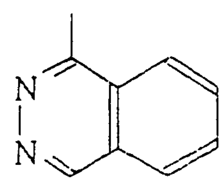 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 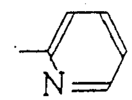 | H |
| $-OCH_3$ | $-OCH_3$ | $-C(CH_3)_3$ | 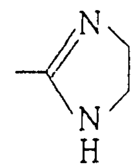 | H |
| $-OCH_3$ | $-OCH_3$ | $-C(CH_3)_3$ | 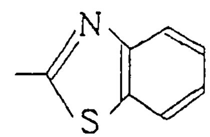 | H |
| $-OCH_3$ | $-OCH_3$ | $-C(CH_3)_3$ | 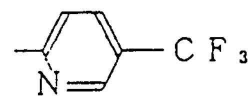 | H |
| $-OCH_3$ | $-OCH_3$ | $-C(CH_3)_3$ | 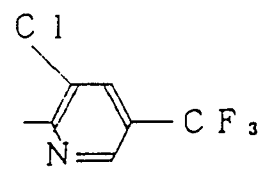 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 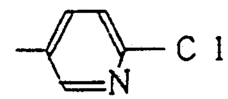 | H |
| $-OCH_3$ | $-OCH_3$ | $-C(CH_3)_3$ | 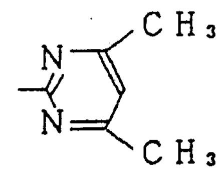 | H |
| $-OCH_3$ | $-OCH_3$ | $-C(CH_3)_3$ | 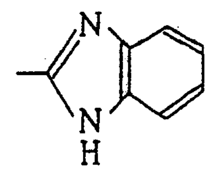 | H |
| $-OCH_3$ | $-OCH_3$ | $-C(CH_3)_3$ | 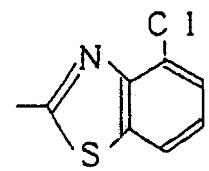 | H |
| $-OCH_3$ | $-OCH_3$ | $-C(CH_3)_3$ | 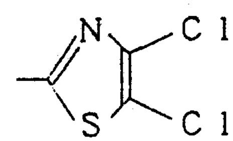 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 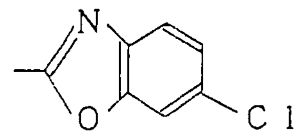 | H |
| $-OCH_3$ | $-OCH_3$ | 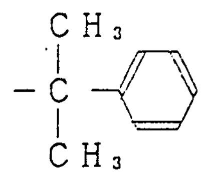 | 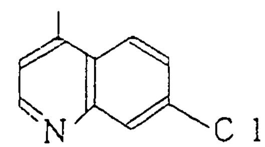 | H |
| $-OCH_3$ | $-OCH_3$ | $-CH_2CH(CH_3)_2$ | 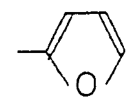 | H |
| $-OCH_3$ | $-OCH_3$ | $-CH_2CH_2CH_3$ | 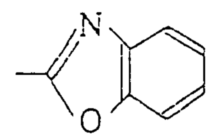 | H |
| $-OCH_3$ | $-OCH_3$ | $-C_2H_5$ | 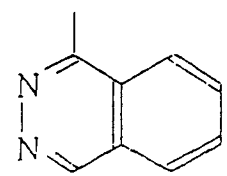 | H |

Table 1 (Continued)

| $R^1$ | $R^2$ | $R^3$ | $R^4$ | $R^5$ |
|---|---|---|---|---|
| $-OCH_3$ | $-OCH_3$ | 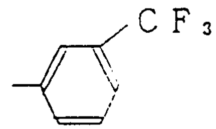 | 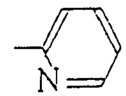 | H |
| $-OCH_3$ | $-OCH_3$ | $-CH_2CH_2CH_2CH_3$ | 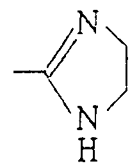 | H |
| $-OCH_3$ | $-OCH_3$ | 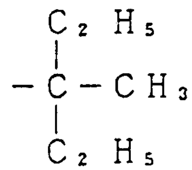 | 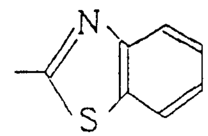 | H |
| $-OCH_3$ | $-OCH_3$ | 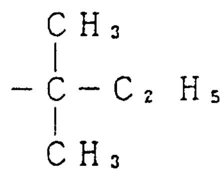 | 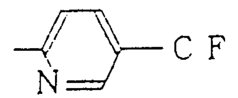 | H |
| $-OCH_3$ | $-OCH_3$ | $-CH_2CH_2CH_3$ | 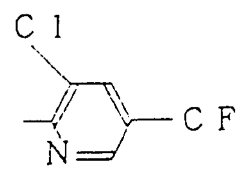 | H |

Table 1 (Continued)

| $R^1$ | $R^2$ | $R^3$ | $R^4$ | $R^5$ |
|---|---|---|---|---|
| $-OCH_3$ | $-OCH_3$ | $-C_2H_5$ | 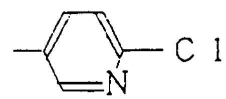 | H |
| $-OCH_3$ | $-OCH_3$ | $-CH_2CH(CH_3)_2$ | 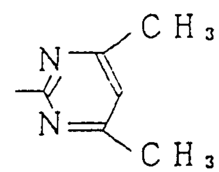 | H |
| $-OCH_3$ | $-OCH_3$ | 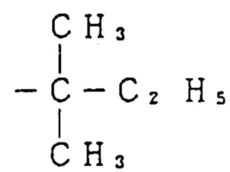 | 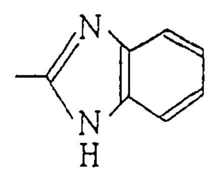 | H |
| $-OCH_3$ | $-OCH_3$ | 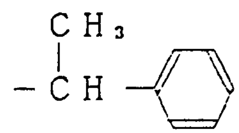 | 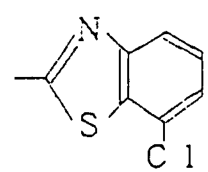 | H |
| $-OCH_3$ | $-OCH_3$ | 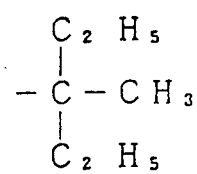 | 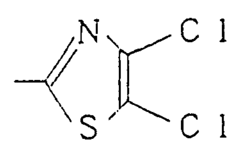 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH_2CH_2CH_3$ | 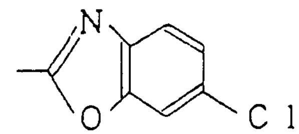 | H |
| $-OCH_3$ | $-OCH_3$ | $-CH(CH_3)_2$ | 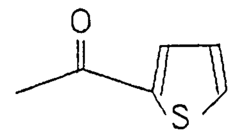 | H |
| $-OCH_3$ | $-OCH_3$ | $-CH(CH_3)_2$ | 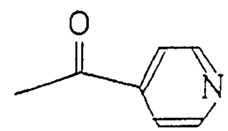 | H |
| $-OCH_3$ | $-OCH_3$ | $-CH(CH_3)_2$ | 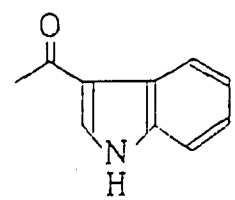 | H |
| $-OCH_3$ | $-OCH_3$ | $-CH(CH_3)_2$ | 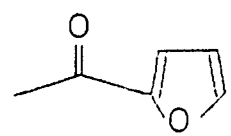 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 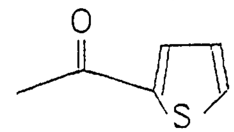 | H |
| $-OCH_3$ | $-OCH_3$ | $-CH(CH_3)CH_2CH_3$ | 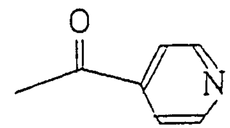 | H |
| $-OCH_3$ | $-OCH_3$ | $-CH(CH_3)CH_2CH_3$ | 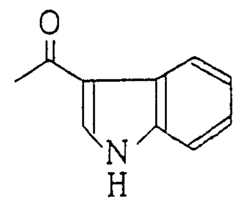 | H |
| $-OCH_3$ | $-OCH_3$ | $-CH(CH_3)CH_2CH_3$ | 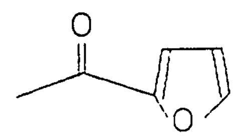 | H |
| $-OCH_3$ | $-OCH_3$ | $-C(CH_3)_3$ | 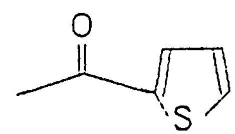 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 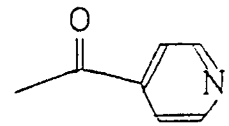 | H |
| $-OCH_3$ | $-OCH_3$ | $-C(CH_3)_3$ | 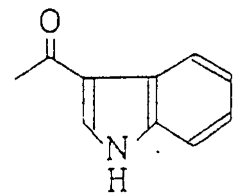 | H |
| $-OCH_3$ | $-OCH_3$ | $-C(CH_3)_3$ | 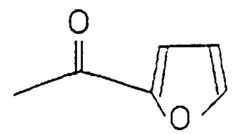 | H |
| $-OCH_3$ | $-OCH_3$ | $-CH_2CH_2CH_3$ | 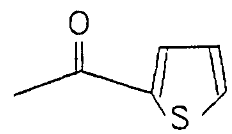 | H |
| $-OCH_3$ | $-OCH_3$ | $-C_2H_5$ | 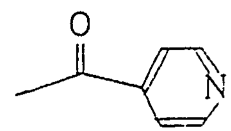 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH_2CH(CH_3)_2$ | 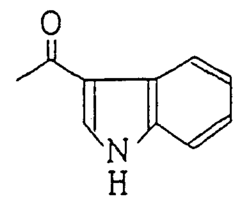 | H |
| $-OCH_3$ | $-OCH_3$ | 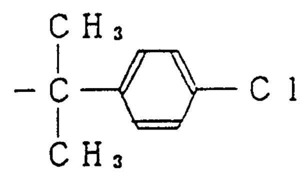 | 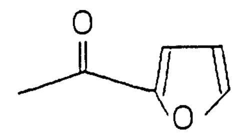 | H |
| $-OCH_3$ | $-OCH_3$ | $-C(CH_3)_3$ | 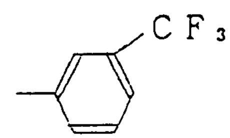 | H |
| $-OCH_3$ | $-OCH_3$ | 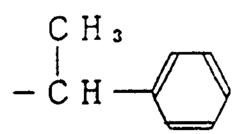 | H | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 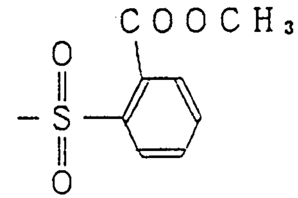 | H |
| $-OCH_3$ | $-OCH_3$ | 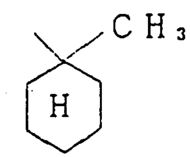 | H | H |
| $-OCH_3$ | $-OCH_3$ | 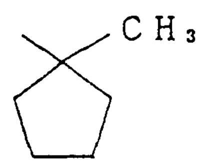 | H | H |
| $-OCH_3$ | $-OCH_3$ | 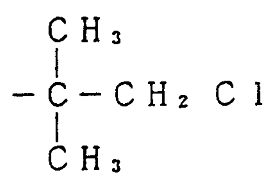 | H | H |

Table 1 (Continued)

| $R^1$ | $R^2$ | $R^3$ | $R^4$ | $R^5$ |
|---|---|---|---|---|
| $-OCH_3$ | $-OCH_3$ | 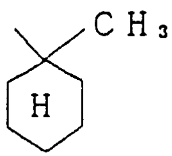 | 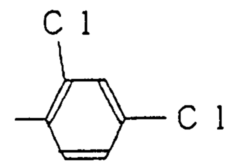 | H |
| $-OCH_3$ | $-OCH_3$ | 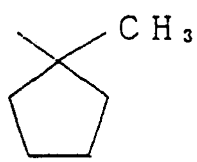 | 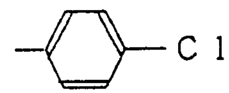 | H |
| $-OCH_3$ | $-OCH_3$ | 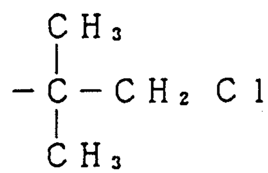 | 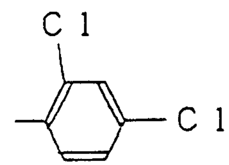 | H |
| $-OCH_3$ | $-OCH_3$ | 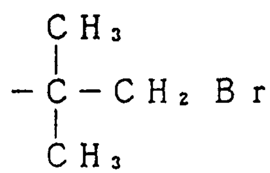 | 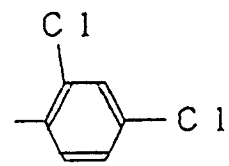 | H |

Table 1 (Continued)

| $R^1$ | $R^2$ | $R^3$ | $R^4$ | $R^5$ |
|---|---|---|---|---|
| $-OCH_3$ | $-OCH_3$ | 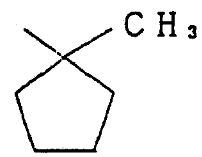 | 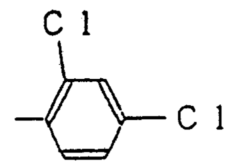 | H |
| $-OCH_3$ | $-OCH_3$ | $-CH_3$ | 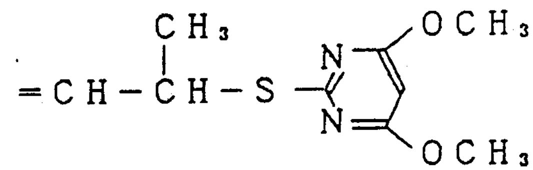 | |
| $-OCH_3$ | $-OCH_3$ | $-C_2H_5$ | 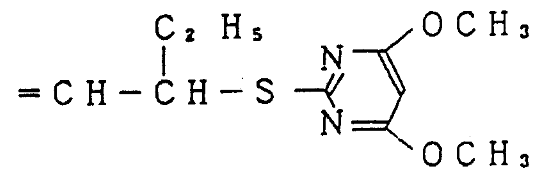 | |
| $-OCH_3$ | $-OCH_3$ | $-CH_2CH_2CH_3$ | 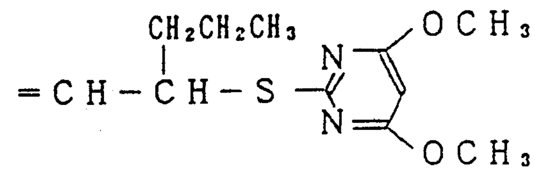 | |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 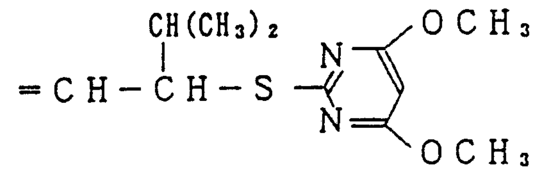 | |
| $-OCH_3$ | $-OCH_3$ | $-CH_2CH_2CH_2CH_3$ | 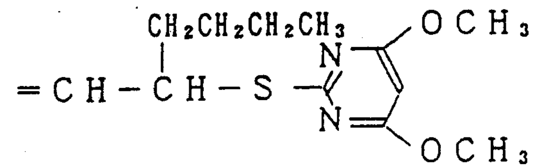 | |
| $-OCH_3$ | $-OCH_3$ | $-C(CH_3)_3$ | 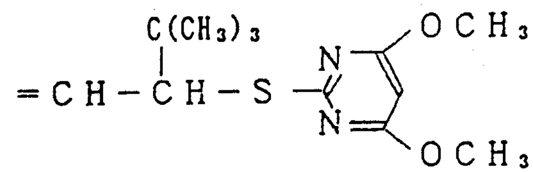 | |
| $-OCH_3$ | $-OCH_3$ | $-CH_2CH(CH_3)_2$ | 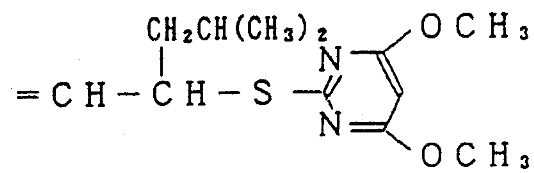 | |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 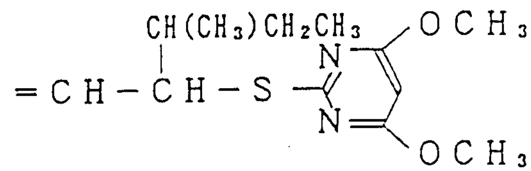 | |
| $-OCH_3$ | $-OCH_3$ | 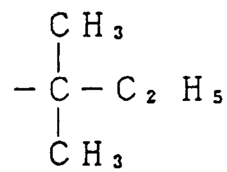 | 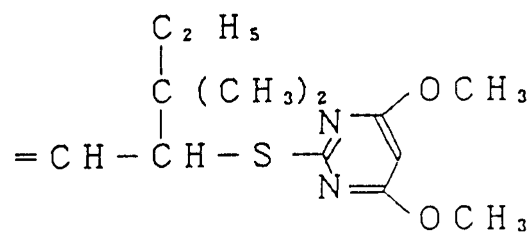 | |
| $-OCH_3$ | $-OCH_3$ | 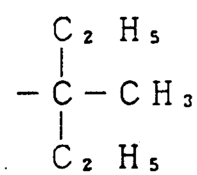 | 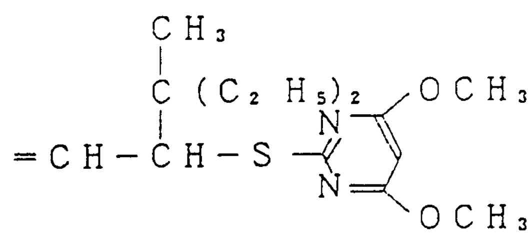 | |

Table 1 (Continued)

| $R^1$ | $R^2$ | $R^3$ | $R^4$ | $R^5$ |
|---|---|---|---|---|
| $-OCH_3$ | $-OCH_3$ | 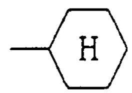 | 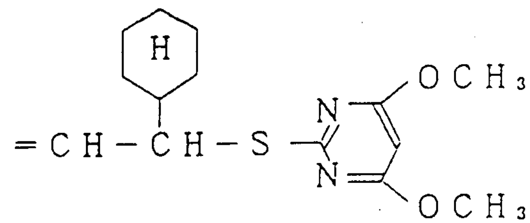 | |
| $-OCH_3$ | $-OCH_3$ | 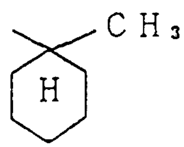 | 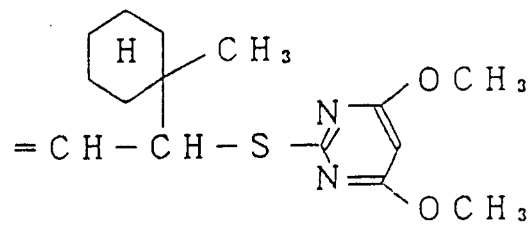 | |
| $-OCH_3$ | $-OCH_3$ | 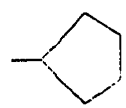 | 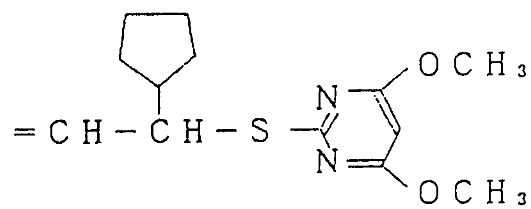 | |

Table 1 (Continued)

| $R^1$ | $R^2$ | $R^3$ | $R^4$ | $R^5$ |
|---|---|---|---|---|
| $-OCH_3$ | $-OCH_3$ | 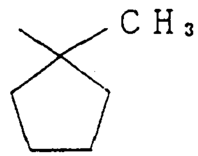 | 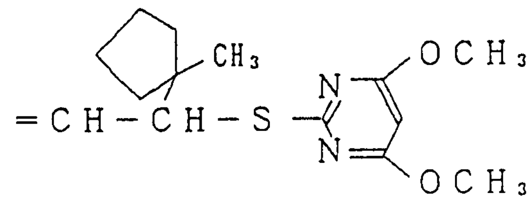 | |
| $-OCH_3$ | $-OCH_3$ | 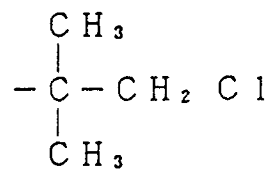 | 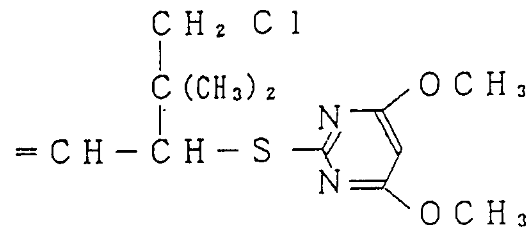 | |
| $-OCH_3$ | $-OCH_3$ | $-C(CH_3)_3$ | 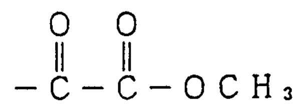 | H |
| $-OCH_3$ | $-OCH_3$ | $-CH(CH_3)_2$ | 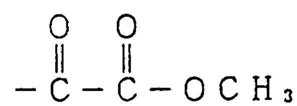 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | $-\overset{O}{\overset{\|}{C}}-NH-CH_3$ | H |
| $-OCH_3$ | $-OCH_3$ | $-C(CH_3)_3$ | $-\overset{O}{\overset{\|}{C}}-NH-C_2H_5$ | H |
| $-OCH_3$ | $-OCH_3$ | $-C(CH_3)_3$ | $-\overset{O}{\overset{\|}{C}}-NH-CH_2CH_2CH_3$ | H |
| $-OCH_3$ | $-OCH_3$ | $-C(CH_3)_3$ | $-\overset{O}{\overset{\|}{C}}-NH-(CH_2)_3CH_3$ | H |
| $-OCH_3$ | $-OCH_3$ | $-C(CH_3)_3$ | $-\overset{O}{\overset{\|}{C}}-N\begin{matrix}CH_3\\CH_3\end{matrix}$ | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 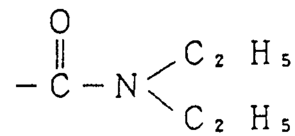 | H |
| $-OCH_3$ | $-OCH_3$ | $-C(CH_3)_3$ | 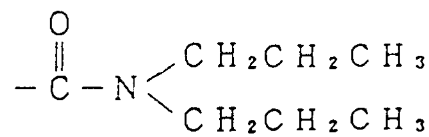 | H |
| $-OCH_3$ | $-OCH_3$ | $-C(CH_3)_3$ | 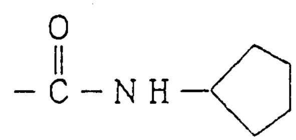 | H |
| $-OCH_3$ | $-OCH_3$ | $-C(CH_3)_3$ | 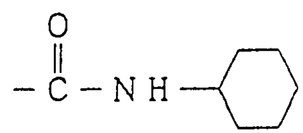 | H |
| $-OCH_3$ | $-OCH_3$ | $-C(CH_3)_3$ | 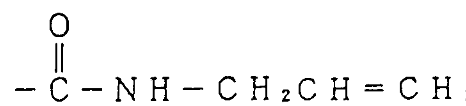 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 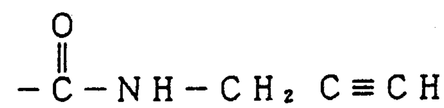 | H |
| $-OCH_3$ | $-OCH_3$ | $-C(CH_3)_3$ | 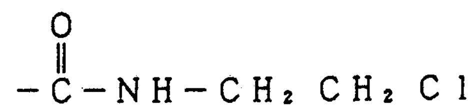 | H |
| $-OCH_3$ | $-OCH_3$ | $-C(CH_3)_3$ | 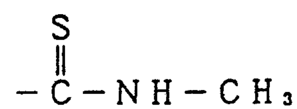 | H |
| $-OCH_3$ | $-OCH_3$ | $-C(CH_3)_3$ | 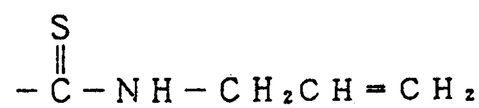 | H |
| $-OCH_3$ | $-OCH_3$ | $-C(CH_3)_3$ | 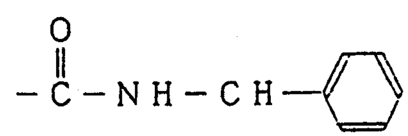 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 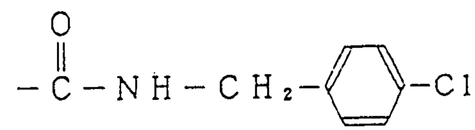 | H |
| $-OCH_3$ | $-OCH_3$ | $-C(CH_3)_3$ | 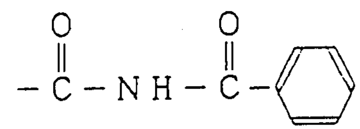 | H |
| $-OCH_3$ | $-OCH_3$ | $-C(CH_3)_3$ | 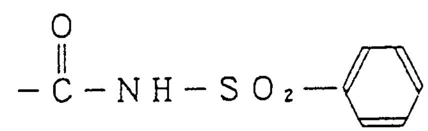 | H |
| $-OCH_3$ | $-OCH_3$ | $-C(CH_3)_3$ | 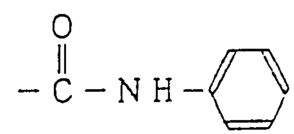 | H |
| $-OCH_3$ | $-OCH_3$ | $-C(CH_3)_3$ | 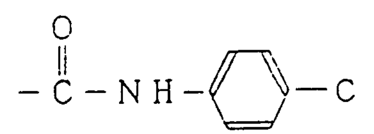 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 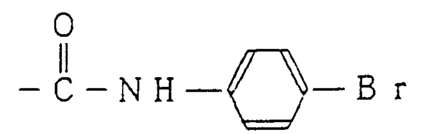 | H |
| $-OCH_3$ | $-OCH_3$ | $-C(CH_3)_3$ | 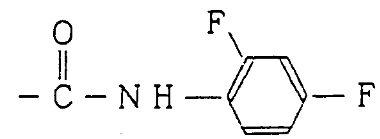 | H |
| $-OCH_3$ | $-OCH_3$ | $-C(CH_3)_3$ | 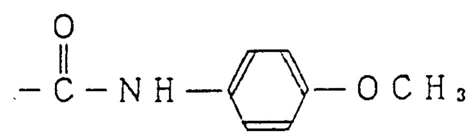 | H |
| $-OCH_3$ | $-OCH_3$ | $-C(CH_3)_3$ | 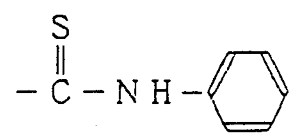 | H |
| $-OCH_3$ | $-OCH_3$ | $-C(CH_3)_3$ | 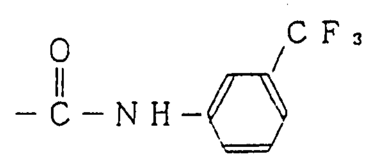 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 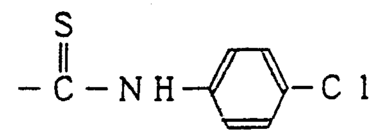 | H |
| $-OCH_3$ | $-OCH_3$ | $-C(CH_3)_3$ | 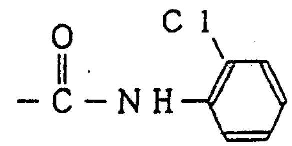 | H |
| $-OCH_3$ | $-OCH_3$ | $-CH(CH_3)_2$ | $-C(=O)-NH-CH_3$ | H |
| $-OCH_3$ | $-OCH_3$ | $-CH(CH_3)_2$ | $-C(=O)-NH-C_2H_5$ | H |
| $-OCH_3$ | $-OCH_3$ | $-CH(CH_3)_2$ | 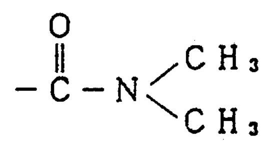 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 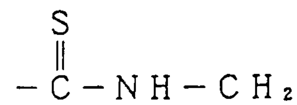 | H |
| $-OCH_3$ | $-OCH_3$ | $-CH(CH_3)_2$ | 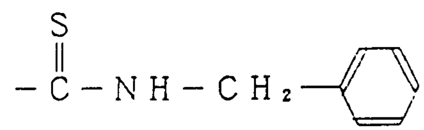 | H |
| $-OCH_3$ | $-OCH_3$ | $-CH(CH_3)_2$ | 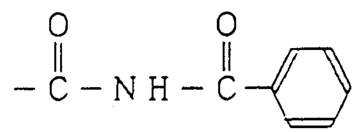 | H |
| $-OCH_3$ | $-OCH_3$ | $-CH(CH_3)_2$ | 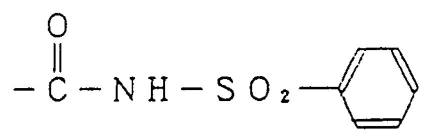 | H |
| $-OCH_3$ | $-OCH_3$ | $-CH(CH_3)_2$ | 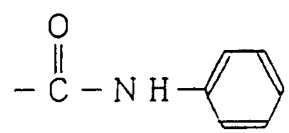 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 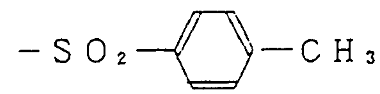 | H |
| $-OCH_3$ | $-OCH_3$ | $-C(CH_3)_3$ | 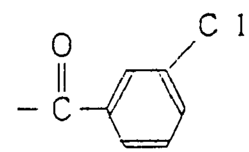 | H |
| $-OCH_3$ | $-OCH_3$ | $-C(CH_3)_3$ | 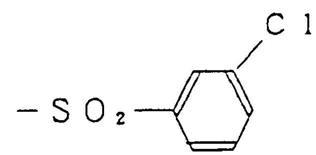 | H |
| $-OCH_3$ | $-OCH_3$ | $-C(CH_3)_3$ | 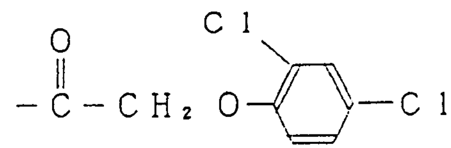 | H |
| $-OCH_3$ | $-OCH_3$ | 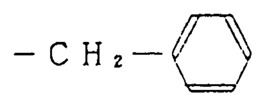 | 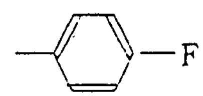 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 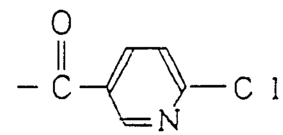 | H |
| $-OCH_3$ | $-OCH_3$ | $-C(CH_3)_3$ | 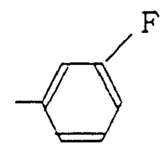 | H |
| $-OCH_3$ | $-OCH_3$ | $-C(CH_3)_3$ | 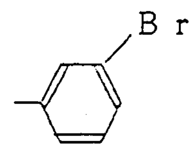 | H |
| $-OCH_3$ | $-OCH_3$ | $-C(CH_3)_3$ | 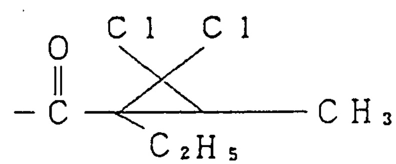 | H |
| $-OCH_3$ | $-OCH_3$ | $-C(CH_3)_3$ | 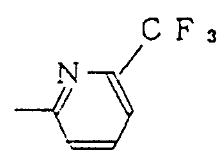 | $CH_3$ |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 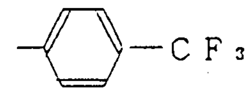 | H |
| $-OCH_3$ | $-OCH_3$ | $-C(CH_3)_3$ | 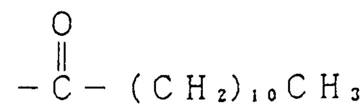 | H |
| $-OCH_3$ | $-OCH_3$ | $-C(CH_3)_3$ | 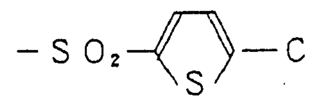 | H |
| $-OCH_3$ | $-OCH_3$ | $-C(CH_3)_3$ | 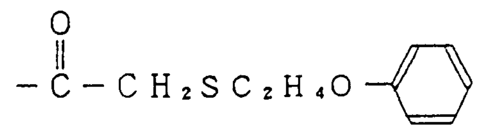 | H |
| $-OCH_3$ | $-OCH_3$ | $-C(CH_3)_3$ | 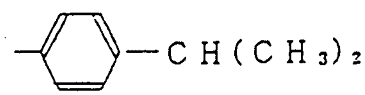 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 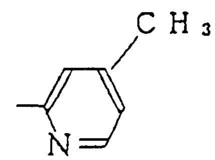 | $CH_3$ |
| $-OCH_3$ | $-OCH_3$ | $-C(CH_3)_3$ | 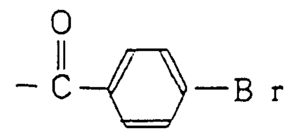 | H |
| $-OCH_3$ | $-OCH_3$ | $-C(CH_3)_3$ | 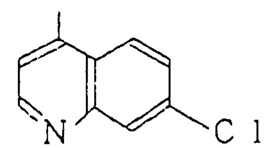 | H |
| $-OCH_3$ | $-OCH_3$ | $-C(CH_3)_3$ | 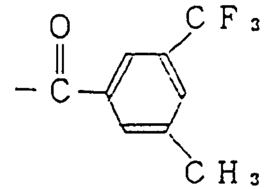 | H |
| $-OCH_3$ | $-OCH_3$ | $-C(CH_3)_3$ | 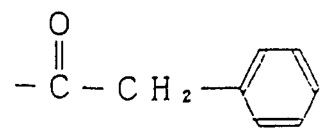 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 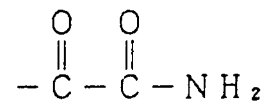 | H |
| $-OCH_3$ | $-OCH_3$ | $-C(CH_3)_3$ | 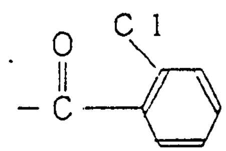 | H |
| $-OCH_3$ | $-OCH_3$ | $-C(CH_3)_3$ | 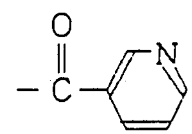 | H |
| $-OCH_3$ | $-OCH_3$ | $-C(CH_3)_3$ | 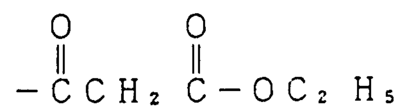 | H |
| $-OCH_3$ | $-OCH_3$ | $-C(CH_3)_3$ | 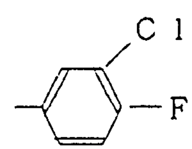 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | $-\overset{O}{\overset{\|}{C}}(CH_2)_4\overset{O}{\overset{\|}{C}}NHNH_2$ | H |
| $-OCH_3$ | $-OCH_3$ | $-C(CH_3)_3$ | 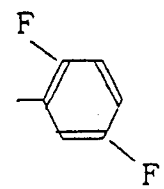 | H |
| $-OCH_3$ | $-OCH_3$ | $-C(CH_3)_3$ | $-\overset{O}{\overset{\|}{C}}-NHNH_2$ | H |
| $-OCH_3$ | $-OCH_3$ | $-C(CH_3)_3$ | $-\overset{O}{\overset{\|}{C}}-NHNH-CH_3$ | H |
| $-OCH_3$ | $-OCH_3$ | $-C(CH_3)_3$ | $-\overset{O}{\overset{\|}{C}}-NHNH-C_2H_5$ | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 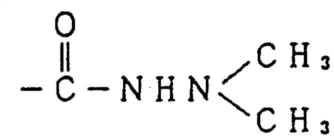 | H |
| $-OCH_3$ | $-OCH_3$ | $-C(CH_3)_3$ | 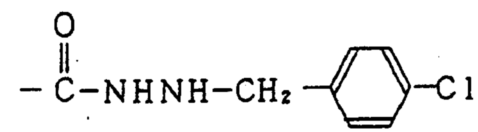 | H |
| $-OCH_3$ | $-OCH_3$ | $-C(CH_3)_3$ | 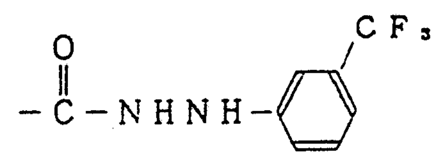 | H |
| $-OCH_3$ | $-OCH_3$ | $-CH(CH_3)_2$ | 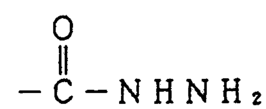 | H |
| $-OCH_3$ | $-OCH_3$ | $-CH(CH_3)_2$ | 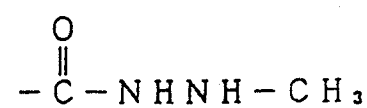 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 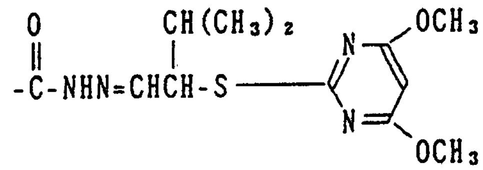 | H |
| $-OCH_3$ | $-OCH_3$ | $-C(CH_3)_3$ | 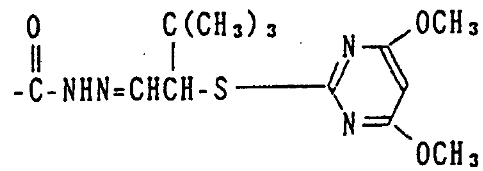 | H |
| $-OCH_3$ | $-OCH_3$ | $-CH(CH_3)_2$ | 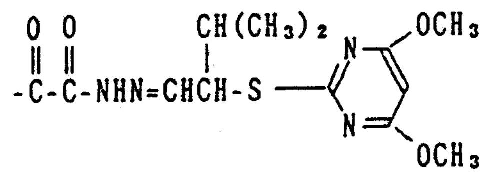 | H |
| $-OCH_3$ | $-OCH_3$ | $-C(CH_3)_3$ | 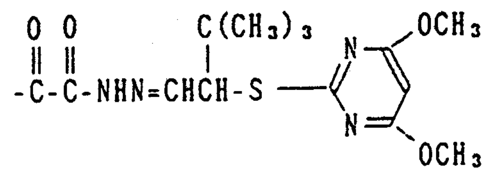 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 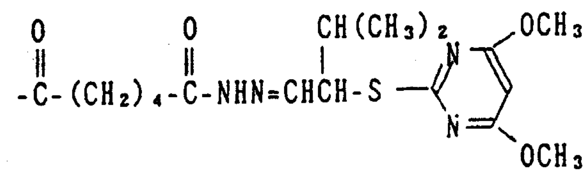 | H |
| $-OCH_3$ | $-OCH_3$ | $-C(CH_3)_3$ | 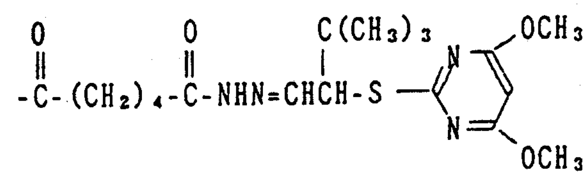 | H |
| $-OCH_3$ | $-OCH_3$ | $-C(CH_3)_3$ | 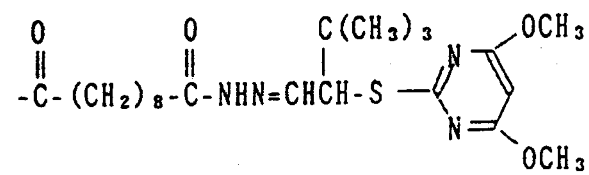 | H |
| $-OCH_3$ | $-OCH_3$ | $-C(CH_3)_3$ | 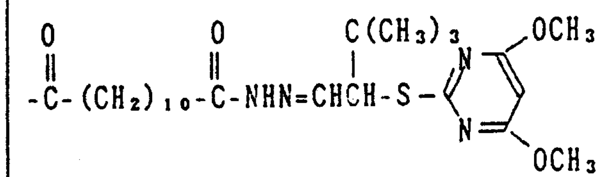 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 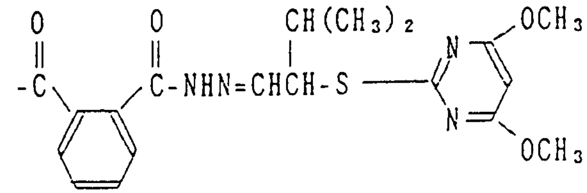 | H |
| $-OCH_3$ | $-OCH_3$ | $-C(CH_3)_3$ | 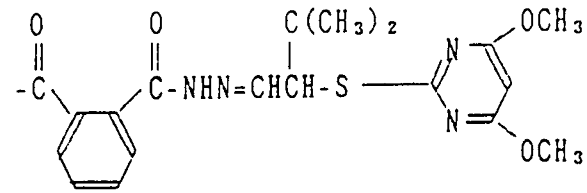 | H |
| $-OCH_3$ | $-OCH_3$ | $-CH(CH_3)_2$ | 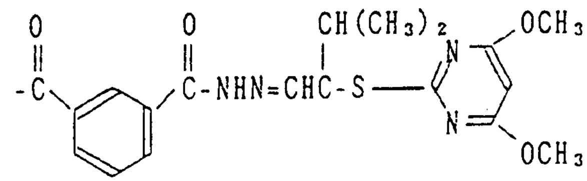 | H |
| $-OCH_3$ | $-OCH_3$ | $-C(CH_3)_3$ | 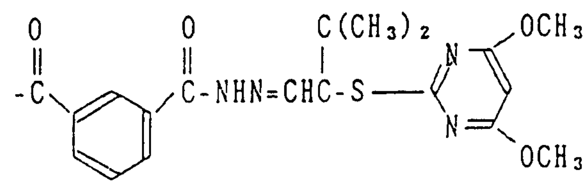 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 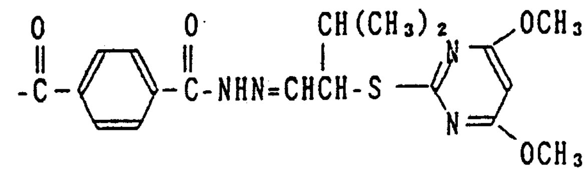 | H |
| $-OCH_3$ | $-OCH_3$ | $-C(CH_3)_3$ | 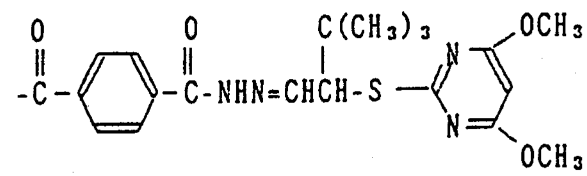 | H |
| $-OCH_3$ | $-OCH_3$ | $-CH(CH_3)_2$ | $-\overset{O}{\overset{\|}{C}}-(CH_2)_4-\overset{O}{\overset{\|}{C}}-NHNH_2$ | H |
| $-OCH_3$ | $-OCH_3$ | $-C(CH_3)_3$ | $-\overset{O}{\overset{\|}{C}}-(CH_2)_4-\overset{O}{\overset{\|}{C}}-NHNH-CH_3$ | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 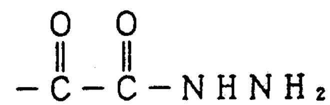 | H |
| $-OCH_3$ | $-OCH_3$ | $-C(CH_3)_3$ | 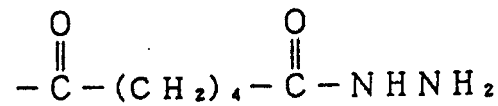 | H |
| $-OCH_3$ | $-OCH_3$ | $-C(CH_3)_3$ | 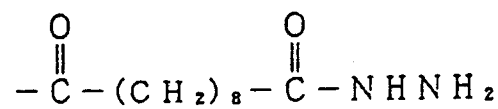 | H |
| $-OCH_3$ | $-OCH_3$ | $-C(CH_3)_3$ | 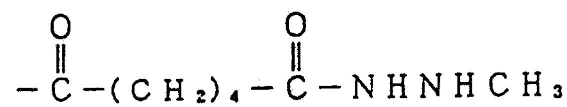 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 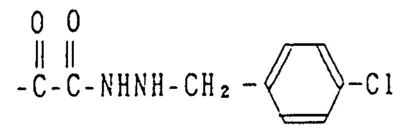 | H |
| $-OCH_3$ | $-OCH_3$ | $-C(CH_3)_3$ | 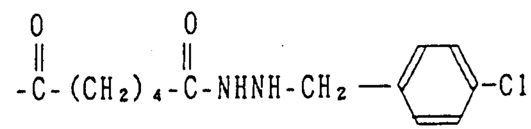 | H |
| $-OCH_3$ | $-OCH_3$ | $-C(CH_3)_3$ | 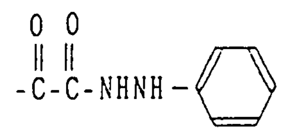 | H |
| $-OCH_3$ | $-OCH_3$ | $-C(CH_3)_3$ | 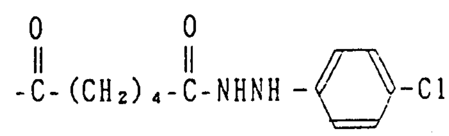 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 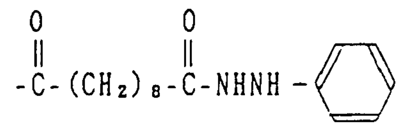 | H |
| $-OCH_3$ | $-OCH_3$ | $-CH(CH_3)_2$ | 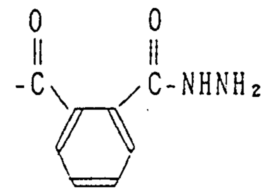 | H |
| $-OCH_3$ | $-OCH_3$ | $-CH(CH_3)_2$ | 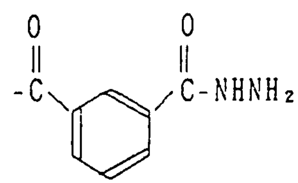 | H |
| $-OCH_3$ | $-OCH_3$ | $-CH(CH_3)_2$ | 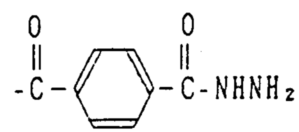 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 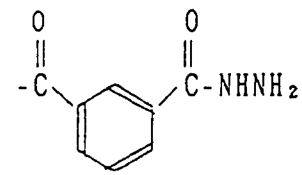 | H |
| $-OCH_3$ | $-OCH_3$ | $-C(CH_3)_3$ | 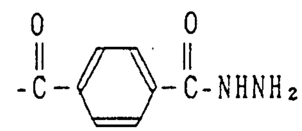 | H |
| $-OCH_3$ | $-OCH_3$ | $-C(CH_3)$ | 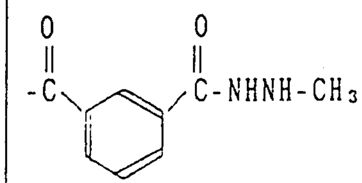 | H |
| $-OCH_3$ | $-OCH_3$ | $-C(CH_3)$ | 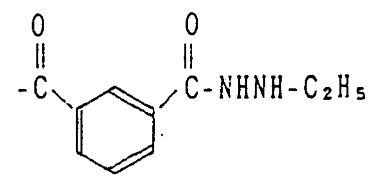 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 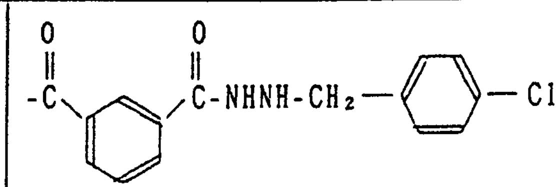 | H |
| $-OCH_3$ | $-OCH_3$ | $-C(CH_3)_3$ | 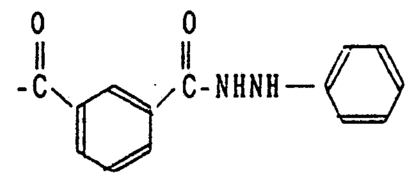 | H |
| $-OCH_3$ | $-OCH_3$ | $-C(CH_3)_3$ | 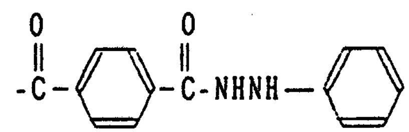 | H |
| $-OCH_3$ | $-OCH_3$ | $-C(CH_3)_3$ | 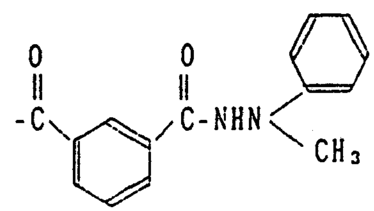 | H |

Table 1 (Continuei)

| $R^1$ | $R^2$ | $R^3$ | $R^4$ | $R^5$ |
|---|---|---|---|---|
| $-OCH_3$ | $-OCH_3$ | $-C(CH_3)_3$ | 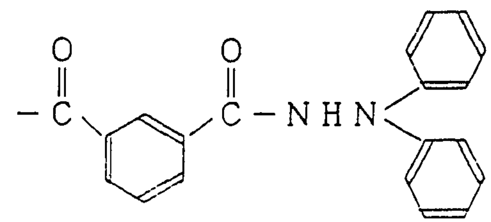 | H |
| $-OCH_3$ | $-OCH_3$ | $-C(CH_3)_3$ | 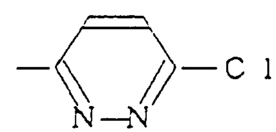 | H |

Table 1 (Continued)

| $R^1$ | $R^2$ | $R^3$ | $R^4$ | $R^5$ |
|---|---|---|---|---|
| $-OCH_3$ | $-OCH_3$ | $-C(CH_3)_3$ | 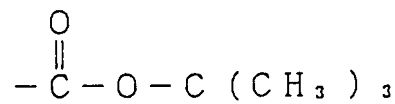 | H |
| $-OCH_3$ | $-OCH_3$ | $-CH(CH_3)_2$ | 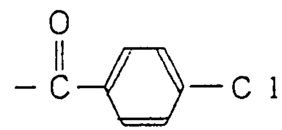 | H |
| $-OCH_3$ | $-OCH_3$ | $-C(CH_3)_3$ | $-SO_2-CH_3$ | H |
| $-OCH_3$ | $-OCH_3$ | $-C(CH_3)_3$ | 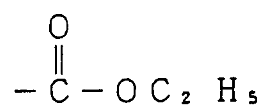 | H |
| $-OCH_3$ | $-OCH_3$ | $-C(CH_3)_3$ | 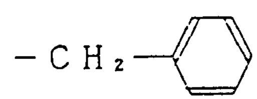 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)CH_2CH_3$ | 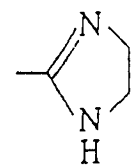 | H |
| $-OCH_3$ | $-OCH_3$ | $-CH(CH_3)CH_2CH_3$ | 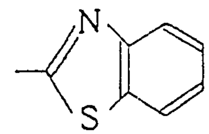 | H |
| $-OCH_3$ | $-OCH_3$ | $-CH(CH_3)CH_2CH_3$ | 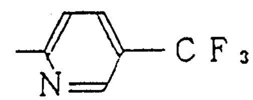 | H |
| $-OCH_3$ | $-OCH_3$ | $-CH(CH_3)CH_2CH_3$ | 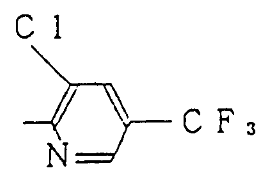 | H |
| $-OCH_3$ | $-OCH_3$ | $-CH(CH_3)CH_2CH_3$ | 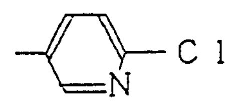 | H |

Table 1 (Continued)

| $R^1$ | $R^2$ | $R^3$ | $R^4$ | $R^5$ |
|---|---|---|---|---|
| $-OCH_3$ | $-OCH_3$ | $-CH(CH_3)_2$ | 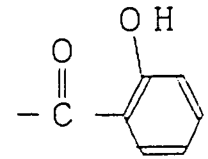 | H |
| $-OCH_3$ | $-OCH_3$ | $-CH(CH_3)_2$ | 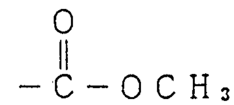 | H |
| $-OCH_3$ | $-OCH_3$ | $-CH(CH_3)_2$ | $-SO_2CH_3$ | H |
| $-OCH_3$ | $-OCH_3$ | $-CH(CH_3)_2$ | 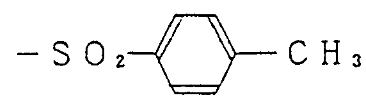 | H |
| $-OCH_3$ | $-OCH_3$ | $-CH(CH_3)_2$ | 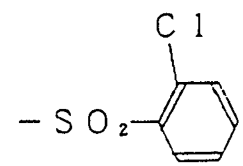 | H |
| $-OCH_3$ | $-OCH_3$ | $-CH(CH_3)_2$ | 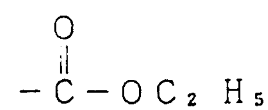 | H |

Table 1 (Continued)

| $R^1$ | $R^2$ | $R^3$ | $R^4$ | $R^5$ |
|---|---|---|---|---|
| $-OCH_3$ | $-OCH_3$ | 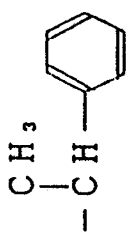 | $-C(CH_3)_3$ | H |
| $-OCH_3$ | $-OCH_3$ | $-C(CH_3)_3$ | 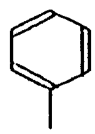 | 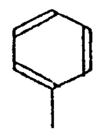 |
| $-OCH_3$ | $-OCH_3$ | $-C(CH_3)_3$ | 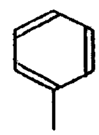 | $-CH_3$ |
| $-OCH_3$ | $-OCH_3$ | $-C(CH_3)_3$ | 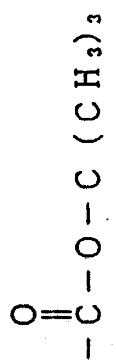 | H |
| $-OCH_3$ | $-OCH_3$ | 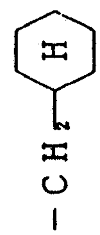 | $-CH_3$ | H |
| $-OCH_3$ | $-OCH_3$ | $-CH(CH_3)_2$ | 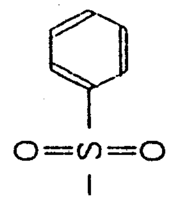 | H |

When in the process (a), if, for example, 2-(4,6-dimethoxy-2-pyrimidinylthio)-3-methylbutanal and N,N-dimethylhydrazine are used as starting materials, the course of the reaction can be represented by the following equation:

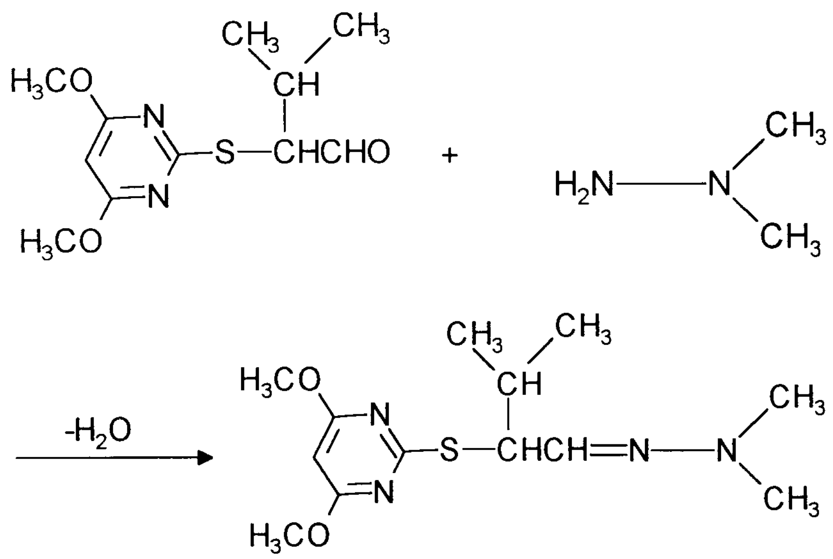


When in the process (b), if, for example, 2-(4,6-dimethoxy-2-pyrimidinylthio)-3-methylbutanal and hydrazine are used as starting materials, the course of the reaction can be represented by the following equation:

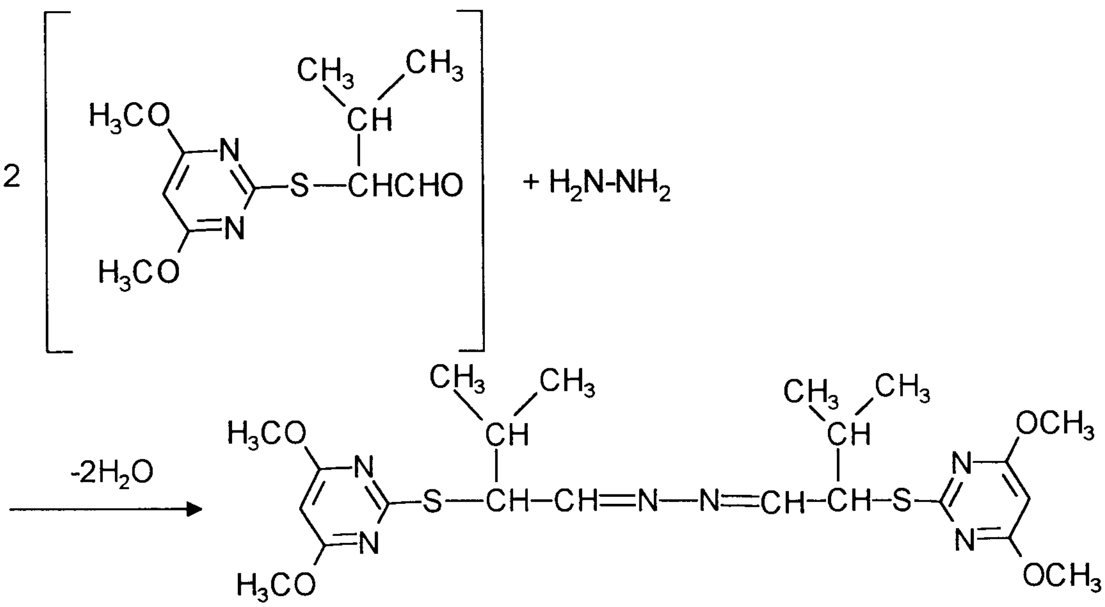


When in the process (c), if, for example, 2-(4,6-dimethoxy-2-pyrimidinylthio)-3-methylbutanal hydrazone and benzenesulfonyl chloride are used as starting materials, the course of the reaction can be represented by the following equation:

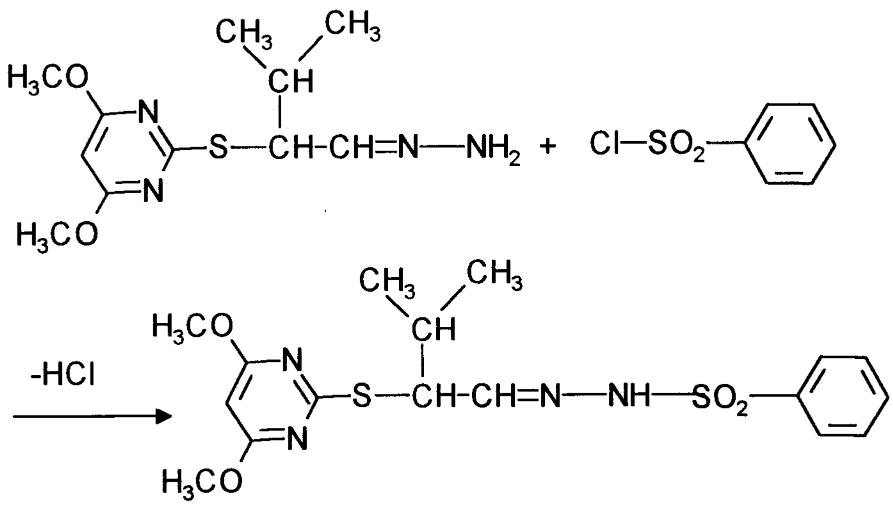


When in the process (d), if, for example, 2-(4,6-dimethoxy-2-pyrimidinylthio-)-3-methylbutanal hydrazone and propionic anhydride are used as starting materials, the course of the reaction can be represented by the following equation:

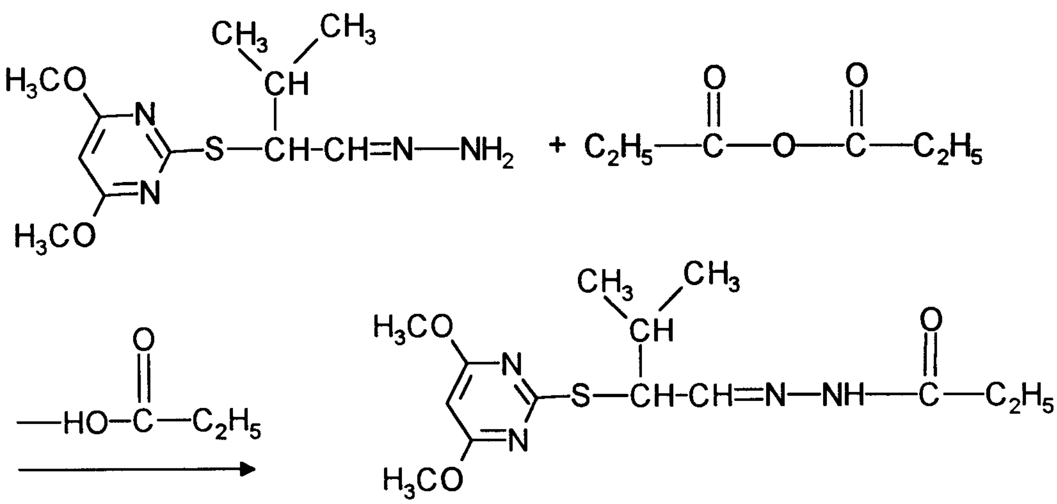

When in the process (e), if, for example, 2-(4,6-dimethoxy-2-pyrimidinylthio)-3-methylbutanal hydrazone and ethyl oxalyl chloride are used as starting materials, the course of the reaction can be represented by the following equation:

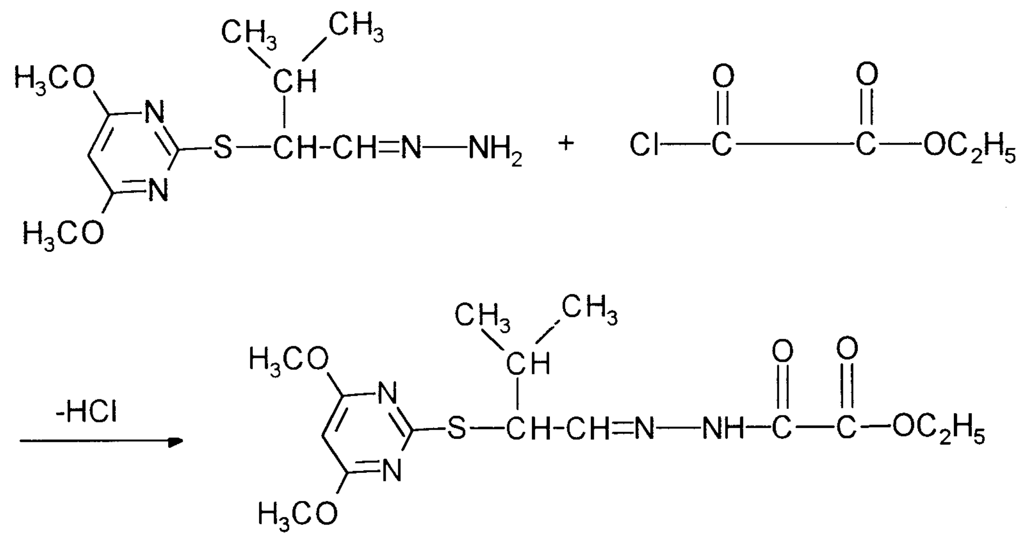

In the process (a) and (b), the starting compounds of the formula (II) mean compounds based on the above definitions of $R^1$, $R^2$ and $R^3$, preferably substituents based on the above preferred definitions of $R^1$, $R^2$ and $R^3$.

The compounds of the formula (II) can be obtained in the same way as described in Japanese Patent Application No. 118450/1992.

As specific examples of the formula (II), there may be mentioned:
2-(4,6-dimethoxy-2-pyrimidinylthio)-3-methylbutanal,
2-(4,6-dimethoxy-2-pyrimidinylthio)-3,3-dimethylbutanal,
2-(4,6-dimethoxy-2-pyrimidinylthio)-2-cyclohexylethanal,
2-(4,6-dimethoxy-2-pyrimidinylthio)-3-butanal,
2-(4,6-dimethoxy-2-pyrimidinylthio)-3-pentanal,
2-(4,6-dimethoxy-2-pyrimidinylthio)-3-cyclohexylpropanal,
2-(4,6-dimethoxy-2-pyrimidinylthio)-3-methylpentanal,
2-(4,6-dimethoxy-2-pyrimidinylthio)-4-methylpentanal,
2-(4,6-dimethoxy-2-pyrimidinylthio)-3-phenyl-3-methylbutanal, and
2-(4,6-dimethoxy-2-pyrimidinylthio)-3-phenylpropanal.

In the process (a), as specific examples of the hydrazine derivatives of the formula (III) mean compounds based on the above definitions of $R^4$ and $R^5$, preferably based on the above preferred definitions of $R^4$ and $R^5$.

The hydrazine derivatives of the formula (III) are well known in the field of organic chemistry, and as the specific examples of the formula (III), there may be mentioned:
hydrazine,
N,N-dimethylhydrazine,
N,N-diphenylhydrazine,
phenylhydrazine,
p-chlorophenylhydrazine,
2,4-dichlorophenylhydrazine,
tert-butylhydrazine,
p-bromophenylhydrazine,
methylhydrazine,
semicarbazide,
thiosemicarbazide.

In the processes (c), (d) and (e), the starting compounds of the formula (Ia) mean compounds based on the above definitions of $R^1$, $R^2$ and $R^3$, preferably substituents based on the above preferred definitions of $R^1$, $R^2$ and $R^3$. The starting compounds of the formula (Ia) can be obtained by the process (a).

As specified examples of the formula (Ia), there may be mentioned:
2-(4,6-dimethoxy-2-pyrimidinylthio)-3-methylbutanal hydrazone,
2-(4,6-dimethoxy-2-pyrimidinylthio)-3,3-dimethylbutanal hydrazone,
2-(4,6-dimethoxy-2-pyrimidinylthio)-2-cyclohexylethanal hydrazone,

2-(4,6-dimethoxy-2-pyrimidinylthio)-3-butanal hydrazone, and
2-(4,6-dimethoxy-2-pyrimidinylthio)-3-pentanal hydrazone.

In the processes (c) the starting compounds of the formula (IV) mean compounds based on the above definitions of $R^6$ and $R^{12}$, preferably substituents based on the above preferred definitions of $R^6$, $R^{12}$ preferably represents chloro or bromo.

The starting compounds of the formula (IV) are well known compounds in the field of organic chemistry. As specific examples of the compounds of the formula (IV) there may be mentioned:
benzenesulfonylchloride,
3-methoxycarbonylbenzenesulfonylchloride,
p-toluenesulfonylchloride, and
methanesulfonylchloride.

In the processes (d) the starting compounds of the formula (V) mean compounds based on the above definitions of $R^8$, preferably substituents based on the above preferred definitions of $R^8$.

The starting compounds of the formula (V) are well known compounds in the field of organic chemistry. As specific examples of the compounds of the formula (V) there may be mentioned:
propionic anhydride,
pivalic anhydride, and
acetic anhydride.

In the process (e) the starting compounds of the formula (VI) mean compounds based on the above definitions of $R^6$, $R^7$ and $R^{12}$, preferably substituents based on the above preferred definitions of $R^6$, $R^7$ and $R^{12}$.

The starting compounds of the formula (VI) are well known compounds in the field of organic chemistry. As specific examples of the compounds of the formula (VI) there may be mentioned:
methyl oxalyl chloride or bromide,
ethyl oxalyl chloride or bromide,
2-(or 3-) thenoyl chloride or bromide,
2-(or 3-)furoyl chloride or bromide,
isonicotinoyl chloride or bromide,
benzoylchloride or bromide.

In carrying out the process (a) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, tetrachloromethane, 1,2-dichloroethane, chlorobenzene, dichlorobenzene and the like; ethers such as diethyl ether, methyl ethyl ether, diisopropyl ether, dibutyl ether, dioxane, dimethoxyethane(DME), tetrahydrofurane (THF) and the like; ketones such as acetone, methylethyl ketone (MEK), methyl-iso-propyl ketone, methyl-iso-butyl ketone (MIBK) and the like; nitriles such as acetonitrile, propionitrile and the like; alcohols such as methanol, ethanol, isopropanol, butanol, ethylene glycol and the like; esters such as ethyl acetate, amyl acetate and the like, acid amides such as dimethyl formamide (DMF), dimethyl acetamide (DMA) and the like; sulfones and sulfoxides such as dimethyl sulfoxide (DMSO), sulfolane and the like; and bases such as pyridine.

The process (a) according to the invention is carried out preferably in the presence of an acid or acid binder.

As example of such acid binder may be mentioned: inorganic bases including hydroxide, carbonate, bicarbonate, alcolate of alkali metals such as, for example, sodium hydrogen carbonate, potassium hydrogen carbonate, sodium carbonate, potassium carbonate, organic bases including tertiary amines, dialkylaminoanilines, and pyridines such as, for example, triethylamine, tributylamine, 1,1,4,4-tetramethylenediamine (TMEDA), N,N-dimethylaniline, N,N-diethylaniline, pyridine, 4-dimethylamino pyridine (DMAP), 1,4-diaza-bicyclo-[2,2,2]octane (DABCO), 1,8-diazabicyclo[5,4,0]-undec-7-ene (DBU) and the like.

As example of such acid may be mentioned: hydrochloric acid, hydrobromic acid, p-toluene sulfonic acid, trifluoro acetic acid.

In the above mentioned process (a), the reaction temperature can be varied within a substantially wide range. In general, the reaction is carried out at a temperature of from about -40 °C to about 200 °C, preferably from 0 °C to about 120 °C.

Further, the reaction is carried out under normal pressure, although it is also possible to employ a higher or reduced pressure.

When the above mentioned process (a) according to the present invention is carried out, use is made, for example, about 1.0 to 2.0 mols of the compound of the formula (III) in a diluent such as acetonitrile per 1

mol of the compounds represented by the general formula (II) to obtain the desired compounds.

In carrying out the process (b) mentioned above, use may be made of the same reaction conditions as those of the process (a) in the presence of acids.

When the above mentioned process (b) according to the present invention is carried out, use is made, for instance, 2.0 to 2.3 mols of the compounds of the formula (II) in a diluent per 1 mol of the compounds of hydrazine to obtain the desired compounds.

In carrying out the process (c) mentioned above, use may be made, as suitable diluent, of any inert solvent.

Examples of such diluents are water; aliphaltic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethyl-ether (DGM), and the like; ketones such as acetone, methylethyl ketone (MEK), methyl-iso-propyl ketone, methyl-iso-butyl ketone, and the like; nitriles such as acetonitrile, propionitrile acrylnitrile and the like; alcohols; methanol, ethanol, isopropanol, butanol, ethylene glycol and the like; esters such as ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and base such as pyridine.

The process (c) according to the invention is carried out preferably in the presence of acid binder. As examples of such acid binder may be mentioned:

inorganic bases including hydroxide, carbonate, bicarbonate, alcolate of alkali metals such as, for example, sodium hydrogen carbonate, potassium hydrogen carbonate, sodium carbonate, potassium carbonate, organic bases including tertiary amines, dialkylaminoanilines, and pyridines such as, for example, triethylamine, tributylamine, 1,1,4,4-tetra-methylenediamine (TMEDA), N,N-dimethylaniline, N,N-diethylaniline, pyridine, 4-dimethylamino pyridine (DMAP), 1,4-diaza-bicyclo-[2,2,2]octane (DABCO), 1,8-diazabicyclo[5,4,0]-undec-7-ene (DBU), organic lithium compounds such as methyl lithium, n-butyl lithium, sec-butyl lithium, tert-butyl lithium, phenyl lithium, dimethyl copper lithium, lithium diisopropyl amide, lithium cyclohexylisopropyl amide, lithium dicyclohexyl amide, n-butyl lithium • DABCO, n-butyl lithium • DBU, n-butyl lithium • TMEDA and the like.

In the above mentioned process (c), the reaction temperature can be varied within a substantially wide range. In general, the reaction is carried out at a temperature of from about -30 °C to about 100 °C, preferably from 0 °C to about 50 °C.

Further, the reaction is carried out under normal pressure, although it is also possible to employ a higher or reduced pressure.

When the above mentioned process (c) according to the present invention is carried out, use is made, for example, 1.0 to 1.2 mol of the compounds of the formulae (IV), (V) and (IV) in a diluent such as pyridine per mol of the compounds of the formula (Ia) to obtain the desired compounds.

In carrying out the process (d) mentioned above, use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethyl-ether (DGM), and the like; ketones; acetone, methylethyl ketone (MEK), methyl-iso-propyl ketone, methyl-iso-butyl ketone, and the like; nitriles such as acetonitrile, propionitrile acrylnitrile and the like; alcohols; methanol, ethanol, isopropanol, butanol, ethylene glycol and the like; esters such as ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and bases such as pyridine.

The process (d) according to the invention is carried out preferably in the presence of an acid binder. As examples of such an acid binder may be mentioned:

inorganic bases including hydroxide, carbonate, bicarbonate, alcolate of alkali metals such as, for example, sodium hydrogen carbonate, potassium hydrogen carbonate, sodium carbonate, potassium carbonate, organic bases, including tertiary amines, dialkylaminoanilines, and pyridines such as, for example, triethylamine, tributylamine, 1,1,4,4-tetral-methylenediamine (TMEDA), N,N-dimethylaniline, N,N-diethylaniline, pyridine, 4-dimethylamino pyridine (DMAP), 1,4-diaza-bicyclo-[2,2,2]octane (DABCO), 1,8-

dicabicyclo[5,4,0]-undec-7-ene (DBU), organic lithium compounds such as methyl lithium, n-butyl lithium, sec-butyl lithium, tert-butyl lithium, phenyl lithium, dimethyl copper lithium, lithium diisopropyl amide, lithium cyclohexylisopropyl amide, lithium dicyclohexyl amide, n-butyl lithium • DBU, n-butyl lithium • TMEDA and the like.

In the above mentioned process (d), the reaction temperature can be varied within a substantially wide range. In general, the reaction is carried out at a temperature of from about -30°C to about 100°C, preferably from 0°C to about 50°C.

Further, the reaction is carried out under normal pressure, although it is also possible to employ a higher or reduced pressure.

When the above mentioned process (d) according to the present invention is carried out, use is made, for example, 1.0 to 1.2 mol of the compounds of the formula (V) in a diluent such as pyridine per mol of the compounds of the formula (Ia) to obtain the desired compounds.

In carrying out the process (e) mentioned above, use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethyl-ether (DGM), and the like; ketones; acetone, methylethyl ketone (MEK), methy-iso-propyl ketone, methyl-iso-butyl ketone, and the like; nitriles such as acetonitrile, propionitrile, acrylnitrile and the like; alcohols; methanol, ethanol, isopropanol, butanol, ethylene glycol and the like; esters such as ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and bases such as pyridine.

The process (e) according to the invention is carried out preferably in the presence of acid binder. As examples of such an acid binder may be mentioned:
inorganic bases including hydroxide, carbonate, bicarbonate, alcolate of alkali metals such as, for example, sodium hydrogen carbonate, potassium hydrogen carbonate, sodium carbonate, potassium carbonate, organic bases including tertiary amines, dialkylaminoanilines, and pyridines such as, for example, triethylamine, tributylamine, 1,1,4,4-tetra-methylenediamine (TMEDA), N,N-dimethylaniline, N,N-diethylaniline, pyridine, 4-dimethylamino pyridine (DMPA), 1,4-diaza-bicyclo-[2,2,2]octane (DABCO), 1,8-diazabicyclo[5,4,0]-undec-7-ene (DBU), organic lithium compounds such as methyl lithium, n-butyl lithium, sec-butyl lithium, tert-butyl lithium, phenyl lithium, dimethyl copper lithium, lithium diisopropyl amide, lithium dicyclohexyl amide, n-butyl lithium • DABCO, n-butyl lithium • DBU, n-butyl lithium • TMEDA and the like.

In the above mentioned process (e), the reaction temperature can be varied within a substantially wide range. In general, the reaction is carried out at a temperature of from about -30°C to about 100°C, preferably from 0°C to about 50°C.

Further, the reaction is carried out under normal pressure, although it is also possible to employ a higher or reduced pressure.

When the above mentioned process (e) according to the present invention is carried out, use is made, for example, 1.0 to 1.2 mol of the compounds of the formula (IV) in a diluent such as pyridine per mol of the compounds of the formula (Ia) to obtain the desired compounds.

The active compounds according to the invention can be used as defoliants, desiccants, agents for destroying broad-leaved plants and, especially, as weed-killers. By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:
Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fim-

bristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, tablets, aerosols, natural and synthesis materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example, by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and groun synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorgamc and organic meals, and granules of organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcoholethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohols and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyatiine dyestuffs, and trace nutrients, such as salts of iron, manganese boron, copper, cobalt, molybdenum and zinc. The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.001 and 10 kg of active compound per hectare of soil surface, preferably between 0.01 and 5 kg per ha.

The preparation and use of the active compounds according to the invention can be seen from the following examples.

**Preparation Examples:**

Example 1

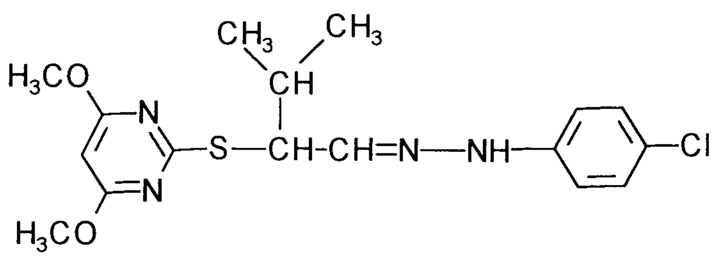

To an acetonitorile solution (60 ml) of 2-(4,6-dimethoxy-2-pirimidinylthio)-3-methylbutanal (5 g) and p-chlorophenylhydrazine hydrochloride (5.25 g) was added potassium carbonate, followed by a three-hours stirring at room temperature. After any unsoluble substance has been filtered off, the acetonitrile was removed from the reaction liquid under reduced pressure and the residue was purified through silica gel column chromatography (ethyl acetate : hexane = 1:5) to give 2(4,6-dimethoxy-2-pyrimidinylthio)-3-methylbutanal-4-chloro-phenylhydrazone (6.5 g).
$n_D^{20}$ = 1.5736

Example 2

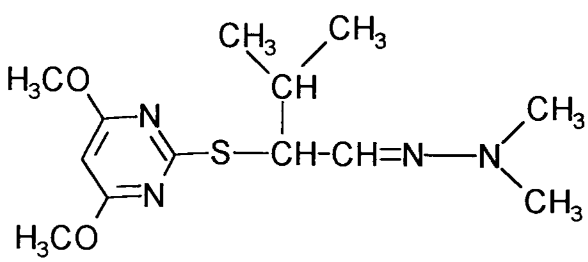

2(4,6-dimethoxy-2-pyrimidinylthio)-3-methylbutanal (2 g), and N,N-dimethyl-hydrazine (0.7 g) were stirred in acetonitril (50 ml) for ten hours at room temperature. The reaction liquid was distilled at reduced pressure to obtain a residue that was in turn purified under separation through a silica gel column chromatography (eluent: chloroform) to give 2-(4,6-dimethoxy-2-pyrimidinylthio)-3-methylbutanal dimethylhydrazone (2.05 g).
$n_D^{20}$ = 1.5254.

Example 3

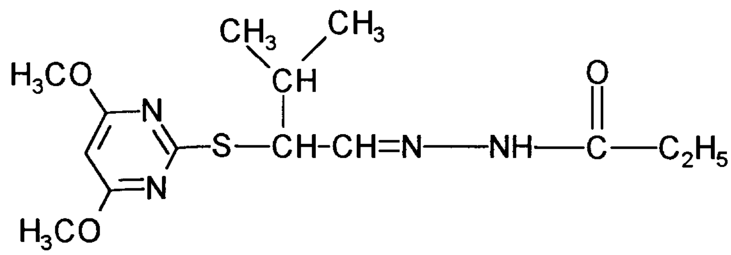

2-(4,6-dimethoxy-2-pyrimindinylthio)-3-methylbutanal hydrazone (1.0 g) was dissolved in pyridine (5 ml), followed by dropwise addition of propionic anhydride (5 ml) thereto at room temperature. After three hours stirring, the reaction liquid was poured onto ice water, followed by extraction with ethyl acetate and then by washing with 1N hydrochloric acid. After drying with anhydrous sodium sulfate, the solvent was distilled off at reduced pressure, to give an oily substance that was then purified through a silica gel column chromatography (ethyl acetate : hexane = 2 : 5) to obtain 2-(4,6-dimethoxy-2-pyrimidinylthio)-3-methylbutanal propionylhydrazone (0.8 g).
mp. 118.5 - 122.5 ° C

Example 4

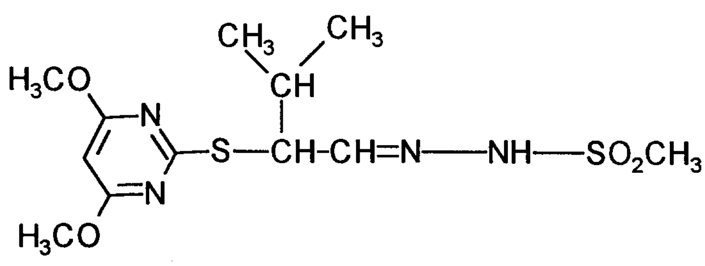

2-(4,6-dimethoxy-2-pyrimidinylthio)-3-methylbutanal hydrazone (1.0 g) was dissolved in pyridine (30 ml), followed by addition of methansulfonyl chloride (0.8 g) thereto at room temperature ad then by a three-hour stirring. The reaction liquid was poured into water (100 ml), followed by extraction with ethyl acetate and then by washing with water, 2N hydrochloric acid and water in that order. After drying with anhydrous sodium sulfate, the solvent was distilled off at reduced pressure, to obtain a crude product that was purified by silica gel column chromatography (ethyl acetate : hexane = 3 : 5) to obtain 2-(4,6-dimethoxy-2-pyrimidinylthio)-3-butanal methylsulfonylhydrazone (0.9 g).
$n_D^{20}$ = 1.5204

Example 5

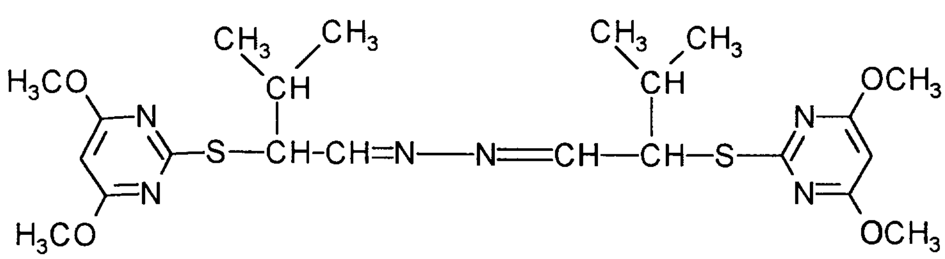

2-(4,6-dimethoxy-2-pyrimidinylthio)-3-methybutanal hydrazone (5.2 g) was dissolved in acetonitrile (50 ml), followed by dropwise addition of hydrazone anhydride (0.64 g) thereto at room temperature. After three-hour stirring, p-toluene sulfonic acid (0.1 g) was added to the reaction liquid, followed by further addition of an

acetonitrile solution of 2-(4,6-dimethoxy-2-pyrimidinylthio)-3-methylbutanal. After three-hour stirring, the solvent was removed from obtained crude product which was then purified by silica gel column chromatography (ethyl acetate : hexane = 15) to obtain 2-(4,6-dimethoxy-2-pyrimidinylthio)-3-methylbutanal azine (0.8 g).

$n_D^{20}$ = 1.5623

In the following are shown these compounds that were prepared in the same manner as the above-mentioned Synthesis Examples 1 to 5, together with those that were actually prepared by said Example.

| Compound No. | Chemical Name |
|---|---|
| No. 1: | 2-(4,6-dimethoxy-2-pyrimidinyl-thio)-3-methylbutanal 4-chlorophenylhydrazone. $n_D^{20}$ =1.5736 |
| No. 2: | 2-(4,6-dimethoxy-2-pyrimidinylthio)-3,3-dimethylbutanal 4-bromophenylhydrazone. mp. 120-123°C |
| No. 3: | 2-(4,6-dimethoxy-2-pyrimidinylthio)-3-methylbutanal N-methyl-N-phenylhydrazone. $n_D^{20}$ =1.5961 |
| No. 4: | 2-(4,6-dimethoxy-2-pyrimidinylthio)butanal hydrazone. $n_D^{20}$ =1.5728 |
| No. 5: | 2-(4,6-dimethoxy-2-pyrimidinylthio)-3-methylbutanal hydrazone. $n_D^{20}$ =1.5517 |
| No. 6: | 2-(4,6-dimethoxy-2-pyrimidinylthio)-3,3-dimethyl-butanal hydrazone. $n_D^{20}$ =1.5736 |
| No. 7: | 2-(4,6-dimethoxy-2-pyrimidinylthio)-3-methyl-3-phenyl-butanal hydrazone. $n_D^{20}$ =1.5716 |
| No. 8: | 2-(4,6-dimethoxy-2-pyrimidinylthio)-3-cyclohexyl-propanal hydrazone. $n_D^{20}$ =1.5489 |
| No. 9: | 2-(4,6-dimethoxy-2-pyrimidinylthio)butanal-4-chloro-phenyl-hydrazone. $n_D^{20}$ =1.6020 |
| No. 10: | 2-(4,6-dimethoxy-2-pyrimidinylthio)-3-methylbutanal methyl-hydrazone. $n_D^{20}$ =1.5351 |
| No. 11: | 2-(4,6-dimethoxy-2-pyrimidinylthio)-3-methylbutanal tert-butylhydrazone. $n_D^{20}$ =1.5208 |
| No. 12: | 2-(4,6-dimethoxy-2-pyrimidinylthio)-3-methylbutanal methyl-hydrazone. $n_D^{20}$ =1.5254 |
| No. 13: | 2-(4,6-dimethoxy-2-pyrimidinylthio)-3-methylbutanal phenyl-hydrazone. $n_D^{20}$ =1.5631 |
| No. 14: | 2-(4,6-dimethoxy-2-pyrimidinylthio)-3-methylbutanal-2-chlorophenylhydrazone. $n_D^{20}$ =1.5775 |
| No. 15: | 2-(4,6-dimethoxy-2-pyrimidinylthio)-3-methylbutanal-3-chlorophenylhydrazone. $n_D^{20}$ =1.5844 |

No. 16: 2-(4,6-dimethoxy-2-pyrimidinylthio)-3-methylbutanal-2,4-dichlorophenylhydrazone. $n_D^{20}$ =1.5983

No. 17: 2-(4,6-dimethoxy-2-pyrimidinylthio)-3-methylbutanal-p-tolyl-hydrazone. $n_D^{20}$ =1.5760

No. 18: 2-(4,6-dimethoxy-2-pyrimidinylthio)-3-methylbutanal-4-nitro-phenylhydrazone. mp. 102.5-103.5°C

No. 19: 2-(4,6-dimethoxy-2-pyrimidinylthio)-3-methylbutanal-2,4-dinitrophenylhydrazone. mp. 126-128°C

No. 20: 2-(4,6-dimethoxy-2-pyrimidinylthio)-3-methylbutanal-4-methoxyphenylhydrazone. $n_D^{20}$ =1.5778

No. 21: 2-(4,6-dimethoxy-2-pyrimidinylthio)-3-methylbutanal-propionylhydrazone. mp. 118.5-122.5°C

No. 22: 2-(4,6-dimethoxy-2-pyrimidinylthio)-3-methylbutanal-benzoylhydrazone. $n_D^{20}$ =1.5653

No. 23: 2-(4,6-dimethoxy-2-pyrimidinylthio)-3-methylbutanal-4-chlorobenzoylhydrazone. mp. 128-132°C

No. 24: 2-(4,6-dimethoxy-2-pyrimidinylthio)-3-methylbutanal-methylsulfonylhydrazone. $n_D^{20}$ =1.5204

No. 25: 2-(4,6-dimethoxy-2-pyrimidinylthio)-3-methylbutanal-benzenesulfonylhydrazone. $n_D^{20}$ =1.5746

No. 26: 2-[2-(4,6-dimethoxy-2-pyrimidinylthio)-3-methyl-butylidene hydrazinosulfonyl]benzoic acid methylester. $n_D^{20}$ =1.5587

No. 27: 2-(4,6-dimethoxy-2-pyrimidinylthio)-3,3-dimethyl-butanal methylhydrazone. $n_D^{20}$ =1.5408

No. 28: 2-(4,6-dimethoxy-2-pyrimidinylthio)-3,3-dimethyl-butanal tert.-butylhydrazone. $n_D^{20}$ =1.5191

No. 29: 2-(4,6-dimethoxy-2-pyrimidinylthio)-3,3-dimethyl-butanal dimethylhydrazone. $n_D^{20}$ =1.5345

No. 30: 2-(4,6-dimethoxy-2-pyrimidinylthio)-3,3-dimethyl-butanal 2-chlorophenylhydrazone. $n_D^{20}$ =1.5079

No. 31: 2-(4,6-dimethoxy-2-pyrimidinylthio)-3,3-dimethyl-butanal-4-chlorophenylhydrazone. $n_D^{20}$ =1.5722

No. 32: 2-(4,6-dimethoxy-2-pyrimidinylthio)-3,3-dimethyl-butanal-4-nitrophenylhydrazone. $n_D^{20}$ =1.6053

No. 33: 2-(4,6-dimethoxy-2-pyrimidinylthio)heptanal-4-nitrophenyl-hydrazone. mp. 127-128°C

No. 34: 2-(4,6-dimethoxy-2-pyrimidinylthio)-3-phenylpro-panal-4-nitrophenylhydrazone. m.p. 194-195.5°C

No. 35: 2-(4,6-dimethoxy-2-pyrimidinylthio)-3,3-dimethyl-butanal phenylhydrazone. $n_D^{20}$ =1.5731

No. 36: 2-(4,6-dimethoxy-2-pyrimidinylthio)-3-cyclohexyl-propanal methylhydrazone. $n_D^{20}$ =1.5440

No. 37: 2-(4,6-dimethoxy-2-pyrimidinylthio)-3,3-dimethylbutanal-2-pyridinylhydrazone. mp. 149-151°C

No. 38: 2-(4,6-dimethoxy-2-pyrimidinylthio)-3,3-dimethylbutanal-2-(4-chlorobenzothiazolyl)hydrazone. amorphous

No. 39: 2-(4,6-dimethoxy-2-pyrimidinylthio)-3,3-dimethylbu-tanal-3-trifluoromethylphenylhydrazone). $n_D^{20}$ =1.5474

No. 40: 2-(4,6-dimethoxy-2-pyrimidinylthio)-3-phenylbutanal-hydrazone. $n_D^{20}$ =1.5970

No. 41: 2-(4,6-dimethoxy-2-pyrimidinylthio)-3,3-dimethylbutanal diphenylhydrazone. mp. 88-92°C

No. 42: 2-(4,6-dimethoxy-2-pyrimidinylthio)-3,3-dimethylbutanal-N-methyl-N-phenylhydrazone. $n_D^{20}$ =1.5852

No. 43: 2-(4,6-dimethoxy-2-pyrimidinylthio)-3,3-dimethyl-butanal-tert-butyloxycarbonylhydrazone. $n_D^{20}$ =1.5042

No. 44: 2-(4,6-dimethoxy-2-pyrimidinylthio)-3,3-dimethylbutanal-azine. mp. 177-180°C

No. 45: 2-(4,6-dimethoxy-2-pyrimidinylthio)-2-cyclohexyl-ethanal-hydrazone. $n_D^{20}$ =1.5636

No. 46: 2-(4,6-dimethoxy-2-pyrimidinylthio)-3-methylbutanal-4-bromophenylhydrazone. $n_D^{20}$ =1.5925

No. 47: 2-(4,6-dimethoxy-2-pyrimidinylthio)-3-phenylpropanal-hydrazone. $n_D^{20}$ =1.6020

No. 48: 2-(4,6-dimethoxy-2-pyrimidinylthio)-3-phenylpropanal-methylhydrazone. $n_D^{20}$ =1.6002

No. 49: 2-(4,6-dimethoxy-2-pyrimidinylthio)-3,3-dimethylbutanal acetylhydrazone. mp. 149.5-152.5°C

No. 50: 2-(4,6-dimethoxy-2-pyrimidinylthio)-3,3-dimethylbutanal methylsulfonylhydrazone. $n_D^{20}$ =1.5038

No. 51: 2-(4,6-dimethoxy-2-pyrimidinylthio)-3,3-dimethylbutanal-ethoxycarbonylhydrazone. $n_D^{20}$ =1.5190

No. 52: 2-(4,6-dimethoxy-2-pyrimidinylthio)-3,3-dimethylbutanal ethylhydrazone. $n_D^{20}$ =1.5409

No. 53: 2-(4,6-dimethoxy-2-pyrimidinylthio)-3,3-dimethylbutanal-isopropylhydrazone. $n_D^{20}$ =1.5249

No. 54: 2-(4,6-dimethoxy-2-pyrimidinylthio)-3,3-dimethylbutanal-allylhydrazone. $n_D^{20}$ =1.5482

No. 55: 2-(4,6-dimethoxy-2-pyrimidinylthio)-3,3-dimethylbutanal-3-chlorophenylhydrazone. $n_D^{20}$ =1.5870

No. 56: 2-(4,6-dimethoxy-2-pyrimidinylthio)-3,3-dimethylbutanal-3,4-dichlorophenylhydrazone. $n_D^{20}$ =1.5857

No. 57: 2-(4,6-dimethoxy-2-pyrimidinylthio)-3,3-dimethylbutanal 3,5-dichlorophenylhydrazone. $n_D^{20}$ =1.5930

No. 58: 2-(4,6-dimethoxy-2-pyrimidinylthio)-3,3-dimethylbutanal 2,4-dichlorophenylhydrazone. mp. 120.5-121.5°C

No. 59: 2-(4,6-dimethoxy-2-pyrimidinylthio)-3,3-dimethylbutanal-benzylhydrazone. $n_D^{20}$ =1.5608

No. 60: 2-(4,6-dimethoxy-2-pyrimidinylthio)-3,3-dimethylbutanal-benzoylhydrazone. mp. 147.5-148°C

No. 61: 2-(4,6-dimethoxy-2-pyrimidinylthio)-3,3-dimethylbutanal-p-toluenesulfonylhydrazone. $n_D^{20}$ =1.5610

No. 62: 2-(4,6-dimethoxy-2-pyrimidinylthio)-3,3-dimethylbutanal-3-nitrophenylhydrazone. $n_D^{20}$ =1.5835

No. 63: 2-(4,6-dimethoxy-2-pyrimidinylthio)-3,3-dimethylbutanal-4-tert-butylbenzoylhydrazone. m.p. 136.5-141°C

No. 64: 2-(4,6-dimethoxy-2-pyrimidinylthio)-3,3-dimethyl-butanal-3-chlorobenzoylhydrazone. $n_D^{20}$ =1.5464

No. 65: 2-(4,6-dimethoxy-2-pyrimidinylthio)-3,3-dimethyl-butanal-3-chlorophenylsulfonylhydrazone. $_D^{20}$ =1.5626

No. 66: 2-(4,6-dimethoxy-2-pyrimidinylthio)-3,3-dimethyl-butanal-n-butyrylhydrazone. $n_D^{20}$ =1.5176

No. 67: 2-(4,6-dimethoxy-2-pyrimidinylthio)-3,3-dimethyl-butanal-m-tolylhydrazone. $n_D^{20}$ =1.5810

No. 68: 2-(4,6-dimethoxy-2-pyrimidinylthio)-3-phenylpropanal-4-fluorophenylhydrazone. $n_D^{20}$ =1.6097

No. 69: 2-(4,6-dimethoxy-2-pyrimidinylthio)-2-cyclohexyl-ethanal-hydrazone. $n_D^{20}$ =1.5636

No. 70: 2-(4,6-dimethoxy-2-pyrimidinylthio)-3-methylpentanalhydrazone. $n_D^{20}$ =1.5599

No. 71: 2-(4,6-dimethoxy-2-pyrimidinylthio)pentanalmethyl-hydrazone. $n_D^{20}$ =1.5508

No. 72: 2-(4,6-dimethoxy-2-pyrimidinylthio)pentanalhydrazone. $n_D^{20}$ =1.5698

No. 73: 2-(4,6-dimethoxy-2-pyrimidinylthio)-3,3-dimethylbutanal-5-chlorothiophene-2-sulfonylhydrazone. mp.125.5-128°C

No. 74: 2-(4,6-dimethoxy-2-pyrimidinylthio)-3,3-dimethylbutanal-2-(2-phenoxyethylmercapto)acetylhydrazone. mp.116-117.5°C

No. 75: 2-(4,6-dimethoxy-2-pyrimidinylthio)-3,3-dimethylbutanal-N-[4-(trifluoromethyl)pyrid-2-yl]-N-methyl-hydrazone. mp. 99.5-103.5°C

No. 76: 2-(4,6-dimethoxy-2-pyrimidinylthio)-3,3-dimethyl-butanal 4-isopropylphenylhydrazone. $n_D^{20}$ =1.5650

No. 77: 2-(4,6-dimethoxypyrimidinylthio)-3,3-dimethylbutanal-4-n-propylsemicarbazone. $n_D^{20}$ =1.5453

No. 78: 2-(4,6-dimethoxypyrimidinylthio)-3,3-dimethylbutanal-4,4-dimethylsemicarbazone. amorphous

No. 79: 2-(4,6-dimethoxypyrimidinylthio)-3,3-dimethylbutanal-4-phenylthiosemicarbazone. mp.58-62°C

No. 80: 2-(4,6-dimethoxypyrimidinylthio)-3,3-dimethylbutanal-4-(2-chlorophenyl)semicarbazone. mp. 136-140°C

No. 81: 1-[2-(4,6-dimethoxypyrimidinylthio)-3,3-dimethylbutylidene]-carbonohydrazide. mp. 142-145°C

No. 82: 1-[2-(4,6-dimethoxypyrimidinylthio)-3-methylbutylidene]carbonohydrazide. mp. 147-149.5°C

No. 83: 1,5-bis[2-(4,6-dimethoxypyrimidinylthio)-3-methylbutylidene]carbonohydrazide. mp. 162-166°C

No. 84: 1,5-bis[2-(4,6-dimethoxypyrimidinylthio)-3,3-dimethylbutylidene]carbonohydrazide. mp. 69-71.5°C

No. 85: 2-(4,6-dimethoxypyrimidinylthio)-3,3-dimethylbutanal-7-chloro-4-quinolylhydrazone.mp.175-177.5°C

No. 86: 2-(4,6-dimethoxypyrimidinylthio)-3,3-dimethylbutanal-3,5-bis(trifluoromethyl)benzoyl-hydrazone. mp. 192.5-195.5°C

No. 87: 2-(4,6-dimethoxypyrimidinylthio)-3,3-dimethylbutanal-2-chlorobenzoylhydrazone. $n_D^{20}$ =1.5682

No. 88: 2-(4,6-dimethoxypyrimidinylthio)-3,3-dimethylbutanal-ethoxy-carbonylacetylhydrazone. mp. 96.5-98.5°C

No. 89: 2-(4,6-dimethoxypyrimidinylthio)-3,3-dimethylbutanal-3-chloro-4-fluorophenylhydrazone. $n_D^{20}$ =1.5671

No. 90: 2-(4,6-dimethoxypyrimidinylthio)-3,3-dimethylbutanal-4-methylbenzoylhydrazone. $n_D^{20}$ =1.5414

No. 91: 2-(4,6-dimethoxypyrimidinylthio)-3,3-dimethylbutanal-2-thiophencarbonylhydrazone. $n_D^{20}$ =1.5629

No. 92: 2-(4,6-dimethoxypyrimidinylthio)-3,3-dimethylbutanal-4-nitro-benzylhydrazone. mp. 82-86°C

No. 93: 2-(4,6-dimethoxypyrimidinylthio)-3,3-dimethylbutanal-6-chloropyridazine-3-yl-hydrazone. mp.187-191.5°C

No. 94: 2-(4,6-dimethoxypyrimidinylthio)-3,3-dimethylbutanal-phenylacetyl-hydrazone. $n_D^{20}$ =1.5475.

**Biological test:**

Test Example 1

Post-emergence foliage treatment on upland weeds

Formulation of Active Compounds

Carrier: 5 parts by weight of acetone
Emulsifier: 1 part by weight of benzyloxy polyglycol ether

To produce a suitable formulation of each of the active compounds, 1 part by weight of the active compound was mixed with the stated amount of carrier and with the stated amount of emulsifier, and the resulting emulsifiable concentrate was diluted with water to the desired concentration.

Test Procedures

In a greenhouse, a number of test pots each having an area of 120 $cm^2$ were charged with soil taken out from a cultivated field. Seeds of barnyard grass (Echinochloa crus-gall) and redroot pigweed (Amaranthus retroflexeus) respectively, were sown onto the soil surface in the respective test pots with each of the sown soil surfaces being covered with a soil layer to allow them emerging.

After ten days, predetermined dosages of the active compound formulations prepared as mentioned above were uniformly sprayed onto the foliage portions of the test weeds in the respective test pots.

Three weeks after the application of the active compound formulations, the degrees of herbicidal effect on the weeds were determined. The resulting herbicidal effect were rated according to the following assessment:
Completely killed 100%
Status equivalent to non- treated pots 0%

Test Example 2

Pre-emergence soil treatment test on upland weeds

Test Procedures

In a greenhouse, a number of test pots each having an area of 120 $cm^2$ were charged with soil taken out from a cultivated field. Onto the soil surface in the respective test pots were sown the seeds of barnyard grass (Echinochloa crus-gall) and Amaranthus (Amaranthus retroflexeus) followed by soil covering thereon. Thereafter, predetermined dosages of the active compound formulations mentioned above were uniformly sprayed onto the soil surface of the respective test pots.

Four weeks after the application of the active compound formulations, the degrees of herbicidal effect on the weeds were determined.

In this test most of the compounds 1 to 84 show herbicidal activities between 70 and 100% against typical weeds like as Barnyard grass and Redroot pigweed in pre-emergence-tests and post-emergence-tests as well.

## Claims

**1.** Hydrazones of the formula (I)

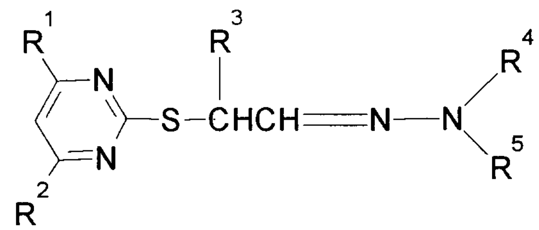

(I)

wherein

$R^1$ represents $C_{1-4}$ alkyl, $C_{1-4}$ alkoxy, halogeno-$C_{1-4}$ alkyl, halogeno-$C_{1-4}$ alkoxy or halogen,

$R^2$ represents $C_{1-4}$ alkoxy, halogeno-$C_{1-4}$ alkyl, halogeno-$C_{1-4}$ alkoxy or halogen,

$R^3$ represents $C_{3-8}$ cycloalkyl which may be substituted, $C_{1-15}$ alkyl which may be substituted or phenyl which may be substituted,

$R^4$ represents hydrogen, $C_{1-6}$ alkyl which may be substituted, $C_{2-6}$ alkenyl, $C_{2-6}$ alkynyl, phenyl which may be substituted, a 5- or 6-membered heterocyclic ring which may be substituted, a condensed heterocyclic ring which may be substituted, or one of the following groups:

$-SO_2$-R6, and

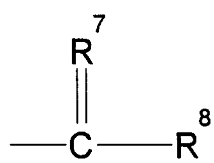

$R^5$ represents hydrogen, phenyl which may be substituted, or $C_{1-8}$ alkyl,

or wherein $R^4$ and $R^5$ together may represent a group represented by

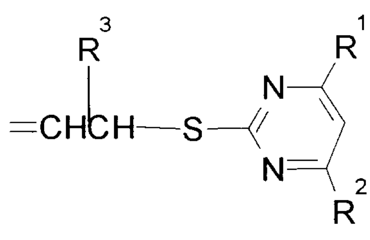

wherein $R^1$, $R^2$ and $R^3$ have the same meanings as mentioned above,

$R^6$ represents $C_{1-6}$ alkyl, phenyl which may be substituted, or a 5-or 6-membered heterocyclic ring which may be substituted,

$R^7$ represents oxygen or sulfur,

$R^8$ represents $C_{1-15}$ alkyl which may be substituted, $C_{1-6}$ alkoxy, phenyl which may be substituted, a 5- or 6-membered heterocyclic ring which may be substituted, a condensed heterocyclic ring which may be substituted, $C_{1-4}$ alkoxy-carbonyl, amino-carbonyl or one of the following groups:

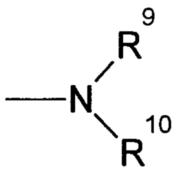,
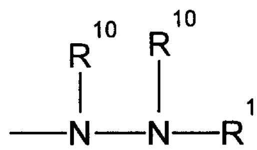
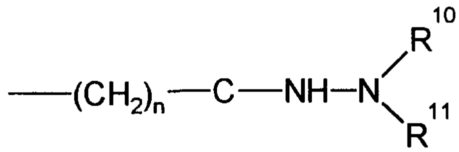

or 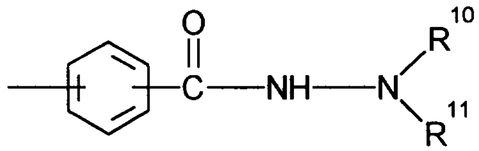

$R^9$ represents hydrogen, $C_{3-7}$ cycloalkyl which may be substituted by $C_{1-4}$ alkyl,$C_{1-6}$ alkyl which may be substituted, phenyl which may be substituted, $C_{2-6}$ alkynyl, benzenesulfonyl which may be substituted or benzoyl which may be substituted,

$R^{10}$ represents hydrogen, $C_{1-8}$ alkyl or phenyl, if two $R^{10}$ are in the same compound then both $R^{10}$ may have the same meanings

$R^{11}$ represents hydrogen, $C_{1-15}$ alkyl which may be substituted, or phenyl which may be substituted,

or $R^{10}$ and $R^{11}$ together represent a group of the following formula:

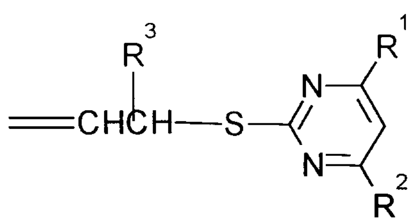

wherein $R^1$, $R^2$ and $R^3$ have the same meanings as mentioned above,
and

n represents an integer of 0 to 10.

**2.** The compounds of formula (I) according to Claim 1, wherein

$R^1$ represents $C_{1-4}$alkyl, $C_{1-4}$alkoxy, halogeno-$C_{1-4}$ alkyl, halogeno-$C_{1-4}$alkoxy or halogen,

$R^2$ represents $C_{1-4}$ alkoxy, halogeno-$C_{1-4}$ alkyl, halogeno-$C_{1-4}$ alkoxy or halogen,

$R^3$ represents $C_{3-6}$ cycloalkyl which is unsubstituted or substituted by $C_{1-4}$ alkyl, $C_{1-15}$ alkyl which is unsubstituted or substituted by one of the group consisting of halogen, $C_{3-8}$ cycloalkyl, phenyl which may be substituted by one of the groups consisting of nitro, cyano, halogen, $C_{1-4}$ alkyl, halogeno-$C_{1-2}$ alkyl and $C_{1-4}$ alkoxy, and naphthyl,

$R^3$ further represents phenyl which is unsubstituted or substituted by one of the group consisting of $C_{1-4}$ alkyl, $C_{1-4}$ alkoxy, halogen, halogeno-$C_{1-4}$ alkyl, nitro and cyano,

$R^4$ represents hydrogen, $C_{1-6}$ alkyl which is unsubstituted or substituted by halogen or phenyl which may be substituted by one of the group consisting of nitro, cyano, halogen, $C_{1-4}$ alkyl, halogeno-$C_{1-4}$ alkyl and $C_{1-4}^{-}$ alkoxy,

$R^4$ further represents $C_{2-6}$ alkenyl, $C_{2-6}$ alkynyl, phenyl which is unsubstituted or substituted by one of the group consisting of hydroxycarbonyl, halogen, $C_{1-4}$ alkyl, $C_{1-4}$ alkoxy, halogeno-$C_{1-4}$ alkyl, halogeno-$C_{1-4}$ alkoxy, nitro, cyano and trifluoromethylsulfonyl,

$R^4$ further represents an optionally substituted 5- or 6-membered heterocyclic ring, the hetero atom(s) of the heterocyclic ring are selected from the group consisting of nitrogen, oxygen and sulfur, and the substituent is selected from the group consisting of halogen, $C_{1-4}$ alkyl and halogeno-$C_{1-4}$ alkyl,

$R^4$ further represents a 5- or 6-membered heterocyclic ring condensed with phenyl which may be substituted (the hetero atom(s) of the condensed heterocyclic ring are selected from the group consisting of nitrogen, oxygen and sulfur, and the substituent is selected from the group consisting of halogen, $C_{1-4}$ alkyl and halogeno-$C_{1-4}$ alkyl), or one of the following

groups;
$-SO_2-R^6$ and

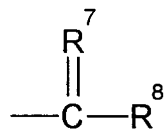

$R^5$ represents hydrogen, phenyl which may be unsubstituted or substituted by one of the group consisting of halogen and $C_{1-4}$ alkyl, or $C_{1-8}$ alkyl,

or wherein $R^4$ and $R^5$ together may represent a group represented by

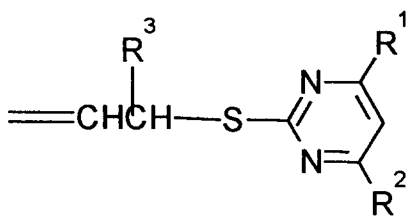

wherein $R^1$, $R^2$ and $R^3$ have the same meanings as mentioned above,

$R^6$ represents $C_{1-4}$ alkyl, phenyl which may be unsubstituted or substituted by one of the groups consisting of fluoro, chloro, bromo, $C_{1-4}$ alkyl, $C_{1-4}$ alkoxy, $C_{1-4}$ alkoxy-carbonyl, halogeno-$C_{1-4}$ alkyl, halogeno-$C_{1-4}$ alkoxy, nitro, cyano and trifluoromethylsulphonyl,

$R^6$ further represents an optionally substituted 5- or 6-membered heterocyclic ring, (the hetero atom(s) of the heterocyclic ring are selected from the group consisting of nitrogen, oxygen and sulfur, and the substituent is selected from the group consisting of halogen, $C_{1-4}$ alkyl and halogeno $C_{1-4}$ alkyl),

$R^7$ represents oxygen or sulfur,

$R^8$ represents $C_{1-15}$ alkyl which may be unsubstituted or substituted by one of the group consisting of halogen, nitro, cyano, $C_{1-4}$ alkyl, $C_{1-4}$ alkoxy, $C_{1-4}$ alkoxy carbonyl, halogeno-$C_{1-4}$ alkyl, halogeno $C_{1-4}$ alkoxy and phenoxy which may be substituted by one of the group consisting of nitro, cyano, halogen, $C_{1-4}$ alkyl, halogeno-$C_{1-4}$ alkyl and $C_{1-4}$ alkoxy, phenoxy-$C_{1-4}$ alkylthio which may be unsubstituted or substituted by one of the group consisting of nitro, cyano, halogen, $C_{1-4}$ alkyl, halogeno-$C_{1-4}$ alkyl and $C_{1-4}$ alkoxy,

$R^8$ further represents $C_{1-6}$ alkoxy, phenyl which may be substituted by the one of the group consisting of hydroxy, halogen, $C_{1-4}$ alkyl, $C_{1-4}$ alkoxy, halogeno-$C_{1-4}$ alkyl, halogeno-$C_{1-4}$ alkoxy, nitro and cyano,

$R^8$ further represents an optionally substituted 5- or 6-membered heterocyclic ring, (the hetero atom(s) of the heterocyclic ring are selected from the group consisting of nitrogen, oxygen and sulfur, and the substituent is selected from the group consisting of halogen, -$C_{1-4}$ alkyl and halogeno-$C_{1-4}$ alkyl), or

$R^8$ further represents $C_{1-4}$ alkoxy-carbonyl, aminocarbonyl or one of the following groups;

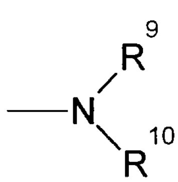
,
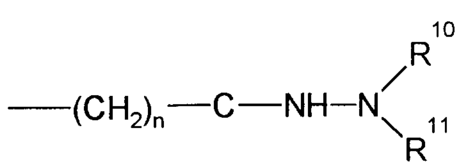
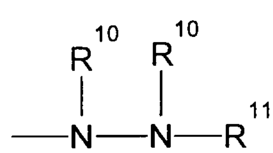

or
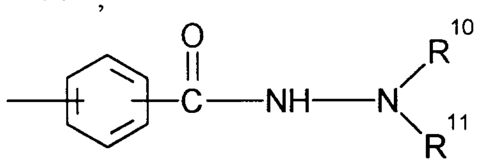

$R^9$ represents hydrogen, $C_{3-7}$ cycloalkyl which is unsubstituted or substituted by $C_{1-4}$ alkyl, $C_{1-6}$ alkyl which is unsubstituted or substituted by one of the group consisting of halogen, $C_{3-8}$ cycloalkyl and phenyl which may be substituted by one of the group consisting of nitro, cyano, halogen, $C_{1-4}$ alkyl, halogeno-$C_{1-4}$ alkyl, $C_{1-4}$ alkoxy, and naphthyl,

$R^9$ further represents phenyl which may be unsubstituted or substituted by one of the group consisting of halogen, $C_{1-4}$ alkyl, $C_{1-4}$ alkoxy, halogeno-$C_{1-4}$ alkyl, nitro and cyano,

$R^9$ further represents $C_{2-6}$ alkenyl, $C_{2-6}$ alkynyl, bensenesulfonyl which may be unsubstituted or substituted by one of the group consisting of halogen, $C_{1-4}$ alkyl, $C_{1-4}$ alkoxy, halogeno-$C_{1-4}$ alkyl, nitro and cyano, or

$R^9$ further represents benzoyl which may be unsubstituted or substituted by one of the group consisting of halogen, $C_{1-4}$ alkyl, $C_{1-4}$ alkoxy, halogeno-$C_{1-4}$ alkyl, nitro and cyano,

$R^{10}$ represents hydrogen, $C_{1-8}$ alkyl, or phenyl, and if two $R^{10}$ are in the same compound then both $R^{10}$ may have the same or different meanings,

$R^{11}$ represents hydrogen, $C_{1-15}$ alkyl which may be uusubstituted or substituted by halogen or phenyl which may be substituted by one of the group consisting of nitro, cyano, halogen, $C_{1-4}$ alkyl, halogeno-$C_{1-4}$ alkyl and $C_{1-4}$ alkoxy,

$R^{11}$ further represents phenyl which may be unsubstituted or substituted by one of the group consisting of halogen, $C_{1-4}$ alkyl, $C_{1-4}$ alkoxy, halogeno-$C_{1-4}$ alkyl, nitro and cyano,

or wherein $R^{10}$ and $R^{11}$ together may represent a group

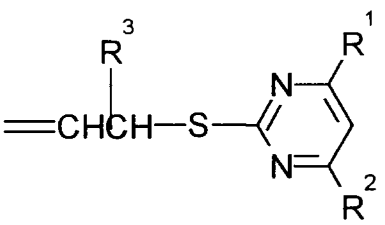

wherein $R^1$ , $R^2$ and $R^3$ have the same meanings as mentioned above,
and

n represents an integer of 0 to 10.

**3.** The compounds of formula (1) according to claim 1, wherein

$R^1$ represents $C_{1-2}$ alkyl, $C_{1-2}$ alkoxy, chloro-$C_{1-2}$ alkyl, fluoro-$C_{1-2}$ alkyl, chloro-$C_{1-2}$ alkoxy, fluoro-$C_{1-2}$ alkoxy, difluoro-$C_{1-2}$ alkoxy, trifluoro-$C_{1-2}$-alkoxy or chloro,

$R^2$ represents $C_{1-2}$ alkoxy, chloro-$C_{1-2}$ alkyl, fluoro-$C_{1-2}$ alkyl, chloro-$C_{1-2}$ alkoxy, fluoro-$C_{1-2}$ alkoxy, difluoro-$C_{1-2}$ alkoxy, trifluoro-$C_{1-2}$ alkoxy or chloro,

$R^3$ represents $C_{3-6}$ cycloalkyl which is unsubstituted or substituted by $C_{1-2}$ alkyl, $C_{1-12}$ alkyl which is unsubstituted or substituted by one of the group consisting of fluoro, chloro, $C_{3-6}$ cycloalkyl and phenyl which may be substituted by one of the group consisting of nitro, cyano, fluorine, chlorine, bromine, $C_{1-2}$ alkyl, fluoro-$C_{1-2}$ alkyl or $C_{1-2}$ alkoxy,

$R^3$ further represents phenyl which may be unsubstituted or substituted by one of the group consisting of $C_{1-2}$ alkyl, $C_{1-2}$ alkoxy, fluorine, chlorine, bromine, fluoro-$C_{1-2}$ alkyl, nitro or cyano,

$R^4$ represents hydrogen, $C_{1-4}$ alkyl which may be unsubstituted or substituted by one of the group consisting of fluorine, chlorine, bromine, phenyl which may be substituted by one of the group consisting of nitro, cyano, fluorine, chlorine, bromine, $C_{1-2}$ alkyl, fluoro-$C_{1-2}$ alkyl and $C_{1-2}$ alkoxy,

$R^4$ further represents $C_{3-6}$ alkenyl, $C_{3-6}$ alkynyl, phenyl which may be unsubstituted or substituted by one of the group consisting of hydroxycarbonyl, fluorine, chlorine, bromine, $C_{1-4}$ alkyl, $C_{1-4}$ alkoxy, fluoro-$C_{1-4}$ alkyl, fluoro-$C_{1-4}$ alkoxy, nitro, cyano and trifluoromethylsulfonyl,

$R^4$ further represents an optionally substituted 5- or 6-membered heterocyclic ring, the hetero atom(s) of the heterocyclic ring being selected from the group consisting of nitrogen, oxygen and sulfur, and the substituent is selected from the group consisting of fluorine, chlorine, methyl, ethyl and fluoro-$C_{1-2}$ alkyl,

$R^4$ further represents an optionally substituted 5- or 6-membered heterocyclic ring, condensed

with phenyl, the hetero atom(s) of the condensed heterocyclic ring are selected from the group consisting of nitrogen, oxygen and sulfur, and the substituent is selected from the group consisting of fluorine, chlorine, methyl, ethyl and fluoro-$C_{1-2}$ alkyl, or one of the following groups:

$-SO_2-R^6$ and

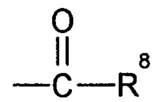

,

$R^5$ represents hydrogen, $C_{1-6}$ alkyl or phenyl which is unsubstituted or substituted by one of the group consisting of fluoro, chloro, methyl, ethyl and butyl,

or $R^4$ and $R^5$ together may represent a group

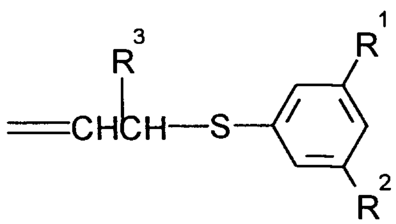

wherein $R^1$, $R^2$ and $R^3$ have the same meanings as mentioned above,

$R^6$ represents $C_{1-4}$ alkyl, phenyl which may be unsubstituted or substituted by one of the group consisting of fluorine, chlorine, bromine, $C_{1-2}$ alkyl, $C_{1-2}$ alkoxy, $C_{1-2}$ alkoxy-carbonyl, fluoro-$C_{1-2}$ alkyl, chloro-$C_{1-2}$ alkyl, fluoro-$C_{1-2}$ alkoxy, chloro-$C_{1-2}$ alkoxy, nitro, cyano and trifluoromethylsulphonyl,

$R^6$ further represents an optionally substituted 5- or 6-membered heterocyclic ring, the hetero atom(s) of the heterocyclic ring being selected from the group consisting of nitrogen, oxygen and sulfur, and the substituent is selected from the group consisting of fluorine, chlorine, methyl, ethyl and fluoro-$C_{1-2}$ alkyl,

$R^8$ represents $C_{1-8}$ alkyl which is unsubstituted or substituted by one of the group consisting of fluorine, chlorine, bromine, $C_{1-2}$ alkyl, $C_{1-2}$ alkoxy, fluoro-$C_{1-2}$ alkyl, chloro-$C_{1-2}$ alkyl, fluoro-$C_{1-2}$ alkoxy, chioro-$C_{1-2}$ alkoxy, nitro, cyano and phenoxy which may be substituted by one of the group consisting of nitro, cyano, fluoro, chloro, bromo, $C_{1-2}$ alkyl, fluoro-$C_{1-2}$ alkyl, $C_{1-2}$ alkoxy and phenoxy-$C_{1-2}$ alkylthio, wherein the phenyl ring may be unsubstituted or substituted by one of the group consisting of nitro, cyano, fluorine, chlorine, bromine, $C_{1-2}$ alkyl, fluoro-$C_{1-2}$ alkyl and $C_{1-2}$ alkoxy,

$R^8$ further represents $C_{1-4}$ alkoxy, phenyl which may be substituted by one of the group consisting of hydroxy, fluorine, chlorine, bromine, $C_{1-4}$ alkyl, $C_{1-4}$ alkoxy, fluoro-$C_{1-2}$ alkyl, fluoro-$C_{1-2}$ alkoxy, nitro and cyano,

$R^8$ further represents an optionally substituted 5- or 6-membered heterocyclic ring, the hetero atoms of the heterocyclic ring are selected from the group consisting of nitrogen, oxygen and sulfur, and the substituent is selected from the group consisting of fluorine, chlorine, methyl, ethyl and fluoro-$C_{1-2}$ alkyl, or the optionally substituted 5- or 6-membered heterocyclic ring is condensed with phenyl, the hetero atoms of the heterocyclic ring are selected from the group consisting of nitrogen, oxygen and sulfur, and the substituent of the heterocyclic or phenyl ring is selected from the group consisting of fluorine, chlorine, methyl, ethyl and fluoro-$C_{1-2}$ alkyl,

$R^8$ further represents $C_{1-2}$ alkoxy-carbonyl, aminocarbonyl or one of the following groups:

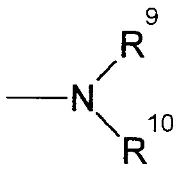,
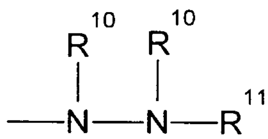
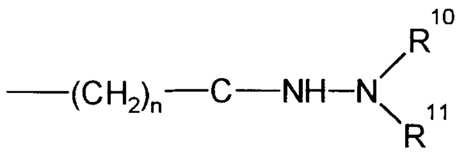

or 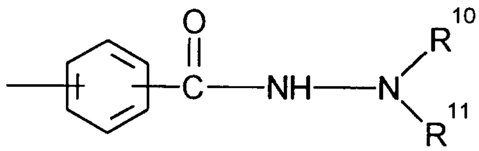

$R^9$ represents hydrogen, $C_{3-6}$ cycloalkyl which may be unsubstituted or substituted

$R^9$ represents $C_{1-4}$ alkyl which may be unsubstituted or substituted by one of the group consisting of chlorine, fluorine, bromine and phenyl which may be unsubstituted or substituted by one of the group consisting of fluorine, chlorine, bromine, $C_{1-2}$ alkyl, fluoro-$C_{1-4}$ alkyl and $C_{1-2}$ alkoxy,

$R^9$ further represents phenyl which may be unsubstituted or substituted by one of the group consisting of fluorine, chlorine, bromine, $C_{1-2}$ alkyl, $C_{1-2}$ alkoxy, fluoro-$C_{1-2}$ alkyl, fluoro-$C_{1-2}$ alkoxy, nitro and cyano,

$R^9$ further represents alkyl, propargyl, benzenesulfonyl which may be unsubstituted or substituted by one of the group consisting of chlorine, fluorine, bromine, $C_{1-2}$ alkyl, $C_{1-2}$ alkoxy, fluoro-$C_{1-2}$ alkyl, nitro ad cyano, or

$R^9$ further represents benzoyl which may be unsubstituted or substituted by one of the group consisting of chlorine, fluorine, bromine, $C_{1-2}$ alkyl, $C_{1-2}$ alkoxy, fluoro-$C_{1-2}$ alkyl, nitro and cyano,

$R^{10}$ represents hydrogen, $C_{1-4}$ alkyl or phenyl, and if two $R^{10}$ are the same compound then both $R^{10}$ may have the same or different meanings,

$R^{11}$ represents hydrogen, $C_{1-8}$ alkyl which may be unsubstituted or substituted by one of the group consisting of chlorine, fluorine, bromine and phenyl which may be unsubstituted or substituted by one of the group consisting of nitro, cyano, fluorine, chlorine, bromine, $C_{1-2}$ alkyl, fluoro-$C_{1-2}$ alkyl and $C_{1-2}$ alkoxy,

$R^{11}$ further represents phenyl which may be unsubstituted or substituted by one of the group consisting of fluorine, chlorine, bromine, $C_{1-2}$ alkyl, $C_{1-2}$ alkoxy, fluoro-$C_{1-2}$ alkyl, nitro and cyano,

or wherein $R^{10}$ and $R^{11}$ together may represent a group

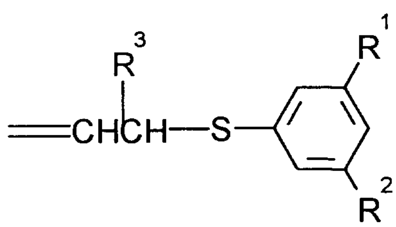

wherein $R^1$, $R^2$ and $R^3$ have the same meanings as mentioned above, and

n represents an integer of 0 to 8.

**4.** The compounds of formula (I) according to claim **1**, wherein

$R^1$ represents methoxy, difluoromethoxy or trifluoromethoxy

$R^2$ represents methoxy, difluoromethoxy or trifluoromethoxy,

$R^3$ represents in each case optionally methyl substituted cyclopropyl, cyclopentyl or cyclohexyl,

$R^3$ further represents $C_{1-5}$ alkyl which may be unsubstituted or substituted by at least one substituent selected from the group consisting of chlorine, fluorine, cyclopentyl, cyclohexyl

and phenyl which may be unsubstituted or substituted by at least one substituent selected from the group consisting of nitro, cyano, fluorine, chlorine, methyl, trifluoromethyl and methoxy, or

$R^3$ further represents phenyl which may be unsubstituted or substituted by at least one substituent selected from the group consisting of methyl, methoxy, fluorine, chlorine, trifluoromethyl, nitro and cyano,

$R^4$ represents hydrogen, $C_{1-4}$ alkyl which may be substituted at least by one substituent selected from the group consisting of fluorine, chlorine and phenyl which may be unsubstituted or substituted by at least one substituent selected from the group consisting of nitro, cyano, fluoro, chloro, methyl, trifluoromethyl and methoxy,

$R^4$ further represents allyl, propargyl, or phenyl which may be substituted at least one substituent selected from the group consisting of fluorine, chlorine, methyl, ethyl, methoxy, trifluoromethyl, trifluoromethoxy, nitro and cyano, pyridyl, furyl, thienyl, imidazolyl, pyrimidinyl, diazolyl and thiazolyl (those heterocyclic rings may be substituted at least by one substituent selected from the group consisting of fluorine, chlorine, methyl and trifluoromethyl,

$R^4$ further represents benzoxazolyl, bensothiazolyl, indolyl, quinolyl, phthalazinyl and benzimidazolyl (those condensed heterocyclic rings may be substituted at least by one substituent selected from the group consisting of fluoro, chloro, methyl and trifluoromethyl, or one of the following groups;

$-SO_2-R^6$ and

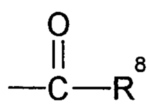

,

$R^5$ represents hydrogen, $C_{1-4}$ alkyl or phenyl, or

$R^4$ and $R^5$ together may represent a group

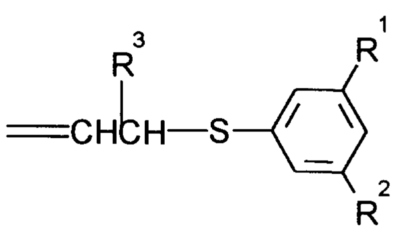

wherein $R^1$, $R^2$ and $R^3$ have the same meanings as mentioned above,

$R^6$ represents $C_{1-4}$ alkyl, phenyl which may be unsubstituted or substituted by one of the group consisting of fluorine, chlorine, methyl, methoxy and trifluoromethyl, or

$R^6$ further represents thiazolyl which may be unsubstituted or substituted fluorine or chlorine,

$R^8$ represents $C_{1-4}$ alkyl, $C_{1-4}$ alkoxy, phenyl which may be substituted at least by one to five substituents selected from the group consisting of fluorine, chlorine, methyl ethyl and methoxy, pyridyl, furyl, thienyl, indolyl, quinolyl, methoxy-carbonyl, or one of the following groups:

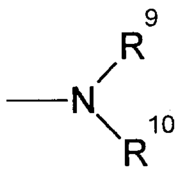,
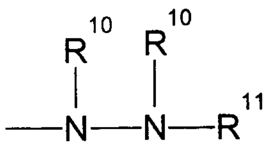
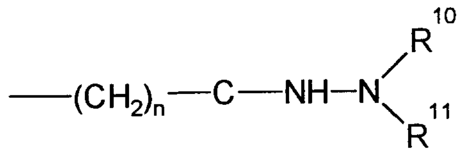

or 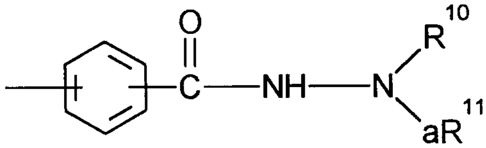

- $R^9$ represents hydrogen, in each case optionally by methyl substituted cyclopentyl and cyclohexyl,
- $R^9$ further represents $C_{1-4}$ alkyl which may be unsubstituted or substituted at least by one substituent selected from the group consisting of chlorine, fluorine, and phenyl which may be unsubstituted or substituted at least by one substituent selected from the group consisting of fluorine, chlorine and methyl,
- $R^9$ further represents phenyl which may be unsubstituted or substituted at least by one substituent selected from the group consisting of fluorine, chlorine, methyl, ethyl, methoxy and trifluoromethyl,
- $R^9$ further represents allyl, propargyl, benzenesulfonyl which may be unsubstituted or substituted at least by one substituent selected from the group consisting of chlorine, fluorine, methyl, ethyl, and methoxy,
- $R^9$ further represents benzoyl which may be unsubstituted or substituted at least by one substituent selected from the group consisting of chlorine, fluorine, methyl, ethyl and methoxy,
- $R^{10}$ represents hydrogen, methyl, ethyl, phenyl, and if two $R^{10}$ are the same compound then both $R^{10}$ have the same or different meanings,
- $R^{11}$ represents hydrogen, $C_{1-4}$ alkyl which may be unsubstituted or substituted at least by one substituent selected from the group consisting of chlorine, fluorine, phenyl which may be unsubstituted or substituted at least by one substituent selected from the group consisting of fluorine, chlorine, methyl, trifluoromethyl and methoxy,
- $R^{11}$ further represents phenyl which may be unsubstituted or substituted at least by one substituent selected from the group consisting of fluorine, chlorine, methyl, ethyl, methoxy and trifluoromethyl,

or wherein $R^{10}$ and $R^{11}$ together may represent a group

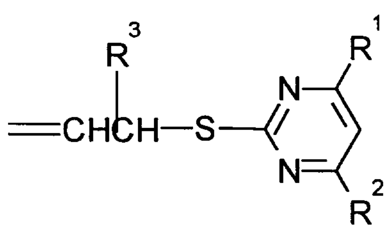

wherein $R^1$, $R^2$ and $R^3$ have the same meanings as mentioned above,
and

- n represents 0, 4 or 8.

**5.** Process for the preparation of hydrazones of the formula (I)

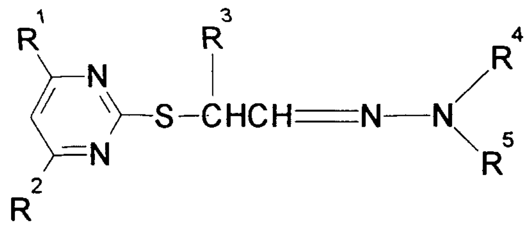

(I)

wherein

$R^1$ represents $C_{1-4}$ alkyl, $C_{1-4}$ alkoxy, halogeno-$C_{1-4}$ alkyl, halogeno-$C_{1-4}$ alkoxy or halogen,

$R^2$ represents $C_{1-4}$ alkoxy, halogeno-$C_{1-4}$ alkyl, halogeno-$C_{1-4}$ alkoxy or halogen,

$R^3$ represents $C_{3-8}$ cycloalkyl which may be substituted, $C_{1-15}$ alkyl which may be substituted or phenyl which may be substituted,

$R^4$ represents hydrogen, $C_{1-6}$ alkyl which may be substituted, $C_{2-6}$ alkenyl,$C_{2-6}$ alkynyl, phenyl which may be substituted, a 5- or 6-membered heterocyclic ring which may be substituted, a condensed heterocyclic ring which may be substituted, or one of the following groups: $-SO_2-R^6$ and

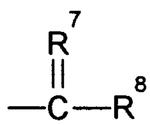

,

$R^5$ represents hydrogen, phenyl which may be subtituted, or $C_{1-8}$ alkyl,

or wherein $R^4$ and $R^5$ together may represent a group represented by

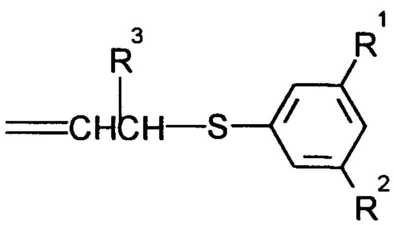

wherein $R^1$ , $R^2$ and $R^3$ have the same meanings as mentioned above,

$R^6$ represents $C_{1-6}$ alkyl, phenyl which may be substituted, or a 5- or 6-membered heterocyclic ring which may be substituted,

$R^7$ represents oxygen or sulfur,

$R^8$ represents $C_{1-15}$ alkyl which may be substituted, $C_{1-6}$ alkoxy, phenyl which may be substituted, a 5- or 6-membered heterocyclic ring which may be substituted, a condensed heterocyclic ring which may be substituted, $C_{1-4}$ alkoxy-carbonyl, amino-carbonyl or one of the following groups:

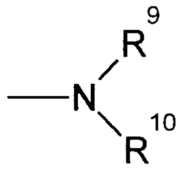 , 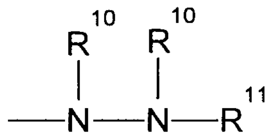 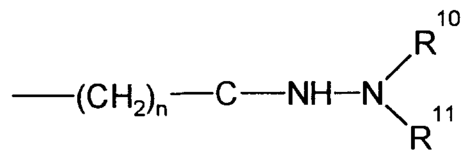

or 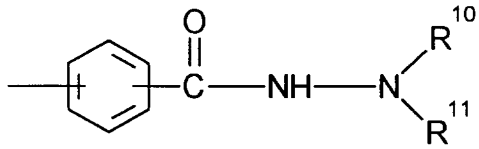

$R^9$ represents hydrogen, $C_{3-7}$ cycloalkyl which may be substituted by $C_{1-4}$ alkyl,$C_{1-6}$ alkyl which maybe substituted, phenyl which may be substituted, $C_{2-6}$ alkenyl, $C_{2-6}$ alkynyl, benzenesulfonyl which may be substituted or benzoyl which may be substituted,

$R^{10}$ represents hydrogen, $C_{1-8}$ alkyl or phenyl, if two $R^{10}$ are in the same compound then both two $R^{10}$ may have the same or different meanings,

$R^{11}$ represents hydrogen, $C_{1-15}$ alkyl which may be substituted, or phenyl which may be substituted,

or $R^{10}$ and $R^{11}$ together represent a group of the following formula:

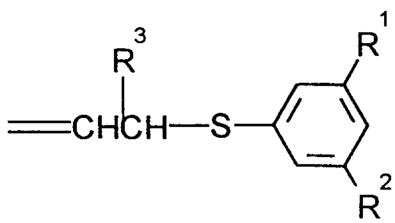

wherein $R^1$, $R^2$ and $R^3$ have the same meanings as mentioned above,
and

n represents an integer of 0 to 10,

characterized in that

(a) compounds of the formula (II)

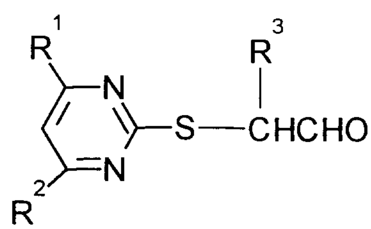 (II)

wherein $R^1$, $R^2$ and $R^3$ represent the same meanings as mentioned above, are reacted with a hydrazone derivative of the formula (III)

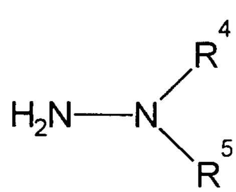

wherein $R^4$ and $R^5$ represent the same meanings as mentioned above,
in the presence of inert solvents and, if appropriate, in the presence of an acid binder or an acid,
or
(b) in the case where $R^4$ and $R^5$ together form a group of the formula

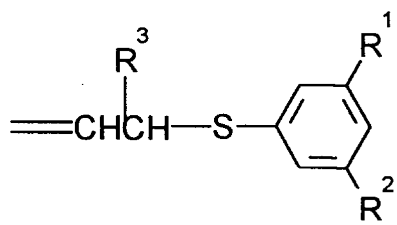

one mol hydrazine is reacted with two mols of a compound of the formula (II), in the presence of inert solvents and, if appropriate, in the presence of an acid,
or
(c) in the case where $R^4$ represents $-SO_2-R^6$ :
a compound of the formula (Ia)

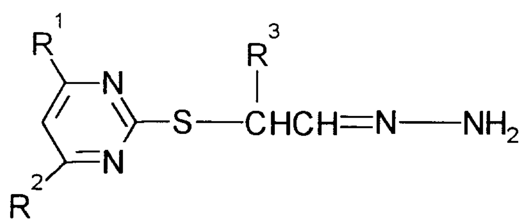 (Ia)

wherein $R^1$, $R^2$ and $R^3$ have the same meanings as mentioned above,
are reacted with a compound of the formula (IV)

$R^{12}—SO_2—R^6$

wherein $R^6$ has the same meaning as mentioned above, and $R^{12}$ represents chlorine or bromine,
in the presence of inert solvents and, if appropriate, in the presence of an acid binder,
or
(d) in the case where $R^4$ represents

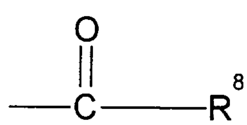

the above mentioned compound of the formula (Ia) is reacted with a compound of the formula (V)

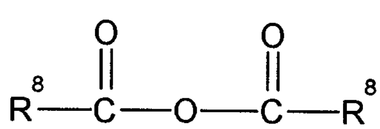 (V)

wherein $R^8$ has the same meaning as mentioned above,
in the presence of inert solvents and, if appropriate, in the presence of an acid binder,
or
(e) in the case where $R^4$ represents

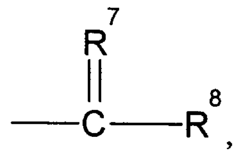

the above-mentioned compound of the formula (Ia) is reacted with a compound of the formula (VI)

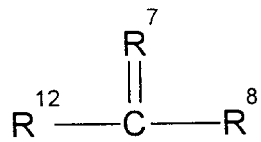 (VI)

wherein $R^7$, $R^8$ and $R^{12}$ have the same meanings as mentioned above,
in the presence of inert solvents and, if appropriate, in the presence of an acid binder.

6. Herbicidal compositions, characterized in that they contain at least one hydrazone of the formula (I) according to Claims 1 to 5.

7. Process for combating weeds, characterized in that hydrazones of the formula (I) according to Claims 1 to 4 are allowed to act on weeds and/or their habitat.

8. Use of hydrazones of the formula (I) according to Claims 1 to 4 for combating weeds.

9. Process for the preparation of herbicidal compositions, characterized in that hydrazones of the formula (I) according to Claims 1 to 4 are mixed with extenders and/or surface active agents.